# EUROPEAN PATENT SPECIFICATION

(11) **EP 1 898 904 B1**
(45) Date of publication and mention of the grant of the patent: **07.10.2009**
(21) Application number: 06772653.9
(22) Date of filing: 08.06.2006
(51) Int. Cl.: A61K 31/40, A61K 9/22, A61K 9/24

(54) **MODIFIED RELEASE 1-[(3-HYDROXY-ADAMANT-1-YLAMINO)-ACETYL]-PYRROLIDINE-2(S)-CARBONITRILE FORMULATION**
1-[(3-HYDROXY-ADAMANT-1-YLAMINO)-ACETYL]-PYRROLIDIN-2(S)-CARBONITRIL-FORMULIERUNG MIT MODIFIZIERTER FREISETZUNG
FORMULATION DE 1-Ý(3-HYDROXY-ADAMANT-1-YLAMINO)-ACETYL¨-PYRROLIDINE-2(S)-CARBONITRILE

(30) Priority: 10.06.2005 US 689719 P; 14.06.2005 US 690309 P
(43) Date of publication of application: 19.03.2008
(73) Proprietor: NOVARTIS AG, 4056 Basel (CH); Novartis Pharma GmbH, 1230 Vienna (AT)
(72) Inventor: KOWALSKI, James, Belle Mead, NJ 08502 (US); LAKSHMAN, Jay, Parthiban, Cedar Knolls, NJ 07927 (US); SERAJUDDIN, Abu, T. M., Flushing, Queens, NY 11358 (US); JOSHI, Yatindra, Princeton, NJ 08540 (US)
(74) Representative: Barbier, Denis Robert
(86) International application number: PCT/US2006/022416
(87) International publication number: WO 2006/135723

(56) References cited:
- EP-A- 1 537 880
- WO-A-01/52825
- WO-A-20/06047248
- CA-A1- 2 519 208
- BURKEY B F ET AL: "Combination Treatment of a DPP-IV Inhibitor NVP-LAF237 with Pioglitazone Completely Normalized Glucose Tolerance in Adult Obese Zucker Rats" DIABETES, NEW YORK, NY, US, vol. 51, no. sup2, 2002, pages A338-A339, XP009061638 ISSN: 0012-1797

## Description

The present invention relates to a sustained release preparation containing a dipeptidyl peptidase IV inhibitor (DPP-IV)' useful for preventing or treating diabetes mellitus, non-insulin-dependent diabetes mellitus, obesity, arthritis, osteoporosis, and other diseases.

DPP-IV is a useful drug for treating diabetes mellitus, non-insulin-dependent diabetes mellitus, obesity, arthritis, osteoporosis, and other diseases because it inhibits inactivation of glucagons-like peptide-I (GLP-I) in blood plasma and increases its incretion action (WO 02/062,764, WO 01/55105, WO 02/02560).

However, as described in detail below, there were some cases in which strong inhibition of DPP-IV activity in a living body was not always preferable.

For example, it has been reported that the DPP-IV inhibitor enhances vasodialation action by substance O and that the DPP-IV activity was elevated when treating chronic rhinosinusitis because there was an inverse relationship between DPP-IV activity of nasal mucosa of a patient with chronic rhinosinusitis and density of inflammatory cells on the nasal mucosa (for example, FASEB J., 2002, 16:1132-1134).

Accordingly, it is considered undesirable to strongly inhibit DPP-IV activity in a patient with diabetes complicating chronic inflammation because it results in aggravation of inflammation.

It has been reported that selective DPP-IV activity is important for glucose homeostasis since GLP-I (9-36) amide, a metabolite from GLP-I with DPP-IV, reduces blood glucose level (Am. J. Physiol. Endocrinol. Metab., 2002, 282:E873-E879).

Further it has been reported that in treatment of a patient with type C Hepatitis using interferon-α, severity of depression and anxiety correlated with decrease of serum DPP-IV activity (for example, Mol. Psychiatry, 2001, 6:475-480).

In view of the current circumstances that there are some cases in which strong inhibition of *in vivo* DPP-IV activity is not always preferable for the living body, the present inventors considered it necessary to develop a preparation that could appropriately inhibit DPP-IV activity and also afford excellent convenience or compliance.

The inventors herein disclose a modified/sustained release 1-[(3-Hydroxy-adamant-1-ylamino)-acetyl]-pyrrolidine-2(S)-carbonitrile (also known as LAF237 or vildagliptin (INN)) formulation for these purposes. The development of a modified/sustained release (MR) formulation comprising vildagliptin is necessary to improve the treatment quality of the treated patients. vildagliptin is an orally active, dipeptidylpepidase IV inhibitor indicated for improvement of glycemic control in type II diabetes.

LAF237 is specifically disclosed in Example 1 of WO 00/34241. LAF237 can be formulated as described in WO 00/34241, or in the International Patent Application No. EP2005/000400 (application number).

Since vildagliptin is sensitive to moisture, one of the main emphases during the Market Formulation (MF) development was product stability. Hydrolysis is the main degradation pathway for vildagliptin. The stability of vildagliptin is known to be affected by initial moisture content of the tablet, excipients with high intrinsic moisture and the amount of excipients present in the tablet (% drug load). The current validated vildagliptin formulation is a DC (direct compression) tablet with a 25% drug load.

Considering the water-sensitivity of vildagliptin, Clinical service form (CSF) and Market Formulation (MF) development was planned to focus on techniques that would not require the use of water, such as direct compression.

vildagliptin is classified as Category II, therefore, no special handling practices are required.

The vildagliptin drug substance (DS) is sensitive to moisture.

Very tight packaging is necessary. Current retest date is 30 months with storage at 25°C.

It is well known that patient adherence to a drug regimen indirectly correlates with frequency of dosing i.e. greater adherence is seen with a once daily (OD) dosing regimen compared to a twice daily (BID) dosing regimen. The majority of data currently available for vildagliptin is derived from studies based on the immediate release (IR) formulation of vildagliptin given BID.

Replacing the 50mg IR BID dose with a OD modified release dose will provide the dual benefit of dosing convenience to the patient and a inovative OD product for treating patients.

The present invention provides a pharmaceutical tablet formulation, capable of being compressed, preferably directly compressed, into a tablet having adequate hardness/friability, an acceptable disintegration time, low sensitivity to moisture, improved stability, an acceptable dissolution pattern and improved pharmacokinetic profile in treated patients. The pharmaceutical tablet formulation, can also be used in e.g. a capsule, tablet, compressed table, direct compressed tablets, granule.

### Summary of the Invention

The subject invention provides a pharmaceutical tablet formulation comprising per unit dosage form e.g. per tablet the following ingredients:
(a) a compound as an active ingredient, wherein the compound has a formula: 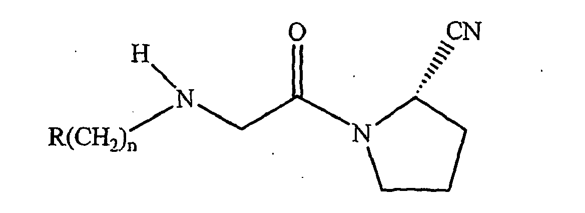 wherein R is substituted adamantyl and n is an integer from 0 to 3; or a pharmaceutically acceptable salt thereof;
(b) a hydroxypropyl methylcellulose with an apparent viscosity of 80,000 cP to 120,000 cP (nominal value 100,000 cP) when present in a 1% solution;
(c) a microcrystalline cellulose; and
(d) a magnesium stearate

The subject invention also provides a pharmaceutical tablet formulation, comprising per 400 mg tablet the following ingredients:
(a) vildagliptin or a pharmaceutically acceptable salt thereof in an amount of about 100 mg;
(b) a hydroxypropyl methylcellulose in an amount of about 160 mg, wherein the hydroxypropyl methylcellulose has an apparent viscosity of 80,000 cP to 120,000 cP (nominal value 100,000 cP) when present in a 1% solution;
(c) a microcrystalline cellulose in an amount of 120 mg;
(d) a lactose in an amount of about 16 mg; and
(e) a magnesium stearate in an amount of 4 mg.

The subject invention also provides a pharmaceutical tablet formulation comprising per unit dosage form e.g. per tablet the following ingredients:
(a) a compound as' an active ingredient, wherein the compound has a formula: 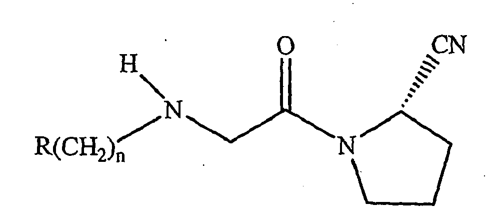 wherein R is substituted adamantyl and n is an integer from 0 to 3; or a pharmaceutically acceptable salt thereof; and
(b) a hydroxypropyl methylcellulose with an apparent viscosity of 80,000 cP to 120,000 cP (nominal value 100,000 cP) when present in a 1% solution.

### Brief Description of the Figures

Figure 1. Process Flow. This figure shows the selected manufacturing process.
Figure 2. Dissolution Profile for Fast and Slow Release.
Figure 3. vildagliptin MR 100mg tablet compression profile for CSF formulation containing 40% HPMC.
Figure 4. vildagliptin MR 100 mg tablet friability for CSF Formulation containing 40% HPMC.
Figure 5. Effect of roller compaction and dwell time, 30% HPMC, 100mg tablet. 30% HPMC was evaluated to improve the compression profile. The effect of the tablet dwell time was simultaneously evaluated at speeds from 40 to 80rpm.
Figure 6. Effect of roller compaction on 100mg tablet hardness. A Fitzpatrick (Chilsonator, model IR220) was evaluated with the interim powder blend without the addition of magnesium stearate, at increasing compaction forces.
Figure 7. Effect of roller compaction on 150mg tablet hardness. A Fitzpatrick (Chilsonator, model IR220) was evaluated with the interim powder blend without the addition of magnesium stearate, at increasing compaction forces.
Figure 8. Effect of roller compaction on the 100mg MR dissolution profile. The effect of roller compaction on the dissolution profile was studied with 40% HPMC K100M and compared to the dissolution profile of the clinical batch.
Figure 9. Effect of roller compaction on the 150mg MR dissolution profile. The effect of roller compaction on the dissolution profile was studied with 40% HPMC K100M and compared to the dissolution profile of the clinical batch.
Figure 10. Effect of roller compaction on vildagliptin 100mg hardness using a Bepex roller compactor. The effect of increasing compaction forces was studied.
Figure 11. Effect of roller compaction on vildagliptin 150 mg tablets using Bepex roller compactor. The effect of increasing compaction forces was studied.
Figure 12. Effect of roller compaction on 100mg tablet. The effect of Fitzpatrick (Chilsonator, model IR220) roller compactor at compaction forces from 500 to 10,000 lb/in on a powder blend containing 40% HPMC.
Figure 13. Effect of roller compaction on 150mg tablets. The effect of Fitzpatrick (Chilsonator, model IR220) roller compactor at compaction forces from 500 to 10,000 lb/in on a powder blend containing 40% HPMC.
Figure 14. Effect of roller compaction on 100mg tablet friability. Roller compaction forces greater than 5,000 lb/in (or 43.75 KN) produced tablets with a compression profile worse than the CSF without roller compaction or pre-compression.

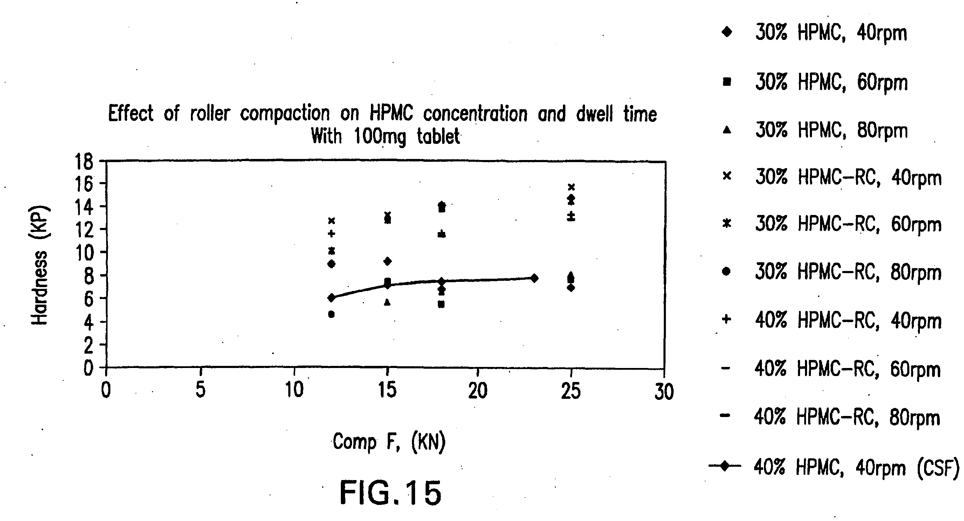

Figure 15. Effect of roller compaction on HPMC concentration and dwell time with 100mg tablet.

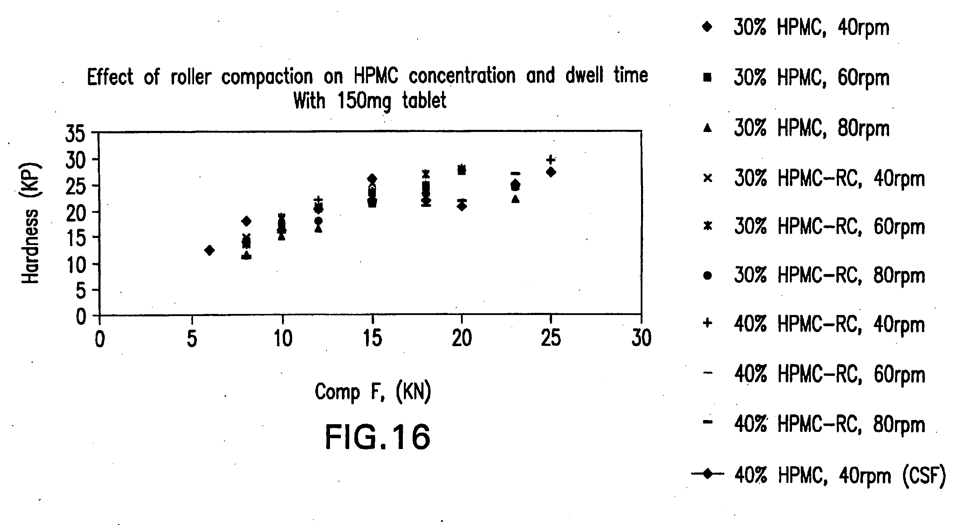

Figure 16. Effect of roller compaction on HPMC concentration and dwell time with 150mg tablet.
Figure 17. Dissolution profile for 100mg tablet.
Figure 18. Dissolution profile for 150mg tablet.
Figure 19. Effect of roller compaction on sieve analysis.
Figure 20. Effect of roller compaction on sieve analysis.

### Detailed Description of the Invention

### Terms

"DPP-IV inhibitor" means a compound that inhibits enzymatic activity of DPP-IV [Classification by IUBMB; EC3.4.14.5]. The compound may be either peptidyl or non-peptidyl. As long as the DPP-IV inhibitor retains its inhibitory activity, the form may be different before and after administration into the living body. Namely, the DPP-IV inhibitor may be "active metabolites" which have DPP-IV inhibitory activity after undergoing structural changes through metabolism in the living body. Further, the DPP-IV inhibitor may be a "prodrug" that is changed into an activated form by reactions with enzymes, stomach acids, etc. under *in vivo* physiological conditions. In addition, the DPP-IV inhibitory activity can be confirmed by utilizing, for example, the method by Raymond et al. (Diabetics, 1998, 47:1253-1258) .

Specific examples of the DPP-IV inhibitor include
(1) A compound of the formula: 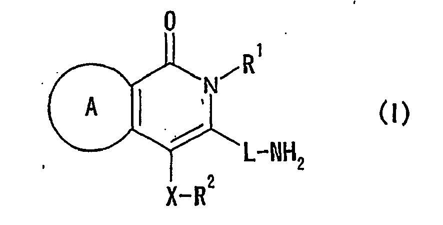 (wherein ring A is a 5- to 10-membered aromatic ring which may be substituted; R¹ and R² are each a hydrocarbon group which may be the same or different and may be substituted, or a heterocyclic group which may be substituted; X is a bond, -O-, -S-, -SO-, -SO₂- or -NR³-(R³ is a hydrogen atom or a hydrocarbon group which may be substituted); and L is a divalent hydrocarbon group) described in WO 02/062764.
   The compound represented by formula (I) may be an anhydrous or hydrous substance, as well as a prodrug.
   Suitable examples of the compound represented by formula (I) include the following compounds.
   Compound I-a
   A compound wherein the ring A is a benzene ring which may have 1 or 2 substitutions selected from:
   1) a cyano group;
   2) a C₁₋₁₀ alkyl group (preferably, ethyl) or a C₂₋₁₀ alkenyl group (preferably, ethenyl) which may be substituted with a carbamoyl group or a carboxyl group, respectively;
   3) a hydroxyl group whch may be substituted (preferably, a 1-10 C alkoxy group (preferably, methoxy, isopropoxy) which may have 1 to 3 substituents selected from a carbomoyl group, a carboxyl group and a 2-5 C alkoxycarbonyl group (preferably, methoxycarbonyl); a hydroxyl group; a 7-13 C aralkyloxy group (preferably, benzyloxy)) (more preferably carbamoylmethoxy);
   4) an acyl group (preferably, a C₁₋₆ alkyl-carbonyl (preferably acetyl), carbamoyl, mono- or di-(C₁₋₆ alkyl which may have 1 to 3 subsitutents selected from a halogen atom and a C₁₋₆ alkoxy-carbonyl)-carbamoyl (preferably, methycarbamoyl, ethycarbamoyl, propylcarbamoyl, dimethylcarbamoyl, trifluoroethylcarbamoyl, ethoxycarbonylmethylcarbamoyl, etc.) C₃₋₁₀ cycloalkyl-carbamoyl (preferably, cyclopropylcarbamoyl), C₇₋₁₃ aralkyl-carbamoyl (preferably, benzylcarbamoyl), a nitrogen-containing heterocycle-carbonyl which may be substituted with hydroxyl (preferably, pyrrolidinylcarbonyl, piperidinocarbonyl), C₁₋₆ alkylsulfonyl (preferably, methylsulfonyl), a C₁₋₆ alkylsulfinyl (preferably, methylsulfinyl), carboxyl, C₁₋₆ alkoxy-carbonyl (preferably, methoxycarbonyl), and thiocarbamoyl);
   5) an amino group which may be substituted (preferably, carbamoylamnino);
   6) a thiol group (preferably, a 1-10 C alkylthio group which may be substituted with a carbamoyl group (preferably methylthio);
   7) a heterocyclic group which may be substituted (an aromatic heterocyclic group (preferably, furyl, thienyl, oxazolyl, oxadiazolyl, thiazolyl, tetrasolyl, pyridyl, pyrrolyl, triazolyl) or a non-aromatic heterocyclic group (preferably, dioxoisoindole, 5-oxooxadiazole-3-yl, 5-oxothiaxialole 3-yl), respectively, which may have 1 or 2 substituents preferably selected from a C₁₋₆ alkyl group which may be substituted with 1 to 3 halogen atoms (preferably, methyl, trifluoromethyl), a carboxyl group, a 2-8 calkoxy carbonyl (preferably, ethoxycarbonyl), a cyano group, a carboamoyl group, anamino group, a mono- or di-C₂₋₁₀ alkanoylamnio group (e.g. acetylamnio isopentanoylamnio), C₁₋₁₀ alkoxy-carbonylamnio group (e.g. methoxycarbonylamnio), a carbamoylamino group, a mono- or di-C₁₋₁₀ alkyl-carbamoylamnio group (e.g., methylcarbmoylamino, dimethylcaramoylamino), a C₆₋₁₄ aryl-carbonylaminogroup (e.g., benzoylamnio), a C₃₋₁₀ cycloalkyl-carbonylamnio group, a C₇₋₁₃ aralkyloxy-carbonylamnio, dimethylsulfonylamnio), a mono- or di-C₁₋₁₀ alkyl sulfonyl amino group (e.g., methyl sulfonyl amino, diemthyl sulfonylamino), a C₆₋₁₄ arylsulfonylamnio group and a C₁₋₆ alkoxy-carbanoylamnino group (e.g., methoxycarbamoylamnio);
   8) a benzene ring, which may have 1 or 2 substituents slected from amidino groups; R¹ is a 4-10 C alkyl group (preferably, isobutyl, neopentyl) or a 4-10 cycloalkylalkyl group (preferably, cyclopropylmethyl); R² is a 6-14 C aryl group (preferably phenyl) which may have 1 or 2 substituents selected from a halogen atom (preferably, fluorine, chlorine) and C₁₋₆ alkyl (preferably methyl); X is a bond; and L is C₁₋₁₀alkylene (preferably, -CH₂-).
      Compound I-b
      A compound wherein the ring A is a benzene ring which may have 1 or 2 substituents selected from:
      1) a C₁₋₁₀ alkyl group (preferably, ethyl) or a C₂₋₁₀ alkenyl group (preferably, ehthenyl) which may be substituted with a 2-8 C alkoxycarbonyl group (preferably, ethoxycarbonyl) or a carbamoyl group, respectively;
      2) a hydroxyl group which may be substituted (preferably, a 1-10 C alkoxy group (preferably, methoxy) which may be substituted with a carbamoyl-group; more preferably, carbamoylmethoxy);
      3) an acyl group (preferably, carbamoyl, thiocarbamoyl, carboxyl);
      4) a heterocyclic group which may be substituted (an aromatic heterocyclic group (preferably, furyl, rhienyl, oxazoyl, oxadiazolyl, thiazolyl, tetrasolyl, pyridyl, pyrrolyl, triazoyl) or a non-aromatic heterocyclic group (preferably, 5-oxooxadiazole-3-yl), which may have 1 or 2 substituents preferably selected from a C₁₋₆ alkyl group (preferably, methyl), a carboxyl group, a 2-8 C alkoxycarbonyl group (preferably, ethoxycarbonyl), a cyano group, a carbamoyl group, an amnio group, a mono- or di-C₂₋₁₀ alkanoylamnino group (e.g., acetylamnio, isopentanoylamino), C₁₋₁₀ alkoxy-carbonylamnio group (e.g. methoxycarbonylamnio), a carbamoylamnio group, a mono- or di-C₁₋₁₀ alkyl-carbamoylamnio group (e.g., methylcarbamoylamnio, dimethylcarbamoylamino group), a C₆₋₁₄ aryl-carbonylamnio group (e.g., benzoylamino), a C₃₋₁₀ cycloalkyl-carbonylamino group (e.g., methylsulfonylamino, dimethylsulfonylamino), C₆₋₁₄ arylsulfonylamnio group and a C₁₋₆ alkoxy-carbamoylamino group (e.g., mehtoxycarbamoylamino)); R¹ is a 4-10 C alkyl group (preferably, isobutyl, neopentyl) or a 4-10 C cycloalkylalkyl group (preferably, cyclopropylmethyl); R² is a 1-10 C alkyl group (preferably, butyl) which amy be substituted with 1 to 3 halogen atoms; X is -O-; and L is C₁-₁₀ alkylene (preferably, -CH₃-).

      Among the compounds represented by formula (I), preferably are, especially, 1-[2-[(5-cyanopyridin-2-yl)amino]ethylamino]acetyl-2-cyano(S)-pyrrolidone; 1-[(3-hydroxy-adamant-1-ylamino)-acetyl]-pyrrolidine-2(S)-carbonitrile; 2-[3-(aminomethyl)-4-butoxy-2-isobuthyl-1-oxo-1,2-dihydro-6-isoquinolyl[-1,3-thiazole-4-carbonitrile; 2-[3-(aminomethyl)-4-butoxy-2-isobuthyl-1-oxo-1,2-dihydro-6-isoquinolyl]-1,3-thiazole-4-carboxylic acid; 2-[3-(aminomethyl)-4-butoxy-2-isobuthyl-1-oxo-1,2-dihydro-6-isoquinolyl]-1,3-thiazole-4-carboxamide; ethyl 2-[3-(aminomethyl)-4-butoxy-2-isobuthyl-1-oxo-1,2-dihydro-6-isoquinolyl]-1,3-thiazole-4-carboxylate; (E) 3-[3-(aminomethyl)-4-butoxy-2-isobuthyl-1-oxo-1,2-dihydro-6-isoquinolyl]-2-propenamide; (E)-3-[3-(aminomethyl)-2-isobuthyl-4-phenyl-1-oxo-1,2-dihydro-6-isoquinolyl]-2-propenamide; 3-(aminomethyl)-2-isobuthyl-1-oxo-4-phenyl-1,2-dihydro-6-isoquinolinecarboxamide; 2-{[3-(aminomethyl)-2-isobuthyl-4-phenyl-1-oxo-1,2-dihydro-6-isoquinolyl]oxy}acctamide.
(2) A compound represented by a formula 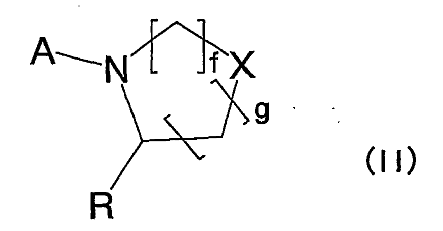 (wherein f is 1 or 2; g is 0, 1 or 2; X is -CH₂-, -O-, -S-, -SO-, -SO₂- or -NR₃- (R³ is a hydrogen atom or a C₁₋₆ alkyl group); R is a hydrogen atom, a cyano group, -CHO, -B(OH)₂, P(O)(O-R³), CC-R⁴, or CH=N-R⁵(R⁴ is a hydrogen atom, a fluorine atom, C₁₋₆ alkyl group, cyano group, nitro group, -OR³, -CO₂R³, or -COR³ (R³ indicates the same meaning as aforementioned); R⁵ is a phenyl group, hydroxyl group, -OR³, -OCOR³, or benzyloxy group (R³ indicates the same meaning as aforementioned); and A indicates an amino acid residue which may be substituted) described in WO 95/15309 etc.
   In the formula, the C₁₋₆ alkyl group indicated by the R³ includes, for example, methyl, ethyl, propyl, isopropyl, butyl, isobutyl, sec-butyl, t-butyl, pentyl, isopentyl, neopentyl, 1-ethylpropyl, hexyl, isohexyl, 1,1-dimethyl, 2,2-dimentylbutyl, 3,3-dimethylbutyl, 2-ethylbutyl and the like.
   The amino acid residue in "amino acid residue which may be substituted" indicated by A includes a group in which OH of a carboxyl group constituting α- or β-amino acids has been removed from these amino acids.
   Herein, α-amino acids include, for example, alanine, arginine, asparagine, aspartic acid, glutamine, glutamic acid, glycine, histidine, isoleucine, leucine, lycine, methionine, phenylalanine, proline, serine, threonine, tryptophan, tyrosine, valine, citrulline, ornithine, homocystein and the like. β-Amino acids include, for example, β-alanine, β-aminocyclopropanoic acid, β-aminocyclobutanoic acid, β-aminocyclopentanoic acid, β-aminocyclohexanoic acid, β-aminocycloheptanoic acid, β-aminocyclooctanoic acid. The β-amino acids may have unsaturated bonds in a carbon chain constituting the amino acids.
   The α- and β-amino acids as described above may be in any of D-, L-, and DL-forms, preferably in the natural type of L form.
   The said amino acid residue may have 1 or 2 substituents on an amino group constituting the amino acid or on the side chain of the amino acid.
   The said "substituted on the amino group" includes preferably a hydrocarbon group which may be substituted and a piperidinyl group which may be substituted.
   A hydrocarbon group in the "hydrocarbon group which may be substituted" includes, for example, a C₁₋₆ alkyl group, a C₃₋₁₂ cycloalkyl group, a C₂₋₆ alkenyl group, a C₃₋₁₂ cycloalkenyl group, a C₂₋₆ alkenyl group, a C₄₋₁₂ cycloalkadienyl group, a C₆₋₁₄ aryl group (preferably, phenyl group), a C₇₋₁₅ aralkyl group (preferably, benzyl group, phenethyl group), an adamantly group, a bicycle [2,2,1]heptyl group, a bicyclo[3,1,1]heptyl group and the like.
   The hydrocarbon group may have 1 to 3 substituents at substitutable positions, and such substitutions include, for example, a halogen atom (preferably, fluorine, chlorine); a cyano group; a hydroxyl group which may be substituted with an acyl group; a hydroxymethyl group; a C₁₋₆ alkoxy group which may be substituted with 1 to 3 halogen atoms (preferably fluorine); and an amino group which may be mono- or di-substituted with a C₆₋₁₄ aryl group which may be substituted or a heterocyclic group which may be substituted.
   Herein, an acyl group on the "hydroxyl group which may be substituted with an acyl group" includes, for example, the one exemplified as a substitutent on ring A in the said compound I-a.
   A C₆₋₁₄ aryl group in the "C₆₋₁₄ aryl group which may be substituted" includes, for example, a phenyl group or a naphthyl group.
   A heterocyclic group in the "heterocyclic group which may be substituted" includes, for example, a pyridiyl group, a pyrimidyl group, a pyrazyl group, a quinolyl group, an isoquinolyl group, a quinoxalyl group and the like.
   The C₆₋₁₄ aryl group and heterocyclic group may have 1 to 3 substituents at substitutable positions and such substituents include, for example, a halogen atom (preferably, fluorine, chlorine, bromine); a cyano group; a nitro group; a C₁₋₆ alkyl group; a C₁₋₆ alkoxy group which may be substituted with 1 to 3 halogen atoms (preferably fluorine); a carboxyl group; a carbamoyl group; a C₁₋₆ alkylsulfonyl group (preferably, methanesulfonyl group); an aminosulfonyl group which may be mono- or di-substituted with C₁₋₆ alkyl group (preferably diemethylaminosulfonyl group).
   Especially preferable for a substiutent in the said "hydrocarbon group which may be substituted" is a 5-nitro-2-pyridylamino group, 5-cyano-2-pyridylamino group, 2-pyrimidylamino group, 2-pyrazylamino group and the like.
   A substutent in the said "piperidinyl group which may be substituted" includes, for example, a C₁₋₆ alkyl group; a hydroxymethyl group, "a C₆₋₁₄ aryl group which may be substituted" and "a heterocyclic group which may be substituted" exemplified in the said "amino group which may be mono- or di-substituted with C₆₋₁₄ aryl group which may be substituted or the heterocyclic group which may be substituted." The number of substituents is, for example, 1 to 3.
   The said "substituent on the side chain of an amino acid" includes, for example, a hydrocarbon group which may be substituted, a hydroxyl group, a C₁₋₁₀ alkoxy group which may be substituted with 1 to 3 halogen atoms (preferably fluorine), an acyl group, and amino group which may be substituted and the like.
   Herein, a hydrocarbon in the said "hydrocarbon group which may be substituted" includes, for example, a C₁₋₁₀ alkyl group, a C₃-12 cycloalkyl group, a C₂₋₁₀ alkenyl group, a C₃₋₁₂ cycloalkenyl group and the like.
   The hydrocarbon group may have 1 to 3 substituents at substitutable positions, and such substituents include, for example, an amino group, a C₁₋₆ alkyl-carbonylamino group (preferably, acetylamino group), a hydroxyl group, a C₁₋₆ alkoxy group, a heterocyclic group (preferably, pyridyl group) and the like.
   The said "acyl group" is preferably a nitrogen-containing heterocyclic-carbonyl group which may be substituted. The "nitrogen-containing heterocyclic which may be substituted" includes nitrogen-containing heterocycles ((preferably, pyridine, pyridazine, pyrimidine, pyrazine, imidazole, pyrazole, thiazole, isothiazole, oxazole, isooxazole, etc.) which may have 1 to 3 substituents selected from, for example, a halogen atom (preferably, fluorine, chlorine, bromine), a cyano, group, a nitro goup, a C₁₋₆ alkyl group (e.g., trifluoromethyl group) which may be substituted with 1 to 3 halogen atoms (preferably fluorine), a C₁₋₆ alkoxy group, an amino group which may be mono- or di-substituted with C₁₋₆ alkyl group; a hydroxyl group, a carboxy group and a C₁₋₆ alkyl-oxycarbonyl group.
   A substituent in the said "amino group which may be substituted" includes a C₁₋₆ alkyl group, etc. which may have 1 tom 3 substituents selected from, for example, a carboxyl group, a carbamoyl group, a C₁₋₆ alkyl-oxycarbonyl group and a nitrogen-containing heterocyclic group (preferably, pyridyl). These substituents may be coupled with a hydroxyl group, a carboxyl group, an amino group and the like which is on the side chain of an amino acid.
   A suitable example of the compound represented by formula (II) includes N-(N'-substituted glycyl)-2-cyano-pyrrolidine derivatives such as (2S)-1-{{{2-[(5-cyanopyridine-2-yl)amino]ethyl}amino}acetyl}-2-cyano-pyrrolidine (DPP-728) (described in WO 98/19998) represented by the formula: 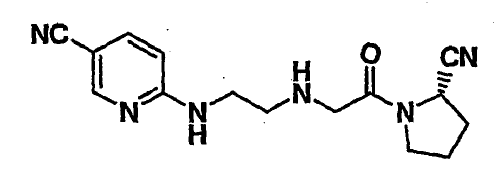 (2S)-1-{[(3-hydroxy-1-adamantyl)amino]acetyl}-2-cyanopyrrolidine (1-[(3-Hydroxy-adamant-1-ylamino)-acetyl]-pyrrolidine-2(S)-carbonitrile) (also known as LAF237 or vildagliptin) (described in WO 00/34241 represented by the formula: 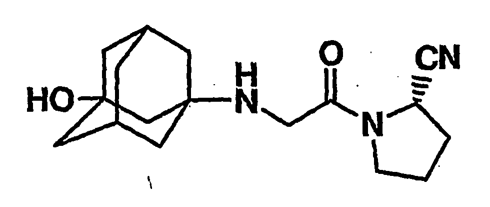 (2S)-1-{{{2-[(pyrimidine-2-ylpiperidine-4-yl-}amino}-acetyl-2-cyano-pyrrolidine (described in WO 02/30890).
   (2S)-1-{{{2-[(pyridine-2-yl)amino]ethyl}amino}acetyl}-2-cyanopyrrolidine and (S)-1-{1-[5-(N,N'-dimethylaminosulfonyl)-2-pyridylamino]-2-methyl-propylamino}acetyl-2-pyrrolidinecarbonitrile (K-361) (described in WO 02/51836); thiazoline or pyrrolidine derivatives such as L-threo-isoleucylthiazoline (P32/98), L-allo-isolencylthiazoline, L-threo-isoeuculpryrrolidine, L-allo-isoleucylpyrrolidine and L-valylpyrrolidine (described in WO 01/72290) represented by the formula 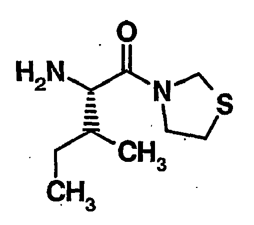
(3) N-substituted 2-cyanopyrrole and 2-cyanopyrroline derivatives described in WO 01/55105. Preferably, (S,S)-1-(2-amino-3,3-dimethylbutylyl)-2,5-dihydro-1H-pyrrole-2-carbonitrile.
(4) Heterocyclic compounds described in WO 02/02560. Preferably, 7-benzyl-8-[6-hydroxymethyl)-1,4-diazepam-1-yl]-1,3-dimethyl-3,7-dihydropurine-2,6-dione.
(5) Pyrrolidine derivatives of a condensed ring with cyclopropane described in WO 01/68603. Preferably, (1S,3S,5S)-2-[(2S)-2-amino-3,3-dimethylbutyryl]-3-cyano-2-azabicyclo[3,1,0]hexane.
(6) Proline derivatives described in WO 02/14271. Preferably, (2S)-1-[(2S,4S)-4-(3-chloro-4-cyanophenyl)amino-2-pyrrolidinylcarbonyl]-2-cyanopyrrolidine.
(7) Cyanopyrrolidine derivatives described in WO 02/38541. Preferably, (2S,4S)-1-[(2S,3S)-2-amino-3-methyl-pentanoyl]-2-cyano-4-fluoropyrrolidine, (2S,4S)-2-cyano-4-fluoro-1-[(1-hydroxymethyl)cyclopentylamino]acetypyrrolidine, and (2S,4S)-2-cyano-4-fluoro-1-[(1-hydroxy-3-adamanylamino]acetylpyrrolidine.
(8) Compounds such as P93/01 described in WO 02/02560, WO 03/055881, WO 03/040174, WO 03/037327, WO 03/035057, WO 03/035067, WO 03/024942, WO 03/024965, WO 03/004498, WO 03/004496, WO 03/000250, WO 03/002530, WO 03/002531, WO 03/002553, WO 03/000180, WO 03/000181, EP 1258476, WO 02/51836, WO 02/68420, US 6,432,969, etc.
(9) Another preferred DPP-4 inhibitor is the compound BMS-477118 disclosed in WO 2001068603 or U.S. Patent No. 6,395,767 (compound of example 60) also known as is (1S,3S,5S)-2-[(2S)-2-amino-2-(3-hydroxytricyclo[3.3.1.1^{3,7}]dec-1-yl)-1-oxoethyl]-2-azabicyclo[3.1.0]hexane-3-carbonitrile, benzoate (1:1) as depicted in Formula M of the patent application WO 2004/052850 on page 2, and the corresponding free base, (1S,3S,5S)-2-[(2-S)-2-amino-2- (3-hydroxy-tricyclo[3.3.1.1^{3,7}]dec-1-yl)-1-oxoethyl]-2-azabicyclo-[3.1.0]hexane-3-carbonitrile (M') and its monohydrate (M") as depicted in Formula M of the patent application WO 2004/652850 on page 3. The compound BMS-477118 is also known as saxagliptin.
(10) Another preferred inhibitor is the compound GSK23A disclosed in WO 03/002531 (example 9) also known as (2S,4S)- 1-((2R)-2-Amino-3-[(4-methoxybenzyl)sulfonyl]-3-methylbutanoyl)-4-fluoropyrrolidine-2-carbonitrile hydrochloride.
(11) Other preferred DPP-IV inhibitors are described in the patent application WO 03/004498 especially examples 1 to 33 and most preferably the compound of the formula 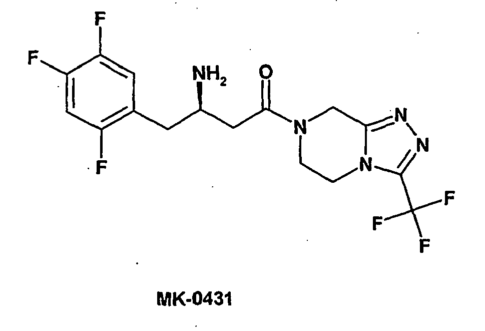 described by the example 7 and also known as MK-0431 or Sitagliptin (INN).
(12) Other preferred DPP-IV inhibitors are described in the patent applications WO 2004/037169 especially those described in the examples 1 to 48 and WO 02/062764 especially the described examples 1 to 293, even preferred are the compounds 3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide described on page 7 and also in the patent application WO2004/02418 especially in the reference examples 1 to 4.

Specific examples of hydroxypropyl methylcellulose include: Metholose SB-4 (commercial name, Shinetsu Chemical Industry Co. Ltd. made) (viscosity of 2 wt.% aqueous solution at 20°C: about 4 mPa·s), TC-5 RW (commercial name, Sinetsu Chemical Industry Co. Ltd. - made) (viscosity of 2 wt.% aqueous solution at 20°C: about 6 mPa·s), TC-5 S (commercial name, Shinetsu Chemical Industry Co. Ltd. - made) (viscosity of 2 wt.% aqueous solutionat 20°C: about 15 mPa·s), Metholose 60SH-50 (commercial name, Shinetsu Chemical Industry Co. Ltd. - made) (viscosity at 20°C: about 50 mPa·s), Metholose 65SH-50 (commercial name, Shinetsu Chemical Industry Co. Ltd. -made) (viscosity of 2 wt.% aqueous solution at 20°C: about 50 mPa·s), Metholose 90SH-100 (commercial name, Shinetsu Chemical Industry Co. Ltd. - made) (viscosity of 2 wt.% aqueous solution at 20°C: about 100 mPa·s), Metholose 90SH-100SR (commercial name, Shinetsu Chemical Industry Co. Ltd. - made) viscosity of 2 wt.% aqueous solution at 20°C: about 100 mPa·s), Metholose 65SH-400 (commercial name, Shinetsu Chemical Industry Co. Ltd. - made) (viscosity of 2 wt.% aqueous solution at 20°C: 400 mPa·s), Metholose 65SH-1500 (commercial name, Shinetsu Chemical Industry Co. Ltd. - made) (viscosity of 2 wt.% aqueous solution at 20°C: about 1500 mPa·s), Metholose 65SH-4000 (commercial name, Shinetsu Chemical Industry Co. Ltd. - made) (viscosity of 2 wt.% aqueous at 20°C: about 1000 mPa·s), Metholose 65SH-1000 (commercial name, Shinetsu Chemical Industry Co. Ltd. - made) (viscosity of 2 wt.% aqueous solution at 20 °C: about 1000 mPa·s), Metholose 90SH-1000 (commercial name, Shinetsu Chemical Industry Co. Ltd. made) (viscosity of 2 wt.% aqueous solution at 20°C: 4000 mPa·s), Metholose 90SH 1000SR (commercial name, Shinetsu Chemical Industry Co. Ltd. - made) (viscosity of 2 wt.% aqueous solution at 20 °C: about 4000 mPa·s), Metholose 90SH-30000 (commercial name, Shinetsu Chemical Industry Co. Ltd. - made) (viscosity of 2 wt.% % aqueous solution at 20°C: about 30000 mPa·s), Metholose 90SH-100000 (commercial name, Shinetsu Chemical Industry Co. Ltd. - made) (viscosity of 2 wt.% aqueous solution at 20°C: about 100000 mPa·s), and Metholose 90SH-100000SR (commercial name, Shinetsu Chemical Industry Co. Ltd. - made) (viscosity of 2 wt.% aqueous solution at 20 °C: about 100000 mPa·s).

"Sustained release preparation" or "modified release preparation" herein means a preparation wherein "the elution rate of the drug at 30 min after starting the test" is less than 85% when conducting, for example, the Second Method (paddle method) of the Elution Test Method in Japan Pharmacopocia using 900 mL of an appropriate test solution when the rotating counts of the drug in the preparation eluted by 100% into the test solution is below 1/3 the saturation solubility of the drug. Also, the conventional test solutions in the pharmaceutical technical field, e.g., water, a butter solution and the like are used. "preparation" means a pharmaceutical formulation or unit dosage form e.g. tablet, granule.

In addition, a preparation wherein the elution rate of a drug at 30 min after starting the test is not less than 85% when conducing the Second Method (paddle method) of the Elution Test Method in Japan Pharmacopoeia under conditions similar to the above is referred to as an immediate release preparation in this specification.

Dosage forms or unit dosage forms of the present sustained release preparation include, for example, an oral formulation such as a tablet, capsule (including a microcapsule), granule, or powder; and a parenteral formulation such as a suppository (e.g., rectal suppository, vaginal suppository, etc.), and these can be safely administered orally or parenterally, respectively. Of these forms, an oral formulation such as a tablet, capsule, or granule is preferred.

The sustained release preparation of the invention can be manufactured by mixing the DPP-IV inhibitor and hydrophilic polymer and molding them. Herein, the mixing and molding are performed according to conventional pharmaceutical methods.

Also, when performing the said mixing and molding, a pharmacologically acceptable carrier may be used.

Herein, the pharmacologically acceptable carriers include different types of conventional organic or inorganic carrier substances, for example, excipients, lusters, binders, disintegrators, and the like as base materials for the preparation. Further, additives for a preparation such as preservatives, antioxidants, coloring agents, and sweeteners may also be used as needed.

When the DPP-IV inhibitor used in the sustained release preparation of the invention is basic, an organic acid may be added to regulate elution behavior of the sustained release preparation. Generally, since solubility of a basic drug is greater in the acidic condition than in the neutral condition, the drug elution from the sustained release preparation may vary depending on the surrounding pH. In such a case, change of the drug elution property based on the surrounding pH can be reduced by use of an organic acid. Since the pH in the body may vary in individual patients, reducing changes of the drug elution based on the surrounding pH is extremely significant for obtaining a uniform drug effect for various patients.

The organic acids include, for example, citric acid, tartaric acid, ascorbic acid, malic acid, fumaric acid, malonic acid, succinic acid, maleic acid, aspartic acid, glutamic acid, etc. Of these, citric acid, tartaric acid, ascorbic acid, etc. are preferred.

The sustained release preparation of the invention has less toxicity and fewer side effects, accordingly it can be administered to mammals (e.g. humans, bovines, horses, dogs, cats, monkeys, mice, and rats) as a preventative and therapeutic agent for various diseases.

The sustained release preparation of the invention may be used as a preventative and therapeutic agent, for example, for diseases such as diabetes (e.g. type 1 diabetes, type 2 diabetes, pregnancy diabetes), hyperlipidemia (e.g. hypertriglyceridemia, hypercholesterolemia, hypoHDLemia, postprandial hyperlipidemia), arterial sclerosis, Impaired Glucose Tolerance (IGT), Impaired Fasting Glucose (IFG), Impaired Fasting Glycemia (IFG), and diabetic complications (e.g., neuropathy, nephropathy, retinopathy, cataract, angiopathy of large vessel, osteopenia, diabetic hyperosmolar coma, infectious diseases (e.g., respiratory infection, urinary tract infection, digestive system infection, dermal soft tissue infection, lower limb infection), diabetic gangrene, dry oral cavity, decrease of audition, cerebrovascular disease, peripheral circulation disorder) and as an agent for reducing blood sugar and the like.

The sustained release preparation of the invention may prevent Abnormal Tolerance, Impaired Fasting Glucose (IFG) or Impaired Fasting Glycemeia (IFG) from developing into diabetes.

Further, the sustained release preparation of the invention can be used to improve pancreatic (β-cell) function, pancreatic (β-cell) regeneration, acceleration of pancreatic (β-cell) regeneration, etc.

Since the DPP-IV inhibitor is an agent accelerating glucose-depending insulin secretion which exhibits selectively accelerating action on insulin secretion in a patient with high blood sugar (e.g., patients who have not less than 126 mg/dL of blood sugar value at fasting or not less than 140 mg/dL of 2-hour value after 75 g Oral Glucose Tolerance Test (75 g OGTT)). The sustained release preparation of the invention is useful as a safe preventative and therapeutic agent for diabetes with less risk for blood vessel complication, induction of hypoglycemia, etc., which are harmful effects of insulin.

While the dosing amount of the sustained release preparation of the present invention varies depending on the subject, administration route, target disease, etc., for oral administration to an adult patient with diabetes, for example, a single dose of the DPP-IV inhibitor, which is the active ingredient, should usually be about 0.01-100 mg/kg weight, preferably 0.05-30 mg/kg weight, more preferably 0.1-10 mg/kg weight, and ideally be administered once or twice a day. Preferably, the sustained release preparation should be administered to continuously obtain DPP-IV inhibitory action in the living body during from before a meal to at least 2 hours after a meal (preferably 4 hours after a meal).

The sustained release preparation's release duration of the DPP-IV inhibitor in the living body should preferably be 1 to 24 hours; more preferably, 2 to 14 hours.

Usually, when using a DPP-IV inhibitor to prevent or treat diabetes, it is necessary to take the DPP-IV inhibitor before every meal since GLP-1, which is a substrate of DPP-IV, is secreted upon intake of food. However, since the sustained release preparation of the invention can release the DPP-IV inhibitor over a long time, it exhibits sufficient inhibitory effect of DPP-IV, even when taken once a day.

The sustained release preparation of the invention may be used in combination with drugs (hereinafter, referred to as concomitant drugs) such as a therapeutic agent for diabetes, a therapeutic agent for diabetic complications, an antihyperlipidemia agent, a depressor, and anti-obesity agent, a diuretic, and an antithromotic agent. In this case, the medication time of the sustained release preparation of the invention and the comcomitant drug is not limited. Thus, these may be simultaneously administered to a subject, or administered at a different time, respectively. Further, the sustained release preparation of the invention and the concomitant drug may be administered as two preparations containing one active ingredient each, or administered as a single preparation containing both active ingredients.

The dosing amount of the concomitant drug can be appropriately selected based on the dose clinically used. The ratio of the sustained release preparation of the invention to the concomitant drug can be appropriately selected based on the subject, administration route, target disease, symptom, combination, etc. For example, when the subject is human, 0.01-100 parts by weight to 1 part by weight of the concomitant drug based on the DPP-IV inhibitor, which is an active ingredient of the sustained release preparation, may be used.

The said therapeutic agents for diabetes (antidiabetic) include, for example, insulin preparations (e.g., animal insulin preparations extracted from the pancreas of a bovine or swine; human insulin preparations synthesized in genetic engineering sing E. coli or yeast; zinc insulin; zinc protamine insulin; fragments or derivatives of insulin, (e.g., INS-1, etc.)), insulin-resistance-improving agents (e.g. Pioglitazone hydrochloride, Lociglytazone (maleate), GI-262570, Reglixane (JTT-501), Netoglitazone (MCC-555), YM-440, KRP-297, CS-011, FK-614, Ragaglitazar (NN-622), Tesaglitazar (AZ-242), DMS-298585, EML-16336, compounds described in WO 99/58510 (e.g., (E)-4-[4-(5-methyl-2-phenyl-4-oxazolylmehtoxy)benzyloxyimino]-4-phenylbutyric acid)), PPARγ agonist, PPARγ antagonist, PPARγ/αdual agonist, α-glucosidase inhibitors (e.g. Voglibose, Acarbose, Migritol, Emiglytate, biguanide agents (e.g., Phenformin, Metformin, Buformine or their salts) (e.g. hydrochloride, furmarate, succinate)), accelerators of insulin secretion (sulfonylurea agents (e.g., Tolbutamide, Glibenclamide, Gliclazide, Chlorpropamide, Tolazamide, Acetohexamide, Glyclopyramide, Glymepyride, Glypizide, Glybuzole, etc.), Repaglinide, Senaglinide, Nateglinide, Mitiglinide or their calcium salt hydrates), GLP-1 receptor agonists (e.g., GLP-1, NN-2211, AC-2993 (exedin-4), BIM-51077, Alb (8,35) h GLP-1 (7,37) NH₂), amyline agonists (e.g., Plamlintide, phosphotyrosine phosphatase inhibitors (e.g. vanadic acid), β3 agonists (e.g., CL-316243, SR-58611-A, UL-TG-307, SB-226552, AJ-9677, BMS-196085, AZ40140), inhibitors of neosugar (e.g., glycogen phosphorylase ihnhibitor, glucose-6-phosphatase inhibitor, glucagon antagonist, somatostatin receptor agonist), sodium-glucose cotransporter (SGLT) inhibitors (e.g., T-1095), etc.

The therapeutic agents for diabetic complications include inhibitors of aldose reductase (e.g., Tolrestat, Epalrestat, Zenarestat, Zopolrestat, Minalrestat, Phydarestat, SNK-860, CT-112), neurotrophy factor and its increasing agents (e.g., NGF, NT-3, BDNF, accelerators of neurotrophin production/secretion (e.g., 4-(4-chlorophenyl)-2-(2-methyl-1-imidazolyl)-5-[3-(2-methylphenoxy)propyl]oxazole etc.)) described in WO 01/14372), ACE inhibitors (e.g., ALT946, Pimagedine, N-phenacylthiazolium bromide (ALT766), (EXO-226), active oxygen-erasing agents (e.g., thiocitic acid), cerebrovascular dilators (e.g., Tiapride, Mexiletine).

The anti-hyperdilidemia agents include statin compounds which are inhibitors of cholesterol synthesis (e.g., Cerivastatin, Pravastatin, Simvastatin, Rovastatin, Atorvastatin, Fluevastatin, Itavastatin, or their salts (e.g., sodium salts)), inhibitors or squalene synthase (e.g., compounds described in WO 97/10224, for example, N-[[(3R,5S)-1-(3-acetoxy-2,2-dimethylpropyl)-7-chloro-5-(2,3-dimethoxyphenyl-2-oxo-1,2,3,5-terahydro-4,1-benzooxazepine-3-yl]acetyl]piperidine-4-azetic acid etc.), fibrate compounds (e.g., Benzafibrate, Clofibrate, Simfibrate, Clinofibrate), ACAT inhibitors (e.g., Avasimibe, Eflucimibe), anion exchange resins (e.g., Cholestyramine), Probucol, nicotinic acid drugs (e.g., nicomol, niceritrol), ethyl icosapentoate, plant sterols (soysterol), γ-oryzanol, etc.

The depressors include angiotensin converting enzyme inhibitors (e.g., Captropril, Enalapril, Delapril), angiotensin II antagonists (e.g., Candesartan, Cilexetil, Losartan, Eprosartan, Valsartan, Telmisartan, Irbesartan, Tasosartin), calcium antagonists (e.g., Manidipine, Nifedipine, Amlodipine, Efonidipine, Nicardipine), potassium channel opening drugs(e.g., Levcromakalim, L-27152, AL671, NIP-121), Clonidine, etc.

The anti-obesity agents include, for example, central anti-obesity agents (e.g., Dexphenfluramine, Phenfluramine, Phentelmine, Sibutramine, Anfepramone, Dexanfetamine, Mazindol, Phenylpropanolamine, Kobenzorex, pancreatic lipase inhibitors (e.g., Orlistat), β3 agonists (e.g., CL 316243, SR-58611-A, UL-TG-307, SB-226552, AJ-9677, BMS-196085, AZ40140), peptidyl anorectics (e.g., Leptin, Ciliary Neurotrophic Factor (CNTF)), cholecystokinin agonists (e.g., FPL-15849)), etc.

The diruetics include, for example, xanthine derivative (e.g., sodium salicylate theobromine, calcium salicylate theobromine), thiazide preparations (e.g. ethiazide, cyclopentiazide, trichlormethiazide, hydrochlorothiazide, hydroflumethiazide, bentylhydrochlorothiazide, penfluthizide, polythiaside, methichlothiazide), anti-aldosterone preparations (e.g., spylonoacetone, trimetellen), carbonic anhydrase inhibitors (e.g., acetazolamide), chlorbenzenesufonamide preparations (e.g., chlortalidone, meflucide, idapamide), azocernide, isosorbide, ethacrynic acid, pyrcladnide, bromethanide, flocemide, etc.

The antithrombotic agents include, for example, heparin (e.g., heparin sodium, heparin calcium, dalteparin sodium), warfarin (e.g., warfarin potasium), antithrombin agents (e.g., aragatroban), thrombolytics (e.g. urokinase, tisokinase, alteplase, nateplase, monteplase, pamiteplase), platelet aggregation inhibitors (e.g., flolopidine hydrochloride, cllostazol, ethyl icosapentoate, beraprost sodium, sarpogrelate hudrochloride), etc.

The concomitant drugs are preferably insulin preparations, insulin-resistance improving agents, α-glucosidase inhibitors, biguanide agents, insulin secretion accelerators (preferably sulonyl urea), etc.

Further, the present invention relates to "a drug comprising a combination of two or more preparations with DPP-IV inhibitor and different releasing rates fo the DPP-IV inhibitor."

Herein, "the DPP-IV inbibitor-containing preparation" may be any preparation containing the DPP-IV inhibitor, either a sustained release or immediate release preparation. In addition, the release control mechanism of the DPP-IV inhibitor in the "DPP-IV inhibitor-containing preparation" has no particular limitations, and the preparation may be any that releases the DPP-IV inhibitor; by passive diffusion accompanying degradation of the prepartion; by response to change of the surrounding pH; by the inner pressure of the swollen inside of the preparation caused by intake of the surrounding water; immediate release by the preparation's disintegration or dissolution, and the like.

Herein, "preparations that release the DPP-IV inhibitor by passive diffusion" include, for example, the sustained release preparation of the invention, (preferably, a matrix tablet using hydorphylic polymers (e.g., hyroxypropylmethylcellulose, hydroxypropylcellulose, and polyethyleneoxixde), a matrix tablet using lipophilic base materials (e.g., carnauba wax, hydrogenated castor oil, hydrogenated rape oil, polyglycerin fatty acid esters), a tablet or a granule coated with sustained release base materials (e.g., cellulose polymers such as ethylcellulose, acrylic acid polymers such as aminoalkyl methacrylate copolymer RS (Eudragit RS (commercial name, Rohm Pharma Co. Ltd. - made), ethyl acrylate-methyl methacrylate copolymer suspension (Eudragit NE (commercial name, Rohm Pharma Co., Ltd. made), etc.

The "preparations that release DPP-IV inhibitor from the preparation by degradation of the preparation" include, for example, a capsule containing polyglycolated glycerides (e.g. Gelucire10/13 (commercial name, GATTEFOSSE Co. Ltd. - made)), etc.

The "preparations that release DPP-IV inhibitor in response to change of the surrounding pH" include, for example, a tablet or granule coated with enteric base materials (acrylic acid polymers such as methacrylate copolymer (Eudragit L (commercial name, Rohm Pharma Co. Ltd. made)), methacrylate copolymer LD (Eudragit L-30D55 (commercial name, Rohm Pharma Co. Ltd. made)), methyacrylate copolymer S (Eudragit S (commercial name, Rohm Pharma Co. Ltd. - made)), etc.

The "preparations that release DPP-IV inhibitor by the inner pressure of the swollen inside of the preparation caused by intake of the surrounding water" include, for example an osmotic system, i.e., Concerta™ (Alza Co. Ltd., Fort Washington, PA), etc.

The "preparations that release DPP-IV inhibitor immediately by the preparation's disintegration or dissolution" include, for example, those that are obtained by mixing the DPP-IV inhibitor with a pharmacologically acceptable carrier and then carrying out molding. Herein, the pharmacologically acceptable carrier includes those similar to the sustained release preparation of the invention. Also, mixing and molding can be performed according to conventional pharmaceutical techniques.

The release control mechanism of the "two or more preparations containing DPP-IV inhibitors" constituting the present drug of the invention may be either same or different from each other. The "two ore more preparations containing DPP-IV inhibitors" may be a single preparation or plural preparations independent to each other. The single preparation includes a single capsule containing two ore more preparations containing the DPP-IV inhibitor; a multi-layer tablet (preferably a 2-layer tablet) or nucleated tablet having multiple release controlling parts, and the like.

The present drug of the invention should preferably be composed of a combination of a sustained release preparation containing a DPP-IV inhibitor and an immediate release preparation containing a DPP-IV inhibitor, and by adopting such a combination, the excellent DPP-IV inhibitory action can be obtained immediately after administration and be maintained over a long term.

While the content of the DPP-IV inhibitor in the sustained release preparation containing a DPP-IV inhibitor varies depending on the type of DPP-IV inhibitor, amount of preparation, etc., the content is, for example 20-30% by weight, preferably 25-35% by weight, and ideally 25% by weight.

Dosage forms of the DPP-IV inhibitor-containing preparation are similar to those of the sustained release preparation of the present invention.

The present drug of the invention has lower toxicity and fewer side effects, accordingly it can be administered to mammals (e.g., humans, bovines, horses, dogs, cats, monkeys, mice, and rats) as preventative and therapeutic agents of various diseases similarly to the said sustained release preparation of the invention.

The dosage of the present drug of the invention may be combined with the two or more preparations containing DPP-IV inhibitors at administration. Such a dosage form includes, for example, 1) administration as a single preparation of two ore more preparations containing DPP-IV inhibitors, 2) simultaneous administration as multiple preparations of two or more preparations containing DPP-IV inhibitors, 3) administration at different times of multiple preparations of two or more preparations contining DPP-IV inhibitors, etc.

While the dosing amount of the drug of the invention varies depending on the subject, administration route, target disease, etc., when orally administering it to an adult patient with diabetes, for example, the usual does of DPP-IV inhibitor, which is the active ingredient should be about 0.01-100 mg/kg weight, preferably 0.5 mg/kg weight, more preferably .1-10 mg/kg weight, and these amounts should ideally be administered once or twice a day. Preferably, the drug of the invention should be administered to continuously obtain action of the DPP-IV inhibitor in the living body from before a meal to at least 2 hours after a meal preferably to 4 hours after a meal.

The present drug of the invention may be used in combination with the concomitant drug similar to the case of the sustained release preparation of the present invention.

The present invention further relates to "the release-controlled preparation containing the DPP-IV inhibitor, which may decrease DPP-IV activity in blood plasma by 10 to 90% (preferably, 10 to 85%) 1 hr after administration", "the release-controlled preparation containing the DPP-IV inhibitor which may decrease DPP-IV activity in blood plasma by 10 to 90% (preferably, 10 to 85%) 8 hrs after administration", "the release-controlled preparation contining the DPP-IV inhibitor which may decrease DPP-IV activity in blood plasma by 10 5o 90% (preferably, 10 to 85%) 12 hrs after administration", "the release-controlled preparation containing the DPP-IV inhibitor, which may decrease DPP-IV activity in the blood plasma by 10 to 90% (preferably, 10 to 85% over 1 to 8 hrs after administration", "the release-controlled preparation containing the DPP-IV inhibitor, which may decrease DPP-IV activity in blood plasma by 10 to 90% (preferably, 10 to 85%) over 1 to 12 hrs after administration", and the like.

Herein, the blood plasma in "DPP-IV activity in blood plasma" means peripheral venous blood plasma. Although the DPP-IV activity and its decrease rate may vary depending on the type of blood plasma (e.g., venous, arterial or portal blood plasma), any release-controlled preparations belong to the present invention of "release-controlled preparation containing the DPP-IV inhibitor" as long as they can decrease DPP-IV activity in peripheral venous blood plasma by 10 to 90% (preferably 10 to 85%).

The present invention of the release-controlled preparation containing the DPP-IV inhibitor can decrease DPP-IV activity in blood plasma by 10 to 90%, preferably 10 to 85%, more preferably 10 to 80%, most preferably 15 to 75%.

The DPP-IV activity in blood plasma can be measured according to, for example, "the method of Raymond et al., Diabetes, Vol. 47, p. 1253- 1258, 1998." As long as the said decrease rate of the DPP-IV activity in blood plasma is within normal errors, it may be different from the said values (10, 15, 75, 80, 85, 90%). Further, the decrease rate of the DPP-IV activity in blood plasma may be different from the said values depending on the method of measuring DPP-IV activity in blood plasma. For example, if measuring conditions such as substrate type, substrate concentration, reaction time, diluted multiples of blood plasma, and the like of DPP-IV activity in blood plasma are different from those in the method described in the above literature, the decrease rate of the DPP-IV activity in blood plasma may be greater than those described above, for example, the value of 90% may be over 95%.

Among preparations containing the DPP-IV inhibitor of the present invention, the "release-controlled preparation containing the DPP-IV inhibitor" of the present invention is a preparation wherein release of the DPP-IV inhibitor is controlled.

Such a preparation is preferably the inventive sustained release preparation. Also, among the drugs of the invention, "a drug comprising a combination of sustained release preparation containing a DPP-IV inhibitor and an immediate release preparation containing a DPP-IV inhibitor" is preferable.

The release-controlled preparation containing the DPP-IV inhibitor of the present invention has lower toxicity and fewer side effects, accordingly it can be administered to mammals (e.g., humans, bovines, horses, dogs, cats, monkeys, mice, and rats) as preventative and therapeutic agents of various diseases similarly to the sustained release preparation of the invention.

While the dosing amount of the release-controlled preparation containing the DPP-IV inhibitor of the present invention varies depending on he .subject, administration route, target disease, etc., when orally administering it to an adult patient with diabetes, for example, the usual does of DPP-IV inhibitor, which is the active ingredient, should be about 0.01-100 mg/kg weight, preferably 0.05-30 mg/kg weight, more preferably 0.1-10 mg/kg weight, and ideally administered once or twice a day. Preferably, the release-controlled preparation containing the DPP-IV inhibitor of the present invention should be administered so that the action of the DPP-IV inhibitor in the living body is continuously obtained from before a meal to at least about 2 hours after a meal (preferably hours after a meal).

The release-controlled preparation containing the DPP-IV inhibitor of the present invention may be used in combination with a concomitant drug as in the case of the sustained release preparation of the invention.

Specific examples of a microcrystalline cellulose include, for example Avicel® PH102 and 101 (FMC Biopolymer, Philadelphia, PA).

Specific examples of a magnesium stearate include, for example Synpro® Magnesium Stearate NF-Vegetable Grade (Ferro Corp., Walton Hills, OH).

Specific examples of a lactose include, for example, Lactose DT and Lactose SD.

In addition, tablets often contain a diluent or filler which are added to increase the bulk weight of the blend resulting in a practical size for compression. One, two, three or more diluent and/or filler can be selected. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 55% or from about 15% to about 45% by weight of the composition. Most preferably the above described compositions comprise one or two fillers selected from microcrystalline cellulose such as Avicel PH 102 and lactose.

Lubricants are typically added to prevent the tableting materials from sticking to punches, minimize friction during tablet compression and allow for removal of the compressed tablet from the die. Such lubricants are commonly included in the final tablet mix in amounts usually about or less than 1% by weight. The lubricant component may be hydrophobic or hydrophilic. Examples of such lubricants include stearic acid, talc and magnesium stearate. Magnesium stearate reduces the friction between the die wall and tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix. The preferred lubricant, magnesium stearate is also employed in the formulation.

Preferably, the lubricant is present in the tablet formulation in an amount of from about 0.25% to about 6%; also preferred is a level of about 0.5% to about 4% by weight; and most preferably from about 0.1% to about 1% by weight. Other possible lubricants include talc, polyethylene glycol, silica and hardened vegetable oils. In an optional embodiment of the invention, the lubricant is not present in the formulation, but is sprayed onto the dies or the punches rather than being added directly to the formulation.

While the present invention is described in more detail in the following Experimental Details, the present invention is not limited to these and the uses of the invention may be varied as long as the scope is not exceeded.

### Embodiments of the Invention

The subject invention provides a pharmaceutical tablet formulation (Formulation C) comprising per unit dosage form e.g. per tablet the following ingredients:
(a) a DPP-IV inhibitor preferably vildagliptin, or a pharmaceutically acceptable salt thereof as an active ingredient,
(b) a hydroxypropyl methylcellulose with an apparent viscosity of 80,000 cP to 120,000 cP (nominal value 100,000 cP) when present in a 1% solution,
(c) and optionally a filler and/or a lubricant.

The subject invention provides a pharmaceutical tablet formulation (Formulation D) comprising per unit dosage form e.g. per tablet the following ingredients:
(a) 10-50% preferably 15-35% by weight on a dry weight basis of a DPP-IV inhibitor preferably vildagliptin, or a pharmaceutically acceptable salt thereof as an active ingredient,
(b) 20-60% preferably 30-50% by weight on a dry weight basis of a hydroxypropyl methylcellulose with an apparent viscosity of 80,000 cP to 120,000 cP (nominal value 100,000 cP) when present in a 1% solution,
(c) and optionally a filler and/or a lubricant.

The subject invention provides a pharmaceutical tablet formulation (Formulation E) comprising per unit dosage form e.g. per tablet the following ingredients:
(a) 10-50% preferably 15-35% by weight on a dry weight basis of a DPP-IV inhibitor preferably vildagliptin, or a pharmaceutically acceptable salt thereof as an active ingredient,
(b) 20-60% preferably 30-50% by weight on a dry weight basis of a hydroxypropyl methylcellulose with an apparent viscosity of 80,000 cP to 120,000 cP (nominal value 100,000 cP) when present in a 1% solution;
(c) a filler; and
(d) a lubricant.

The subject invention provides a pharmaceutical tablet formulation (Formulation F) comprising per unit dosage form e.g. per tablet the following ingredients:
(a) 10-50% preferably 15-35% by weight on a dry weight basis of a DPP-IV inhibitor preferably vildagliptin, or a pharmaceutically acceptable salt thereof as an active ingredient,
(b) 20-60% preferably 30-50% by weight on a dry weight basis of a hydroxypropyl methylcellulose with an apparent viscosity of 80,000 cP to 120,000 cP (nominal value 100,000 cP) when present in a 1% solution;
(c) 15-55% preferably 25-45% by weight on a dry weight basis of a pharmaceutically acceptable filler; and optionally
(d) 0.1-10% preferably 0.1-3% by weight on a dry weight basis of a pharmaceutically acceptable lubricant.

The subject invention provides a pharmaceutical tablet formulation (Formulation G) comprising per unit dosage form e.g. per tablet the following ingredients:
(a) 10-50% preferably 15-35% by weight on a dry weight basis of a DPP-IV inhibitor preferably vildagliptin, or a pharmaceutically acceptable salt thereof as an active ingredient,
(b) 20-60% preferably 30-50% by weight on a dry weight basis of a hydroxypropyl methylcellulose with an apparent viscosity of 80,000 cP to 120,000 cP (nominal value 100,000 cP) when present in a 1% solution;
(c) 15-55% preferably 25-45% by weight on a dry weight basis of one or two pharmaceutically acceptable fillers selected from lactose and microcrystalline cellulose ; and optionally
(d) 0.1-10% preferably 0.1-3% by weight on a dry weight basis of a pharmaceutically acceptable lubricant such as magnesium stearate.

The subject invention provides a pharmaceutical tablet formulation (Formulation H) comprising per unit dosage form e.g. per tablet the following ingredients:
(a) 10-50% preferably 15-35% by weight on a dry weight basis of a DPP-IV inhibitor preferably vildagliptin, or a pharmaceutically acceptable salt thereof as an active ingredient,
(b) 20-60% preferably 30-50% by weight on a dry weight basis of a hydroxypropyl methylcellulose with an apparent viscosity of 80,000 cP to 120,000 cP (nominal value 100,000 cP) when present in a 1% solution;
(c) 25-40% by weight on a dry weight basis of one or two pharmaceutically acceptable fillers selected from lactose and microcrystalline cellulose ; and optionally
(d) 0.1-10% preferably 0.1-3% by weight on a dry weight basis of a pharmaceutically acceptable lubricant.

A pharmaceutical tablet formulation as described herein wherein the filler is selected from lactose and microcrystalline cellulose.

A pharmaceutical tablet formulation (Formulation I) as described herein comprising at leat two fillers preferably lactose and microcrystalline cellulose. In an embodiment, the lactose is present in an amount from 1 to 8% preferably 1 to 5% by weight and microcrystalline cellulose is present in an amount from 25 to 35% by weight

A pharmaceutical tablet formulation (Formulation J) as described herein wherein 20-30% by weight on a dry weight basis of a DPP-IV inhibitor especially LAF237 is contained in the formulation.

A pharmaceutical tablet formulation (Formulation K) as described herein wherein the hydroxypropyl methyl cellulose is present in an amount from 34% to 46% preferably from 38% to 42% by weight.

A formulation as described herein wherein the compound (DPP-IV inhibitor) is 1-[(3-hydroxy-adamant-1-ylamino)-acetyl]-pyrrolidine-2(S)-carbonitrile (LAF237 or vildagliptin).

A formulation (Formulation L), capsule, granule or tablet as described herein wherein vildagliptin (LAF237) is a crystalline form of vildagliptin (crystal "Form A"), characterized by an X-ray diffraction pattern with peaks at about 16.6°, 17.1°, 17.2° +/- 0.3 degrees 2-theta or characterized by an X-ray diffraction pattern with peaks at about 12.0°, 13.5°, 16.6°, 17.1°, 17.2°, 20.1°, 22.5°, 27.4°, 28.1° , +/- 0.3 degrees 2-theta. Such a crystal form is described in the International patent application No. PCT/US2006/001473

The term "vildagliptin" covers any crystalline, form, preferably the crystal form "A" of vildagliptin.

The subject invention provides a pharmaceutical tablet formulation comprising per unit dosage form e.g. per tablet the following ingredients:
(a) a compound as an active ingredient, wherein the compound has a formula: 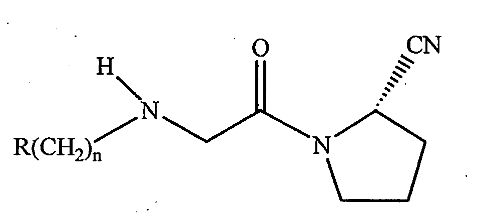 wherein R is substituted adamantyl and n is an integer from 0 to 3, preferably vildagliptin; or a pharmaceutically acceptable salt thereof;
(b) a hydroxypropyl methylcellulose with an apparent viscosity of 80,000 cP to 120,000 cP (nominal value 100,000 cP) when present in a 1% solution;
(c) a microcrystalline cellulose; and
(d) a magnesium stearate.

In an embodiment, relative to the weight of the formulation:
(a) the compound e.g. vildagliptin is present in an amount from 20% to 30% by weight;
(b) the hydroxypropyl methylcellulose is present in an amount from 30% to 50% by weight;
(c) the microcrystalline cellulose is present in an amount from 25% to 35% by weight; and
(d) the magnesium stearate is present in an amount from 0.1% to 3% by weight.

In another embodiment, relative to the weight of the formulation:
(a) the compound e.g. vildagliptin is present in an amount of about 25% by weight;
(b) the hydroxypropyl methylcellulose is present in an amount of about 40% by weight;
(c) the microcrystalline cellulose is present in an amount of about 30% by weight; and
(d) the magnesium stearate is present in an amount of about 1% by weight.

In a further embodiment, the tablet formulation further comprising a lactose. In an embodiment, the lactose is present in an amount from 1% to 8% by weight. In an additional embodiment the lactose is in an amount of about 4% by weight.

In an embodiment, the DPP-IV inhibitor is selected from [S]-1-[2-(5-cyano-2-pyridinylamino)ethylamino]acetyl-2-pyrolidine carbonitrile monohydrochloride, vildagliptin, L-threo-isoleucyl thiazolidine, Sitagliptin, Saxagliptin,3-(aminomethyl)-2-isobuthyl-1-oxo-4-phenyl-1,2-dihydro-6-isoquinolinecarboxamide and 2-{[3-(aminomethyl)-2-isobuthyl-4-phenyl-1-oxo-1,2-dihydro-6-isoquinolyl]oxy}acetamide and optionally pharmaceutical salts thereof.

In an embodiment, the compound (DPP-IV inhibitor) is 1-[2-[(5-cyanopyridin-2-yl)amino]ethylamino]acetyl-2-cyano(S)-pyrrolidone or a pharmaceutically acceptable salt thereof.

In a preferred embodiment, the invention concerns a composition or unit dosage form as described herein wherein the compound (DPP-IV inhibitor) is vildagliptin or a salt thereof. In yet another embodiment, the compound vildagliptin is present in the formulation or a pharmaceutically acceptable salt thereof.

The subject invention also provides a pharmaceutical tablet formulation (Formulation A), comprising per 400 mg tablet the following ingredients:
(a) vildagliptin or a pharmaceutically acceptable salt thereof in an amount of about 100 mg;
(b) a hydroxypropyl methylcellulose in an amount of about 160 mg, wherein the hydroxypropyl methylcellulose has an apparent viscosity of 80,000 cP to 120,000 cP (nominal value 100,000 cP) when present in a 1% solution;
(c) a microcrystalline cellulose in an amount of 120 mg;
(d) a lactose in an amount of about 16 mg; and
(e) a magnesium stearate in an amount of 4 mg.

The subject invention also provides a pharmaceutical tablet formulation (Formulation B), comprising per 600 mg tablet the following ingredients:
(a) vildagliptin or a pharmaceutically acceptable salt thereof in an amount of about 150 mg;
(b) a hydroxypropyl methylcellulose in an amount of about 240 mg, wherein the hydroxypropyl methylcellulose has an apparent viscosity of 80,000 cP to 120,000 cP (nominal value 100,000 cP) when present in a 1% solution;
(c) a microcrystalline cellulose in an amount of 180 mg;
(d) a lactose in an amount of about 24 mg; and
(e) a magnesium stearate in an amount of 6 mg.

In an embodiment, the pharmaceutical tablet formulation comprises combining the ingredients in the amounts recited above.

The subject invention also provides a method of inhibiting dipeptidyl peptidase IV activity in a subject comprising administering to the subject an amount of the above-mentioned pharmaceutical tablet formulations effective to inhibit the activity of dipeptidyl peptidase IV in the subject.

In an embodiment, the subject is a human being.

The subject invention also provides a method of treating a condition alleviated by dipeptidyl peptidase IV inhibition in a subject suffering from the condition comprising administering to the subject a therapeutically effective dose of any of the herein-mentioned pharmaceutical tablet formulations, unit dosage forms e.g. capsule, tablet, compressed table, direct compressed tablets, granule.

The subject invention also covers the use of any of the herein-mentioned pharmaceutical tablet formulations, unit dosage forms e.g. capsule, tablet, compressed table, direct compressed tablets, granule, for the manufacture of a medicament for the treatment of a condition alleviated by dipeptidyl peptidase IV inhibition in a subject suffering from the condition comprising administering to the subject a therapeutically effective dose of any of the above-mentioned pharmaceutical tablet formulations.

The subject invention also covers the use of any of the herein-mentioned pharmaceutical tablet formulations, unit dosage forms e.g. capsule, tablet, compressed table, direct compressed tablets, granule for the treatment of a condition alleviated by dipeptidyl peptidase IV inhibition in a subject suffering from the condition comprising administering to the subject a therapeutically effective dose of any of the above-mentioned pharmaceutical tablet formulations.

Preferably the condition alleviated by dipeptidyl peptidase IV inhibition is selected from non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels.

In an embodiment, the condition is non-insulin-dependent diabetes mellitus.

In another embodiment, the condition is obesity, arthritis, or osteoporosis. In a further embodiment, the subject is a human being.

The subject invention further provides a method of treating a condition alleviated by dipeptidyl peptidase IV inhibition in a subject suffering from the condition comprising administering to subject a therapeutically effective amount of any of the above-mentioned pharmaceutical tablet formulations in combination with a therapeutically effective dose of an anti-diabetic or arthritis drug.

In an embodiment, the subject is a human being.

The subject invention also provides a pharmaceutical tablet formulation comprising per unit dosage form e.g. per tablet the following ingredients:
(a) a compound as an active ingredient, wherein the compound has a formula: 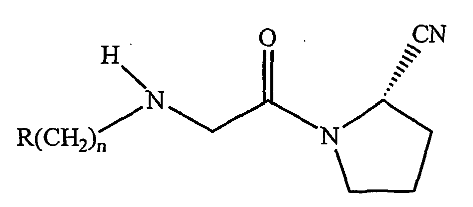 wherein R is substituted adamantyl and n is an integer from 0 to 3; or a pharmaceutically acceptable salt thereof; and
(b) a hydroxypropyl methylcellulose with an apparent viscosity of 80,000 cP to 120,000 cP (nominal value 100,000 cP) when present in a 1% solution.

In one embodiment, the herein described pharmaceutical tablet formulations also contains a filler. In one embodiment, the filler is lactose. In another embodiment, the filler is microcrystalline cellulose. In a further embodiment the filler is microcrystalline cellulose and lactose.

In another embodiment, the herein described pharmaceutical tablet formulations also contains a lubricant. In one embodiment, the lubricant is preferably magnesium stearate.

In yet another embodiment, relative to the weight of the formulation, the hydroxypropyl methyl cellulose is present in the herein described formulations in an amount from 30% to 50% by weight.

In an embodiment, the hydroxypropyl methyl cellulose is present in the herein described formulations in an amount from 34% to 46% preferably from 38% to 42% by weight.

In a further embodiment, the present invention also concerns the herein described pharmaceutical tablet formulation, wherein in the unit dosage form, the ratio of the weight of vildagliptin to the weight of hydroxypropyl methylcellulose is of 0.16 to 2.5, preferably 0.3 to 1.16 or 0.4 to 1.

Preferably the herein expressed ratios or % are expressed on a dry weight basis.

In a further embodiment, the present invention also concerns the herein described pharmaceutical tablet formulation, wherein in the unit dosage form, vildagliptin is present in an amount of 100 mg to 200 mg, preferably 100 mg, 150 mg, or 200 mg or the corresponding amount of any of its salt.

The herein described pharmaceutical tablet formulation can also be used in any unit dosage form, for example, an oral formulation such as a tablet (including multilayer tablets), capsule (including a microcapsule, capsules containing several e.g. two compartments), granule, or powder; or a parenteral formulation such as a suppository or any other pharmaceutical delivery system. Preferably the herein described pharmaceutical tablet formulation are used in the form of capsule, granule or tablet.

In a further embodiment, the present invention also concerns a pharmaceutical multilayer tablet wherein the herein described pharmaceutical tablet formulation, represents one of the tablet layers. This can be done as a multilayer tablet (one over the other) or as a coating one layer surrounding the other one.

Preferably the present invention also concerns a pharmaceutical multilayer tablet wherein the herein described pharmaceutical tablet formulation, represents one layer and a formulation comprising a further active ingredient represent the second layer and/or third layer. Preferably the further active ingredient is a glitazone (e.g. pioglitazone or rosiglitazone) or metformin. Preferably the further or second layer is an immediate release formulation. The herein described pharmaceutical tablet formulation can represent the middle layer and the immediate release formulation comprising a further active ingredient can represent the two external layers. The herein described pharmaceutical tablet formulation can represent the central layer and the immediate release formulation comprising a further active ingredient can represent the surrounding coating layer.

In a further embodiment, the present invention also concerns a pharmaceutical tablet obtained by compression of the herein described pharmaceutical tablet formulation.

In a further embodiment, the present invention also concerns the above pharmaceutical tablet obtained by compression of the herein described pharmaceutical tablet formulation, wherein the herein described pharmaceutical tablet formulation is subject to roller compaction before compression into tablet.

In a further embodiment, the present invention also concerns the above pharmaceutical tablet wherein the hardness range is of between 10 to 13Kp for the 100 mg vildagliptin tablet, and between 11 to 25Kp for the 150 mg vildagliptin tablet.

In the development of the herein described pharmaceutical compositions, the applicant has discovered that the compressed tablets especially direct compressed tablet is particularly advantageous if the particles comprising the DPP-IV inhibitor especially LAF237, have a particle size distribution of less than 250 µm preferably between 10 to 250 µm.

Thus in a further embodiment the present invention concerns the herein described pharmaceutical tablet formulations, wherein the dispersion contains particles comprising DPP-IV inhibitor preferably LAF237, in free form or in acid addition salt form, and wherein at least 60%, preferably 80% and most preferably 90% of the particle size distribution in the formulation is less than 250 µm or preferably between 10 to 250 µm.

The present invention concerns the herein described pharmaceutical tablet formulations, wherein the dispersion contains particles comprising DPP-IV inhibitor preferably LAF237, in free form or in acid addition salt form, and wherein at least 60%, preferably 80% and most preferably 90% of the particle size distribution in the formulation is greater than 10 µm.

The term "wherein at least 60%, preferably 80% and most preferably 90%" means at least 60%, preferably at least 80% and most preferably at least 90%.

The term "wherein at least at least 25%, preferably 35% and most preferably 45%" means at least 25%, preferably at least 35% and most preferably at least 45%.

In particular the present invention concerns the herein described pharmaceutical tablet formulations, wherein the dispersion contains particles comprising DPP-IV inhibitor preferably LAF237, in free form or in acid addition salt form, and wherein at least 25%, preferably 35% and most preferably 45% of the particle size distribution in the formulation is between 50 to 150 µm.

The selected particle size distribution provides an acceptable dissolution profile, as well as acceptable degrees of hardness, friability, Tablet breaking strength, Dispersion Quality and resistance to chipping, as well as a proper disintegration time, required flow and cohesive properties necessary to obtain an acceptable solid dosage form, Improved manufacturing robustness, required compactibility.

Particle size of drug, e.g. LAF237 particles size, is controlled by crystallisazion, drying and/or milling/sieving (non limiting examples are described below). Particle size can also be comminuted using roller compaction and milling/sieving.

Producing the right particle size is well known and described in the art such as in "Pharmaceutical dosage forms: volume 2, 2nd edition, Ed.: H.A.Lieberman, L-Lachman, J.B.Schwartz (Chapter 3: SIZE REDUCTION)".

Multiple particle sizes have been studied and it has been discovered that the herein described specific size range provides unexpected good results for direct compaction.

### PARTICLE SIZE DISTRIBUTION ESTIMATION BY ANALYTICAL SIEVING:

Particle size distribution is measured using Sieve analysis, Photon Correlation Spectroscopy or laser diffraction (international standart ISO 13320-1), or electronic sensing zone, light obstruction, sedimentation or microscopy which are procedures well known by the person skilled in the art. Sieving is one of the oldest methods of classifying powders by particle size distribution. Such methods are well known and described in the art such as in any analytical chemistry text book or by the United State Pharmacopeia's (USP) publication USP-NF (2004 - Chapter 786 - (The United States Pharmacopeial Convention, Inc., Rockville, MD)) which describes the US Food and Drug Administration (FDA) enforceable standards. The used techniques are e.g. described in Pharmaceutical dosage forms: volume 2, 2nd edition, Ed.: H.A.Lieberman, L.Lachman, J.B.Schwartz is a good example. It also mentions (page 187) additional methods: Electronic sensing zone, light obstruction, air permeation, sedimentation in gas or liquid.

In an air jet sieve measurement of particle size, air is drawn upwards, through a sieve, from a rotating slit so that material on the sieve is fluidised. At the same time a negative pressure is applied to the bottom of the sieve which removes fine particles to a collecting device. Size analyses and determination of average particle size are performed by removal of particles from the fine end of the size distribution by using single sieves consecutively. See also "Particle Size Measurement", 5th Ed. , p 178, vol. 1; T. Allen, Chapman & Hall, London, UK, 1997, for more details on this. For a person skilled in the art, the size measurement as such is thus of conventional character.

The herein described formulations can also be in the form of a layer in a multi or 2-layer tablet.

The herein described formulations can also be in the form of capsules, tablets, compressed tables, direct compressed tablets, granules.

The DPP-IV inhibitor compounds especially vildagliptin, useful in this invention are hygroscopic, presents stability problems, and are not inherently compressible into tablets. Consequently, there is a need to provide a free-flowing and cohesive composition capable of being compressed preferably directly, into strong tablets with an acceptable *in vitro* dissolution, pharmacokinetic, and pharmacodynamic profile.

The Paddle method to measure the drug dissolution rate (% of release) is used with 1000ml of 0.01N HCl. Such methods are well known and described in the art such as in any analytical chemistry text book or by the United State Pharmacopeia's (USP) publication USP-NF (2004 - Chapter 711) which describes the US Food and Drug Administration (FDA) enforceable standards.

In a further embodiment, the present invention concerns two pharmaceutical tablet formulations comprising respectively 100 mg or 150 mg of vildagliptin and providing a vildagliptin release profile particularly adapted for providing an improved pharmacokinetic profile. The resulting pharmacokinetic profile result in an improved treatment quality of the patient.

Thus, the present invention concerns;
1) a pharmaceutical tablet formulation comprising 100 mg of vildagliptin or a salt thereof, preferably compressed in the form of a sustained release tablet, wherein;
   - between 10% and 16% preferably between 11% and 15% of vildagliptin is released after 0.5 hour,
   - between 18% and 24% preferably between 19% and 23% of vildagliptin is released after 1 hour,
   - between 30% and 36% preferably between 31% and 35% of vildagliptin is released after 2 hours,
   - between 46% and 52% preferably between 47% and 51% of vildagliptin is released after 4 hour,
   - between 58% and 64% preferably between 59% and 63% of vildagliptin is released after 6 hours,
   - between 67% and 73% preferably between 68% and 72% of vildagliptin is released after 8 hours,
   - between 74% and 80% preferably between 75% and 79% of vildagliptin is released after 10 hours,
   - between 80% and 86% preferably between 81% and 85% of vildagliptin is released after 12 hours,
   - between 91% and 97% preferably between 92% and 96% of vildagliptin is released after 18 hours,
   - between 95% and 100% preferably between 96% and 100% of vildagliptin is released after 24 hours.
2) a pharmaceutical tablet formulation comprising 150 mg of vildagliptin or a salt thereof, preferably compressed in the form of a sustained release tablet, wherein;
   - between 3.8% and 9.8% preferably between 4.8% and 8.8% of vildagliptin is released after 0.25 hour,
   - between 8.1% and 14.1% preferably between 9.1% and 13.1% of vildagliptin is released after 0.5 hour,
   - between 14.7% and 20.7% preferably between 15.7% and 19.7% of vildagliptin is released after 1 hours,
   - between 25.3% and 31.3% preferably between 26.3% and.30.3% of vildagliptin is released after 2 hour,
   - between 40.9% and 46.9% preferably between 41.9% and 45.9% of vildagliptin is released after 6 hours,
   - between 62.1% and 68.1% preferably between 63.1% and 67.1% of vildagliptin is released after 8 hours,
   - between 76.5% and 82.5% preferably between 77.5% and 81.5% of vildagliptin is released after 10 hours,
   - between 83.5% and 89.5% preferably between 84.5% and 88.5% of vildagliptin is released after 12 hours,
   - between 88.5% and 94.5% preferably between 89.5% and 93.5% of vildagliptin is released after 18 hours.

For the above described two pharmaceutical tablet formulations the Paddle method to measure the drug dissolution rate (% of release)has been used.

Preferably the above described two pharmaceutical tablet formulations comprise a hydroxypropyl methylcellulose, preferably between 20% and 60%, between 30% and 50% by weight on a dry weight basis of a hydroxypropyl methylcellulose.

Preferably the above described two pharmaceutical tablet formulations comprise a hydroxypropyl methylcellulose with an apparent viscosity of 80,000 cP to 120,000 cP (nominal value 100,000 cP) when present in a 1% solution, preferably between 20% and 60%, or between 30% and 50% by weight on a dry weight basis of a hydroxypropyl methylcellulose with an apparent viscosity of 80,000 cP to 120,000 cP (nominal value 100,000 cP) when present in a 1% solution.

Preferably the above described two pharmaceutical tablet formulations comprising 150 mg or 100 mg of vildagliptin are in the form of a tablet or multilayer tablet.

Preferably the above described two pharmaceutical tablet formulations comprising 150 mg or 100 mg of vildagliptin comprise any of the herein described pharmaceutical tablet formulations.

Preferably the above described two pharmaceutical tablet formulations are in the form of a tablet obtained by compression of a pharmaceutical tablet formulation as herein described, wherein the pharmaceutical tablet formulation is subject to roller compaction before compression into tablet.

Preferably the above described pharmaceutical tablets comprise 100 mg of vildagliptin or a salt thereof wherein the tablet hardness range is of between 10 to 13Kp.

Preferably the above described pharmaceutical tablets comprise 150 mg of vildagliptin or a salt thereof wherein the tablet hardness range is of between 11 to 25Kp.

In a further aspect, the present invention concerns the use of the herein described pharmaceutical formulations, capsules, tablets, compressed tables, direct compressed tablets, granules for the treatment of conditions, such as non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels.

In a further aspect, the present invention concerns a method of treating conditions, such as non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels, comprising administering to a warm-blooded animal in need thereof a therapeutically effective amounts of a pharmaceutical tablet formulation, capsules, tablets, compressed tables, direct compressed tablets, granules as herein described.

Preferably, the condition is selected from such as non-insulin-dependent diabetes mellitus, obesity, calcitonin-osteoporosis, Heart Failure, Impaired Glucose Metabolism, IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels.

In a further aspect, the present invention concerns a process for preparing a tablet, in unit dosage form, which comprises:
(a) blending a pharmaceutical tablet formulation as described herein above,
(b) compressing the formulation prepared during step (a) to form the compressed tablet in unit dosage form.

In a further aspect, the present invention concerns a process for preparing a tablet, in unit dosage form, which comprises:
(a) blending a pharmaceutical tablet formulation as described herein above,
(b) roller compacting the formulation prepared during step (a)
(c) compressing the formulation prepared during step (b) to form the compressed tablet in unit dosage form.

In a further aspect, the present invention concerns a process for preparing a tablet, in unit dosage form, which comprises:
(d) blending a pharmaceutical tablet formulation as described herein above,
(e) roller compacting the formulation prepared during step (a) with a compaction force comprised between 10 and 16 KN or any of the herein described preferred compaction forces,
(f) compressing the formulation prepared during step (b) to form the compressed tablet in unit dosage form.

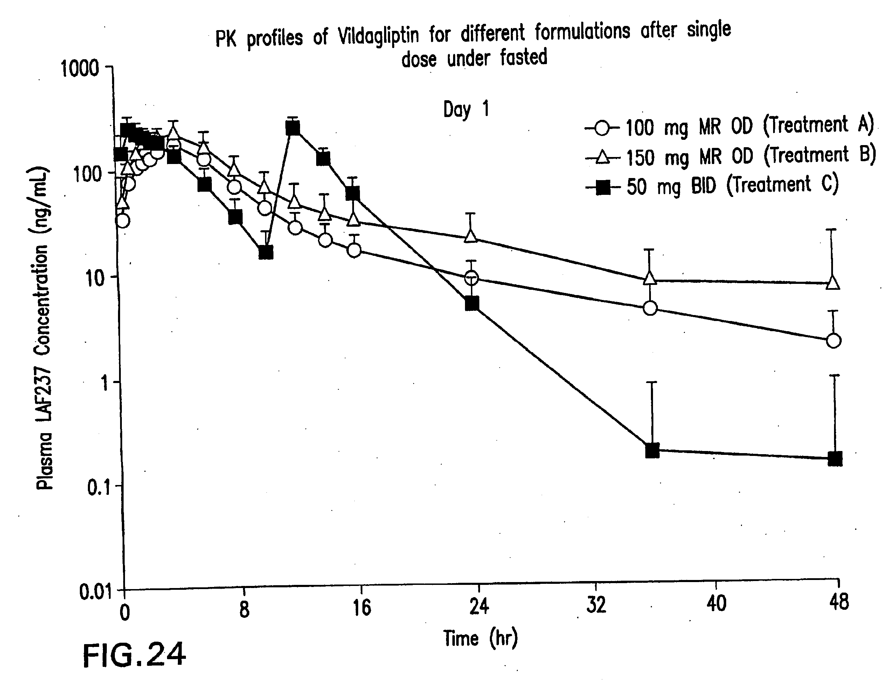

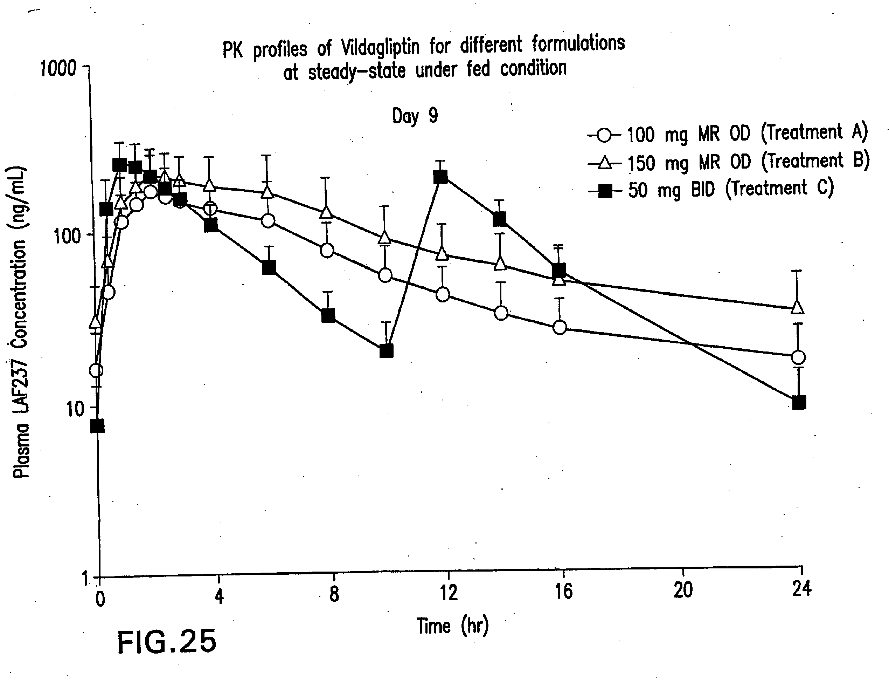

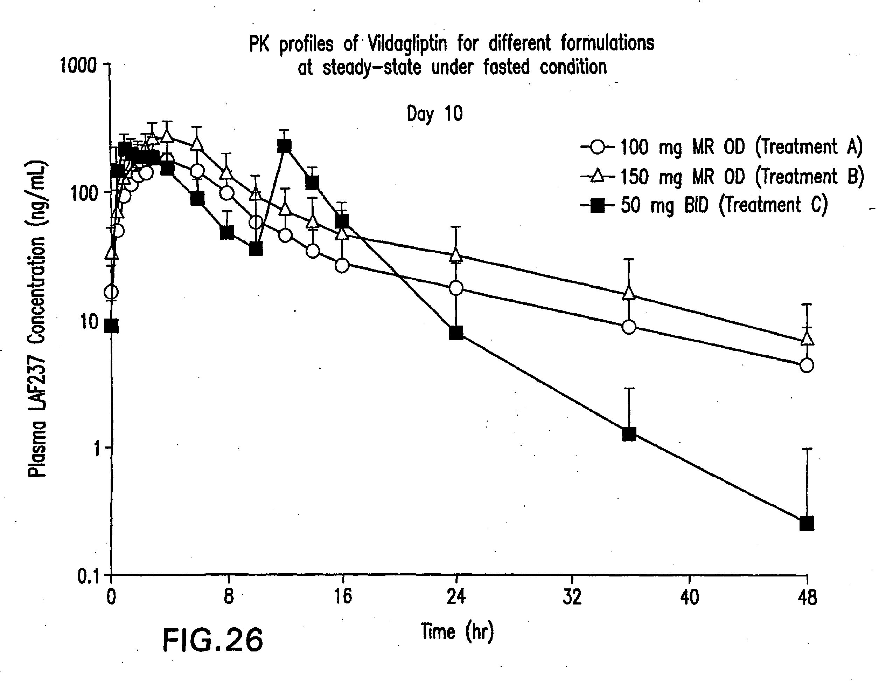

In another embodiment, the present invention provides a 1.00 mg vildagliptin sustained release solid oral pharmaceutical dosage form which is;
i-1) a solid oral pharmaceutical dosage form comprising about 100 mg of vildagliptin free base, or a respective amount of a pharmaceutically acceptable salt thereof, and a carrier medium, said dosage form providing an arithmetic mean maximum plasma concentration of vildagliptin ranging from about 15.8 ng/mL +./-6.85 ng/mL to about 173 ng/mL +/- 52 ng/mL between about 0.5 and about 16 hours following oral administration of said dosage form in a patient not treated with vildagliptin before said administration and wherein patient is under fasted conditions (day 1 of the above described study) ,and/or
i-2) a solid oral pharmaceutical dosage form comprising about 100 mg of vildagliptin free base, or a respective amount of a pharmaceutically acceptable salt thereof, and a carrier medium, said dosage form providing an arithmetic mean maximum plasma concentration of vildagliptin ranging from about 26.3 ng/mL +/-13.1 ng/mL to about 175 ng/mL +/- 62.5 ng/mL between about 0.5 and about 16 hours following administration of said dosage form on day 9 in a patient treated with said dosage form once a day since day 1 and wherein said patient has been served an ADA breakfast within 30 minutes of the morning administration of said dosage form (day 9 of the above described study), and/or
i-3) a solid oral pharmaceutical dosage form comprising about 100 mg of vildagliptin free base, or a respective amount of a pharmaceutically acceptable salt thereof, and a carrier medium, said dosage form providing an arithmetic mean maximum plasma concentration of vildagliptin ranging from about 26.9 ng/mL +/-14.1 ng/mL to about 186 ng/mL +/- 80.6 ng/mL between about 0.5 and about 16 hours following administration of said dosage form on day 10 in a patient treated with said dosage form once a day since day 1 and wherein said patient is under fasted conditions (day 10 of the above described study), and/or
ii-1) a solid oral dosage form comprising about 100 mg vildagliptin free base, or a respective amount of a pharmaceutically acceptable salt thereof, and a carrier medium, said dosage form providing an arithmetic mean AUC_{(0-inf)} of vildagliptin ranging from about 1073 to about 1825 ng·h/mL i.e. 1449 ng·h/mL +/- 376 ng·h/mL following oral administration of said dosage form , in a patient not treated with vildagliptin before said administration and wherein said patient is under fasted conditions (day 1 of the above described study), and/or
ii-2) a solid oral dosage form comprising about 100 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1001 to about 1977 ng·h/mL i.e. 1489 ng·h/mL +/- 488 ng·h/mL following oral administration of said dosage form , on day 9 in a patient treated with said dosage form once a day since day 1 and wherein said patient has been served an ADA breakfast within 30 minutes of the morning administration of said dosage form (day 9 of the above described study), and/or
ii-3) a solid oral dosage form comprising about 100 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1103 to about 2173 ng·h/mL i.e. 1638 ng·h/mL +/- 535 ng·h/mL following oral administration of said dosage form , on day 10 in a patient treated with said dosage form once a day since day 1 and wherein said patient is under fasted conditions (day 10 of the above described study), and/or
iii-1) a solid oral dosage form comprising about 100 mg vildagliptin free base, or a respective amount of a pharmaceutically acceptable salt thereof, and a carrier medium, said dosage form providing an arithmetic mean tₘₐₓ of vildagliptin of 3.61 hr +/- 1.44 hr following oral administration of said dosage form , in a patient not treated with vildagliptin before said administration and wherein said patient is under fasted conditions (day 1 of the above described study), and/or
iii-2) a solid oral dosage form comprising about 100 mg vildagliptin free base, or a respective amount of a pharmaceutically acceptable salt thereof, and a carrier medium, said dosage form providing an arithmetic mean tₘₐₓ of vildagliptin of 2.59.hr +/- 1.4 hr following oral administration of said dosage form , on day 9 in a patient treated with said dosage form once a day since day 1 and wherein said patient has been served an ADA breakfast within 30 minutes of the morning administration of said dosage form (day 9 of the above described study), and/or
iii-3) a solid oral dosage form comprising about 100 mg vildagliptin free base, or a respective amount of a pharmaceutically acceptable salt thereof, and a carrier medium, said dosage form providing an arithmetic mean tₘₐₓ of vildagliptin of 3.74 hr +/- 1.44 hr following oral administration of said dosage form , on day 10 in a patient treated with said dosage form once a day since day 1 and wherein said patient is under fasted conditions (day 10 of the above described study), and/or
iv-1) a solid oral dosage form comprising about 100 mg vildagliptin free base, or a respective amount of a pharmaceutically acceptable salt thereof, and a carrier medium, said dosage form providing an arithmetic mean Cₘₐₓ of vildagliptin of 205 ng/ml +/- 47 ng/ml following oral administration of said dosage form , in a patient not treated with vildagliptin before said administration and wherein said patient is under fasted conditions (day 1 of the above described study), and/or
iv-2) a solid oral dosage form comprising about 100 mg vildagliptin free base, or a respective amount of a pharmaceutically acceptable salt thereof, and a carrier medium, said dosage form providing an arithmetic mean Cₘₐₓ of vildagliptin of 200 ng/ml +/- 64 ng/ml following oral administration of said dosage form , on day 9 in a patient treated with said dosage form once a day since day 1 and wherein said patient has been served an ADA breakfast within 30 minutes of the morning administration of said dosage form (day 9 of the above described study), and/or
iv-3) a solid oral dosage form comprising about 100 mg vildagliptin free base, or a respective amount of a pharmaceutically acceptable salt thereof, and a carrier medium, said dosage form providing an arithmetic mean Cₘₐₓ of vildagliptin of 245 ng/ml +/- 68 ng/ml following oral administration of said dosage form , on day 10 in a patient treated with said dosage form once a day since day 1 and wherein said patient is under fasted conditions (day 10 of the above described study), and/or
v-1) a solid oral dosage form comprising about 100 mg vildagliptin free base, or a respective amount of a pharmaceutically acceptable salt thereof, and a carrier medium, said dosage form providing an arithmetic mean % inhibition of DPP-IV activity over 24 hours of 85.64% +/- 12.76% following oral administration of said dosage form , in a patient not treated with vildagliptin before said administration and wherein said patient is under fasted conditions (day 1 of the above described study), and/or
v-2) a solid oral dosage form comprising about 100 mg vildagliptin free base,or a respective amount of a pharmaceutically acceptable salt thereof, and a carrier medium, said dosage form providing an arithmetic mean % inhibition of DPP-IV activity over 24 hours of 87.78% +/- 16.37% following oral administration of said dosage form , on day 9 in a patient treated with said dosage form once a day since day 1 and wherein said patient has been served an ADA breakfast within 30 minutes of the morning administration of said dosage form (day 9 of the above described study), and/or
v-3) a solid oral dosage form comprising about 100 mg vildagliptin free base, or a respective amount of a pharmaceutically acceptable salt thereof, and a carrier medium, said dosage form providing an arithmetic mean % inhibition of DPP-IV activity over 24 hours of 90.20% +/- 7.35% following oral administration of said dosage form , on day 10 in a patient treated with said dosage form once a day since day 1 and wherein said patient is under fasted conditions (day 10 of the above described study), and/or
vi-1) a solid oral dosage form comprising about 100 mg vildagliptin free base, or a respective amount of a pharmaceutically acceptable salt thereof, and a carrier medium, said dosage form providing a pharmacokinetic profile as substantially depicted in figure 24, following oral administration of said dosage form , in a patient not treated with vildagliptin before said administration and wherein said patient is under fasted conditions (day 1 of the above described study), and/or
vi-2) a solid oral dosage form comprising about 100 mg vildagliptin free base, or a respective amount of a pharmaceutically acceptable salt thereof, and a carrier medium, said dosage form providing a pharmacokinetic profile as substantially depicted in figure 25, following oral administration of said dosage form , on day 9 in a patient treated with said dosage form once a day since day 1 and wherein said patient has been served an ADA breakfast within 30 minutes of the morning administration of said dosage form (day 9 of the above described study), and/or
vi-3) a solid oral dosage form comprising about 100 mg vildagliptin free base, or a respective amount of a pharmaceutically acceptable salt thereof, and a carrier medium, said dosage form providing a pharmacokinetic profile as substantially depicted in figure 26, following oral administration of said dosage form , on day 10 in a patient treated with said dosage form once a day since day 1 and wherein said patient is under fasted conditions, (day 10 of the above described study).

Preferably the 100 mg vildagliptin sustained release solid oral pharmaceutical dosage form comprises a hydroxypropyl methylcellulose, preferably between 20% and 60%, between 30% and 50% or between 34% and 46% by weight on a dry weight basis of a hydroxypropyl methylcellulose.

Preferably the 100 mg vildagliptin sustained release solid oral pharmaceutical dosage form comprises a hydroxypropyl methylcellulose with an apparent viscosity of 80,000 cP to 120,000 cP (nominal value 100,000 cP) when present in a 1% solution, preferably between 20% and 60%, or between 30% and 50% or between 34% and 46% by weight on a dry weight basis of a hydroxypropyl methylcellulose with an apparent viscosity of 80,000 cP to 120,000 cP (nominal value 100,000 cP) when present in a 1% solution.

Preferably the 100 mg vildagliptin sustained release solid oral pharmaceutical dosage form comprises any of the hereinabove described pharmaceutical tablet formulations.

Preferably the 100 mg vildagliptin sustained release solid oral pharmaceutical dosage form comprises a pharmaceutical tablet formulations selected from the herein described formulations A, B, C, D, E, F, G, H, I, J, K, or L.

In another embodiment, the present invention provides a 150 mg vildagliptin sustained release solid oral pharmaceutical dosage form which is;
i-a) a solid oral pharmaceutical dosage form comprising about 150 mg of vildagliptin free base, or a respective amount of a pharmaceutically acceptable salt thereof, and a carrier medium, said dosage form providing an arithmetic mean maximum plasma concentration of vildagliptin ranging from about 30.7 ng/mL +/-21.9 ng/mL to about 223 ng/mL +/- 77.3 ng/mL between about 0.5 and about 16 hours following oral administration of said dosage form in a patient not treated with vildagliptin before said administration and wherein patient is under fasted conditions (day 1 of the above described study), and/or
i-b) a solid oral pharmaceutical dosage form comprising about 150 mg of vildagliptin free base, or a respective amount of a pharmaceutically acceptable salt thereof, and a carrier medium, said dosage form providing an arithmetic mean maximum plasma concentration of vildagliptin ranging from about 48.7 ng/mL +/-25.8 ng/mL to about 223 ng/mL +/- 99.7 ng/mL between about 0.5 and about 16 hours following administration of said dosage form on day 9 in a patient treated with said dosage form once a day since day 1 and wherein said patient has been served an ADA breakfast within 30 minutes of the morning administration of said dosage form (day 9 of the above described study), and/or
i-c) a solid oral pharmaceutical dosage form comprising about 150 mg of vildagliptin free base, or a respective amount of a pharmaceutically acceptable salt thereof, and a carrier medium, said dosage form providing an arithmetic mean maximum plasma concentration of vildagliptin ranging from about 44.6 ng/mL +/-28.5 ng/mL to about 263 ng/mL +/- 84.4 ng/mL between about 0.5 and about 16 hour following administration of said dosage form on day 10 in a patient treated with said dosage form once a day since day 1 and wherein said patient is under fasted conditions (day 10 of the above described study), and/or
ii-a) a solid oral dosage form comprising about 150 mg vildagliptin free base, or a respective amount of a pharmaceutically acceptable salt thereof, and a carrier medium, said dosage form providing an arithmetic mean AUC_{(0-inf)} of vildagliptin ranging from about 1346 to about 3196 ng-h/mL i.e. 2271 ng·h/mL +/- 925 ng·h/mL following oral administration of said dosage form in a patient not treated with vildagliptin before said administration and wherein said patient is under fasted conditions (day 1 of the above described study), and/or
ii-b) a solid oral dosage form comprising about 150 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1277 to about 3207 ng·h/mL i.e. 2242 ng·h/mL +/- 965 ng·h/mL following oral administration of said dosage form , on day 9 in a patient treated with said dosage form once a day since day 1 and wherein said patient has been served an ADA breakfast within 30 minutes of the morning administration of said dosage form (day 9 of the above described study), and/or
ii-c) a solid oral dosage form comprising about 150 mg vildagliptin free base, or a respective amount of a pharmaceutically acceptable salt thereof, and a carrier medium, said dosage form providing an arithmetic mean AUC(₀₋₂₄₎ of vildagliptin ranging from about 1643 to about 3273 ng·h/mL i.e. 2458 ng·h/mL +/- 815 ng·h/mL following oral administration of said dosage form , on day 10 in a patient treated with said dosage form once a day since day 1 and wherein said patient is under fasted conditions (day 10 of the above described study), and/or
iii-a) a solid oral dosage form comprising about 150 mg vildagliptin free base, or a respective amount of a pharmaceutically acceptable salt thereof, and a carrier medium, said dosage form providing an arithmetic mean tₘₐₓ of vildagliptin of 3.57 hr +/- 1.17 hr following oral administration of said dosage form , in a patient not treated with vildagliptin before said administration and wherein said patient is under fasted conditions (day 1 of the above described study), and/or
iii-b) a solid oral dosage form comprising about 150 mg vildagliptin free base, or a respective amount of a pharmaceutically acceptable salt thereof, and a carrier medium, said dosage form providing an arithmetic mean tₘₐₓ of vildagliptin of 2.87 hr +/- 1.59 hr following oral administration of said dosage form , on day 9 in a patient treated with said dosage form once a day since day 1 and wherein said patient has been served an ADA breakfast within 30 minutes of the morning administration of said dosage form (day 9 of the above described study), and/or
iii-c) a solid oral dosage form comprising about 150 mg vildagliptin free base, or a respective amount of a pharmaceutically acceptable salt thereof, and a carrier medium, said dosage form providing an arithmetic mean tₘₐₓ of vildagliptin of 4.13 hr +/- 1.24 hr following oral administration of said dosage form , on day 10 in a patient treated with said dosage form once a day since day 1 and wherein said patient is under fasted conditions (day 10 of the above described study), and/or
iv-a) a solid oral dosage form comprising about 150 mg vildagliptin free base, or a respective amount of a pharmaceutically acceptable salt thereof, and a carrier medium, said dosage form providing an arithmetic mean Cₘₐₓ of vildagliptin of 257 ng/ml +/- 59 ng/ml following oral administration of said dosage form, in a patient not treated with vildagliptin before said administration and wherein said patient is under fasted conditions (day 1 of the above described study), and/or
iv-b) a solid oral dosage form comprising about 150 mg vildagliptin free base, or a respective amount of a pharmaceutically acceptable salt thereof, and a carrier medium, said dosage form providing an arithmetic mean Cₘₐₓ of vildagliptin of 272 ng/ml +/- 111 ng/ml following oral administration of said dosage form , on day 9 in a patient treated with said dosage form once a day since day 1 and wherein said patient has been served an ADA breakfast within 30 minutes of the morning administration of said dosage form (day 9 of the above described study), and/or
iv-c) a solid oral dosage form comprising about 150 mg vildagliptin free base, or a respective amount of a pharmaceutically acceptable salt thereof, and a carrier medium, said dosage form providing an arithmetic mean Cₘₐₓ of vildagliptin of 308 ng/ml +/- 91 ng/ml following oral administration of said dosage form , on day 10 in a patient treated with said dosage form once a day since day 1 and wherein said patient is under fasted conditions (day 10 of the above described study), and/or
v-a) a solid oral dosage form comprising about 150 mg vildagliptin free base, or a respective amount of a pharmaceutically acceptable salt thereof, and a carrier medium, said dosage form providing an arithmetic mean % inhibition of DPP-IV activity over 24 hours of 90.04% +/- 11.91% following oral administration of said dosage form , in a patient not treated with vildagliptin before said administration and wherein said patient is under fasted conditions (day 1 of the above described study), and/or
v-b) a solid oral dosage form comprising about 150 mg vildagliptin free base, or a respective amount of a pharmaceutically acceptable salt thereof, and a carrier medium, said dosage form providing an arithmetic mean % inhibition of DPP-IV activity over 24 hours of 90.4% +/- 17.50% following oral administration of said dosage form, on day 9 in a patient treated with said dosage form once a day since day 1 and wherein said patient has been served an ADA breakfast within 30 minutes of the morning administration of said dosage form (day 9 of the above described study), and/or
v-c) a solid oral dosage form comprising about 150 mg vildagliptin free base, or a respective amount of a pharmaceutically acceptable salt thereof, and a carrier medium, said dosage form providing an arithmetic mean % inhibition of DPP-IV activity over 24 hours of 91.64% +/- 8.47% following oral administration of said dosage form , on day 10 in a patient treated with said dosage form once a day since day 1 and wherein said patient is under fasted conditions (day 10 of the above described study), and/or
vi-a) a solid oral dosage form comprising about 150 mg vildagliptin free base, or a respective amount of a pharmaceutically acceptable salt thereof, and a carrier medium, said dosage form providing a pharmacokinetic profile as substantially depicted in figure 24, following oral administration of said dosage form , in a patient not treated with vildagliptin before said administration and wherein said patient is under fasted conditions (day 1 of the above described study), and/or
vi-b) a solid oral dosage form comprising about 150 mg vildagliptin free base, or a respective amount of a pharmaceutically acceptable salt thereof, and a carrier medium, said dosage form providing a pharmacokinetic profile as substantially depicted in figure 25, following oral administration of said dosage form , on day 9 in a patient treated with said dosage form once a day since day 1 and wherein said patient has been served an ADA breakfast within 30 minutes of the morning administration of said dosage form (day 9 of the above described study), and/or
vi-c) a solid oral dosage form comprising about 150 mg vildagliptin free base, or a respective amount of a pharmaceutically acceptable salt thereof, and a carrier medium, said dosage form providing a pharmacokinetic profile as substantially depicted in figure 26, following oral administration of said dosage form on day 10 in a patient treated with said dosage form once a day since day 1 and wherein said patient is under fasted conditions (day 10 of the above described study).

Preferably the 150 mg vildagliptin sustained release solid oral pharmaceutical dosage form comprises a hydroxypropyl methylcellulose, preferably between 20% and 60%, between 30% and 50% or between 34% and 46% by weight on a dry weight basis of a hydroxypropyl methylcellulose.

Preferably the 150 mg vildagliptin sustained release solid oral pharmaceutical dosage form comprises a hydroxypropyl methylcellulose with an apparent viscosity of 80,000 cP to 120,000 cP (nominal value 100,000 cP) when present in a 1% solution, preferably between 20% and 60%, or between 30% and 50% or between 34% and 46% by weight on a dry weight basis of a hydroxypropyl methylcellulose with an apparent viscosity of 80,000 cP to 120,000 cP (nominal value 100,000 cP) when present in a 1% solution.

Preferably the 150 mg vildagliptin sustained release solid oral pharmaceutical dosage form comprises any of the hereinabove described pharmaceutical tablet formulations.

Preferably the 150 mg vildagliptin sustained release solid oral pharmaceutical dosage form comprises a pharmaceutical tablet formulations selected from the herein described formulations A, B, C, D, E, F, G, H, I, J, K or L.

Any of the above described 100 mg or 150 mg vildagliptin sustained release solid oral pharmaceutical dosage form wherein;
i) the formulation is a matrix formulation containing a pharmaceutically acceptable hydrophilic polymer which can retard diffusion of vildagliptin,
ii) the solid oral pharmaceutical dosage form is a compressed tablet, and optionally
iii) the elution rate of vildagliptin at 30 minutes after starting the test is less than 30% when conducting the Paddle method.

"pharmaceutically acceptable hydrophilic polymer which can retard diffusion of an active ingredient" are known in the art e.g. Hydroxypropyl-methylcelluose.

### Experimental Details

### I. Pharmaceutical Development and Manufacture

### A. Selected Formulation and Process

### Composition

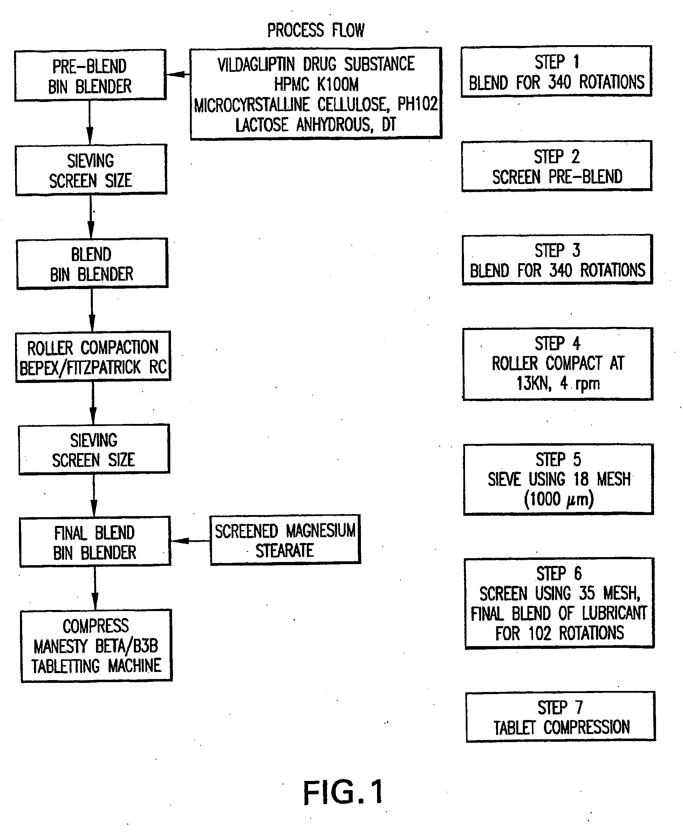

A dry blend process with roller compaction was developed for direct compression, as shown in the process flow diagram (Figure 1). Two dosage strengths, 100 mg and 150 mg of vildagliptin, were selected for further development and are shown in Tables 2-1 and 2-2. This process utilizes a common dry blend for the two strengths.

**Table 2-1 Composition per 100mg dosage form unit for Market Formulation containing 40% HPMC K100M**

| **Product name: vildagliptin Tab 100mg** | | | | |
|---|---|---|---|---|
| Basis number/ Variant: 3768652.003 | | | | |
| **Shape /size /color /imprint:** 11 mm FFBE (round)/white to off-white | | | | |
| **Global material no.** | **Component** | **Composition per unit (mg)** | **Testing monograph** | **Function** |
| 146082 | vildagliptin | 100 | Novartis | Drug Substance |
| 103266 | Microcrystalline cellulose, PH102 | 120 | Ph. Eur., NF | Tablet filler |
| 103282 | Lactose, anhydrous DT | 16 | Ph. Eur., NF | Tablet filler |
| 151580 | Hydroxypropylmethylcelluose K100M | 160 | Ph. Eur., NF | Controlled release polymer |
| 100217 | Magnesium stearate | 4 | Ph. Eur., NF | Tablet lubricants |
| **Total weight** | | **400** | | |

**Table 2-2 Composition per 150mg dosage form unit for Market Formulation containing 40% HPMC K 100M**

| **Product name: vildagliptin Tab 150mg** | | | | |
|---|---|---|---|---|
| Basis number/ Variant: 6001732.001 | | | | |
| **Shape /size /color /imprint:** 17 x 6.7mm ovaloid/white to off-white | | | | |
| **Global material no.** | **Component** | **Composition per unit (mg)** | **Testing monograph** | **Function** |
| 146082 | vildagliptin | 150 | Novartis | Drug Substance |
| 103266 | Microcrystalline cellulose, PH102 | 180 | Ph. Eur., NF | Tablet filler |
| 103282 | Lactose, anhydrous DT | 24 | Ph. Eur., NF | Tablet filler |
| 151580 | Hydroxypropylmethylcelluose K100M | 240 | Ph. Eur., NF | Controlled release polymer |
| 100217 | Magnesium stearate | 6 | Ph. Eur., NF | Tablet lubricant |
| **Total weight** | | **600** | | |

### Process

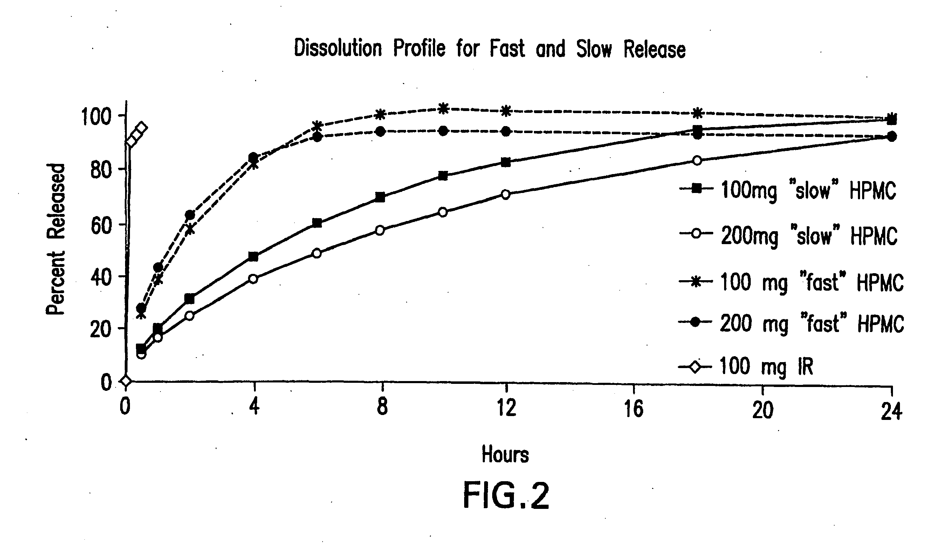

The selected manufacturing process is shown in figure 2-1.

### B. Formulation and Alternative Variants

Several formulation variants were evaluated as potential clinical service forms (CSF) in order to provide the requested release rate profile. The term clinical service forms (CSF) means a formulation adapted for administration to a patient. Several types of release rate profiles were studied; in particular a slow and fast. The profile rate was determined by the amount and type of polymer selected and/or tablet technology. The Market Formulation (MF) selected is a specific slow release rate profile (see Section I.A.)particularly adapted for a once a day dosage regimen and production of compressed tablets. Examples of the fast release rate formulations are shown in Tables 2-3 to 2-7 and alternate formulations with slow release profiles are shown in Tables 2-8 to 2-10.

**Table 2-3 Composition per 100mg dosage form unit for fast release profile containing 20% HPMC K100LV**

| **Product name: vildagliptin Tab 100mg** | | | | |
|---|---|---|---|---|
| **Basis number/ Variant: 3768652.001** | | | | |
| **Shape /size /color /imprint:** /white to off-white | | | | |
| **Global material no.** | **Component** | **Composition per unit (mg)** | **Testing monograph** | **Function** |
| 146082 | vildagliptin | 100 | Novartis | Drug Substance |
| 103266 | Microcrystalline cellulose, PH102 | 170 | Ph. Eur., NF | Tablet filler |
| 103282 | Lactose, anhydrous DT | 46 | Ph. Eur., NF | Tablet filler |
| 151580 | Hydroxypropymethylcelluose K100LVP | 80 | Ph. Eur., NF | Controller release polymer |
| 100217 | Magnesium Stearate | 4 | Ph. Eur., NF | Tablet lubricant |
| **Total weight** | | **400** | | |

**Table 2-4 Composition per 200mg dosage form unit for fast release profile containing 20% HPMC K100LV**

| **Product name: vildagliptin Tab 200mg** | | | | |
|---|---|---|---|---|
| **Basis number/ Variant: 3768660.002** | | | | |
| **Shape /size /color /imprint:** /white to off-white | | | | |
| **Global material no.** | **Component** | **Composition per unit (mg)** | **Testing monograph** | **Function** |
| 146082 | vildagliptin | 200 | Novartis | Drug Substance |
| 103266 | Microcrystalline cellulose, PH102 | 340 | Ph. Eur., NF | Tablet filler |
| 103282 | Lactose, anhydrous DT | 92 | Ph. Eur., NF | Tablet filler |
| 151580 | Hydroxypropylmethylcelluose K100LVP | 160 | Ph. Eur., NF | Controlled release polymer |
| 100217 | Magnesium Stearate | 8 | Ph. Eur., NF | Tablet lubricant |
| **Total weight** | | **800** | | |

**Table 2-5 Composition per 200mg dosage form unit for fast release profile containing 20% HPMC E10M**

| **Product name: vildagliptin Tab 200mg** | | | | |
|---|---|---|---|---|
| **Basis number/ Variant:** | | | | |
| **Shape /size /color /imprint:** /white to off-white | | | | |
| **Global material no.** | **Component** | **Composition per unit (mg)** | **Testing monograph,** | **Function** |
| 146082 | vildagliptin | 200 | Novartis | Drug Substance |
| 103266 | Microcrystalline cellulose, PH102 | 242 | Ph. Eur., NF | Tablet filler |
| 103282 | Lactose, anhydrous DT | 32 | Ph. Eur., NF | Tablet filler |
| 151580 | Hydroxypropylmethylcelluose, E10M | 120 | Ph. Eur., NF | Controlled release polymer |
| 100217 | Magnesium stearate | 6 | Ph. Eur., NF | Tablet lubricant |
| **Total weight** | | **600** | | |

The concentration of HPMC E10M was varied from 20% to 35% to match the target release rate profile. A combination of HPMC E10 (15%) and HPMC K100M (10%) was also evaluated for release rate.

**Table 2-6 Composition per 100mg dosage form unit for fast release profile containing X% polyox**

| **Product name: vildagliptin Tab 100mg** | | | | |
|---|---|---|---|---|
| **Basis number/ Variant:** | | | | |
| **Shape /size /color limprint:** /white to off-white | | | | |
| **Global material no.** | **Component** | **Composition per unit (mg)** | **Testing monograph** | **Function** |
| 146082 | vildagliptin | 100 | Novartis | Drug Substance |
| 103266 | Microcrystalline cellulose, PH102 | 170 | Ph. Eur., NF | Tablet filler |
| 103282 | Lactose, anhydrous DT Polyox ?? | 46 | Ph. Eur., NF | Tablet filler Controlled release polymer |
| 100217 | Magnesium Stearate | 4 | Ph. Eur., NF | Tablet lubricant |
| **Total weight** | | **400** | | |

Bi-layer tablet technology was evaluated using a 50mg immediate release (IR) tablet in one layer, and an 150mg MR as the second layer as shown in Table 2-7.

**Table 2-7 Composition per 200mg dosage form unit for bi-layer tablet containing HPMC K100M**

| **Product name: vildagliptin Tab 200mg** | | | | |
|---|---|---|---|---|
| **Basis number/ Variant:** **NA Shape /size /color /imprint:** /white to off-white | | | | |
| **Global material no.** | **Component** | **Composition per unit (mg)** | **Testing monograph** | **Function** |
| First Layer (IR) 146082 | vildagliptin | 50 | Novartis | Drug Substance |
| 103266 | Microcrystalli ne cellulose, PH102 | 96 | Ph. Eur., NF | Tablet filler |
| 103282 | Lactose, anhydrous DT | 48 | Ph. Eur., NF | Tablet filler |
| | Explotab | 4 | Ph. Eur., NF | Disintegra nt |
| | Magnesium Stearate | 2 | Ph. Eur., NF Lubricant | |
| Total | | 200 | | |
| Second Layer (MR) 146082 | 1-[(3-Hydroxy- adamant-1-ylamino)-acetyl]- pyrrolidine-2(S)-carbonitrile | 150 | Novartis | Drug Substance |
| 151580 | Hydroxypropy lmethylcellulose K100M | 130 | Ph. Eur., NF | Controlled release polymer |
| 103266 | Microcrystalline cellulose, PH102 | 120 | Ph. Eur., NF | Tablet filler |
| 103282 | Lactose, anhydrous DT | 46 | Ph. Eur., NF | Tablet filler |
| 100217 | Magnesium stearate | 4 | Ph. Eur., NF | Tablet lubricant |
| **Total weight** | | **450** | | |

The amount of vildagliptin DS and HPMC K100M were varied in each layer to provide a suitable release rate profile. For example, the DS was varied from 25mg to 50mg (immediate release layer) and 150mg to 175mg (modified release layer). The amount of HPMC K100M was varied from 29% to 40% (modified release layer).

Another exploratory slow release variant was the HPMC concentration was reduced to 30%, with and without roller compaction, as shown in Tables 2-8 and 2-9 . This change did not have an effect on the dissolution profile. However, 30% HPMC K100M exhibited a lower flow quality, 0.21 and 0.37, for both non-roller compacted and roller compacted material, respectively, when compared to 40% HPMC K100M. Filming was also observed during compression with 30% HPMC K100M.

**Table 2-8 Composition per 100mg dosage form unit with 30% HPMC**

| **Product name: vildagliptin Tab 100mg** | | | | |
|---|---|---|---|---|
| **Basis number/ Variant: NA** | | | | |
| **Shape /size /color /imprint:** 11 mm FFBE (round)/white to off-white | | | | |
| **Global material no.** | **Component** | **Composition per unit (mg)** | **Testing monograph** | **Function** |
| 146082 | vildagliptin | 100 | Novartis | Drug Substance |
| 103266 | Microcrystalline cellulose, PH 102 | 120 | Ph. Eur:, NF | Tablet filler |
| 103282 | Lactose, anhydrous DT | 58 | Ph. Eur., NF | Tablet filler |
| 151580 | Hydroxypropylmethylcelluose K100M | 120 | Ph. Eur., NF | Controller release polymer |
| 100217 | Magnesium stearate | 4 | Ph. Eur., NF | Tablet lubricant |
| **Total weight** | | **400** | | |

**Table 2-9 Composition per 150mg dosage form unit with 30% HPMC**

| **Product name: vildagliptin Tab 150mg** | | | | |
|---|---|---|---|---|
| **Basis number/ Variant:** NA **Shape /size /color /imprint:** 17 x 6.7mm ovaloid/white to off-white | | | | |
| **Global material no.** | **Component** | **Composition per unit (mg)** | **Testing monograph** | **Function** |
| 146082 | vildagliptin | 150 | Novartis | Drug Substance |
| 103266 | Microcrystalline cellulose, PH 102 | 180 | Ph. Eur., NF | Tablet filler |
| 103282 | Lactose, anhydrous DT | 24 | Ph. Eur., NF | Tablet filler |
| 151580 | Hydroxypropymethylcelluose K100M | 180 | Ph. Eur., NF | Controlled release polymer |
| 100217 | Magnesium stearate | 6 | Ph. Eur., NF | Tablet lubricant |

**Table 2-10 Composition per 200mg dosage form unit for slow release profile containing 40% HPMC K100M**

| **Product name: vildagliptin Tab 200mg** | | | | |
|---|---|---|---|---|
| **Basis number/ Variant:** 3768660.003 **Shape /size /color /imprint:** /white to off-white | | | | |
| **Global material** no. | **Component** | **Composition per unit (mg)** | **Testing monograph** | **Function** |
| 146082 | vildagliptin | 200 | Novartis | Drug Substance |
| 103266 | Microcrystalli ne cellulose, PH102 | 240 | Ph. Eur., NF | Tablet filler |
| 103282 | Lactose, anhydrous DT | 32 | Ph. Eur., NF | Tablet filler |
| 151580 | Hydroxypropylmethylcelluose K100M | 320 | Ph. Eur., NF | Controlled release polymer |
| 100217 | Magnesium Stearate | 8 | Ph. Eur., NF | Tablet lubricant |
| **Total weight** | | **800** | | |

### Selection of Formulations for the Manufacture of Clinical Variants

The four formulations selected for further clinical evaluation comprised of both types of release rate profiles (slow and fast) at two dosage strengths (100mg and 200mg). The batch size was 4,000 tablets (or 3.1kg). Pre-compression was required to meet the hardness and/or friability requirements. The slow formulation contained 40% HPMC K100M as shown in Tables 2-1 and 2-10. The fast formulation contained 20% HPMC K100 LVP as shown in Tables 2-3 and 2-4.

**Table 2-11 clinical batch summary**

| Rate Profile | Strength | Polymer | Composition | KN Number/Variant | Batch Number |
|---|---|---|---|---|---|
| Slow | 100mg | 40% HPMC K100M | Table 2-1 | 3768652.00 2 | AEUS200200 81 |
| Slow | 200mg | 40% HPMC K100M | Table 2-10 | 3768660.00 3 | AEUS200200 83 |
| Fast | 100mg | 20%HPMC K100LVP | Table 2-3 | 3768652.00 1 | AEUS200200 80 |
| Fast | 200mg | 20% HPMC K100LVP | Table 2-4 | 3768660.00 2 | AEUS200200 82 |

### Formulation Rationale

1-[(3-Hydroxy-adamant-1-ylamino)-acetyl]-pyrrolidine-2(S)-carbonitrile i.e. vildagliptin DS (Drug Substance) is highly water soluble (up to 125mg/mL) and so requires polymers that can act as substantial diffusion barriers for a dramatic reduction in drug dissolution and diffusion. A once a day MR dosage form is likely to require a significant level of polymer load for retarding drug diffusion. During the development, the applicant has selected monolithic formulation with low risk for dose dumping, eliminating coating technologies in favor of a matrix formulation. After intensive studies on compatibility, ability to retard diffusion pharmacokinetic and pharmacodynamic studies, and regulatory acceptance requirements the applicant narrowed the polymer choice to hydrophilic polymers, especially hydroxpropyl methyl cellulose (HPMC).

### Major and Critical Findings During Formulation Development

A critical excipient is the control release polymer, HPMC, as the K100M grade. During the CSF development, various control release polymers were evaluated alone, or in combination, in order to achieve the desired release profile. HPMC, K100M grade, provided the best match to the desired release profile. The "slow" formulation was selected after evaluation in man.

As previously mentioned, pre-compression was utilized during the clinical batch manufacture. Experiments were conducted to further evaluate the CSF formulation and the need for pre-compression.

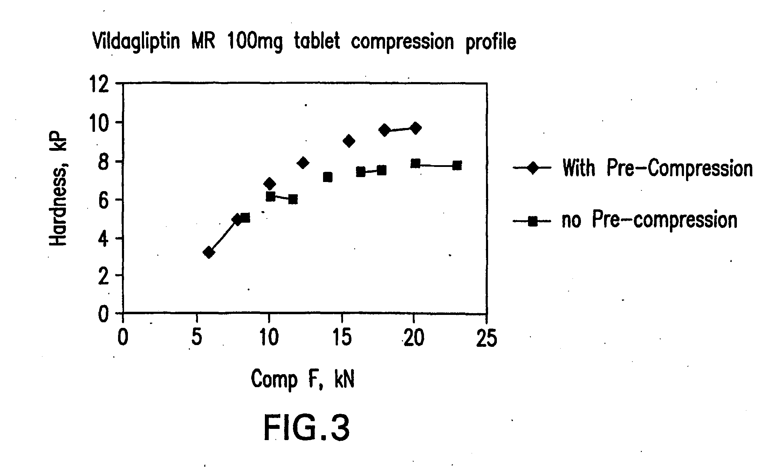

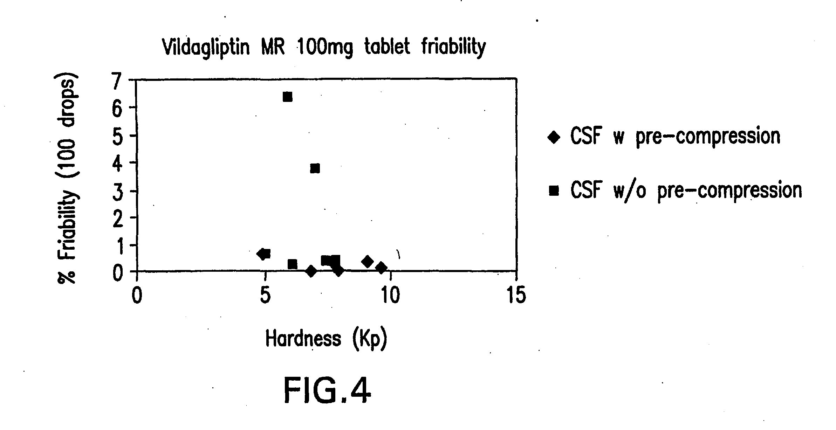

The compression profile for the CSF formulation containing 40% HPMC is shown in Figures 3 and 4.

The data indicated that the compression profile and friability could be improved with pre-compression. Additional studies were conducted to improve the compression profile and to avoid the use of pre-compression.

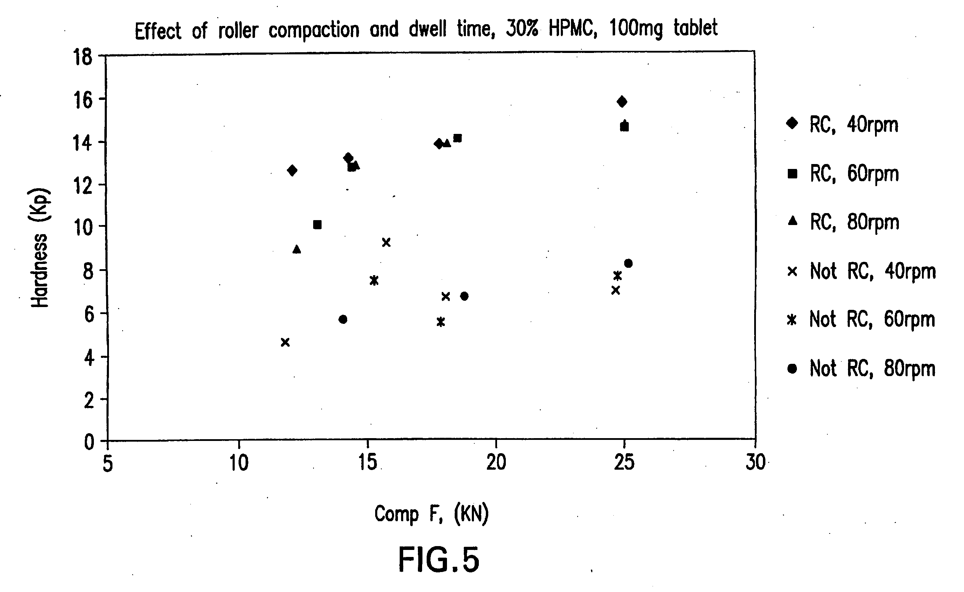

As shown in Figure 5, 30% HPMC K100M was evaluated to improve the compression profile. The effect of tablet dwell time was simultaneously evaluated at speeds from 40 to 80rpm.

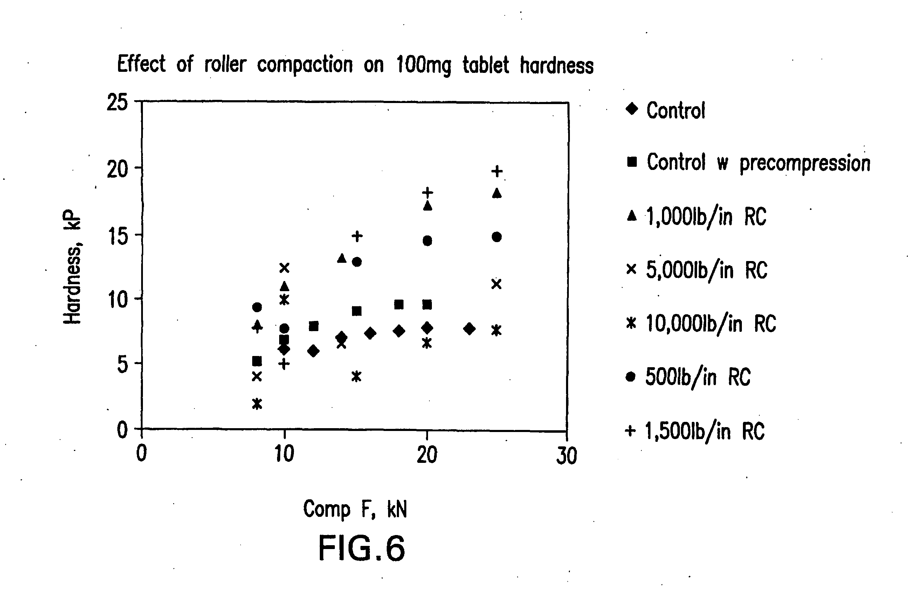

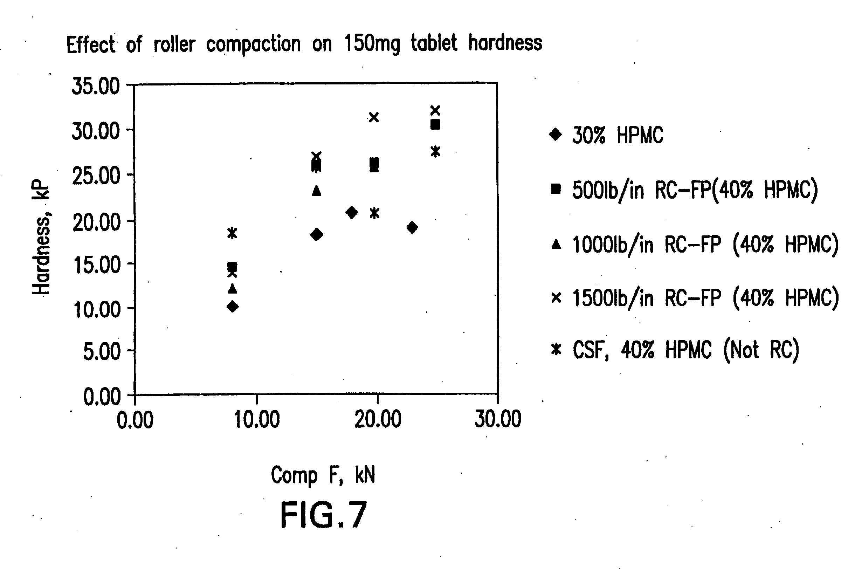

It was determined that an improvement in the compression profile was achieved using roller compaction. As shown in Figures 6 and 7, a Fitzpatrick (Chilsonator®, model IR220) was evaluated with the interim powder blend without the addition of magnesium stearate, at increasing compaction forces.

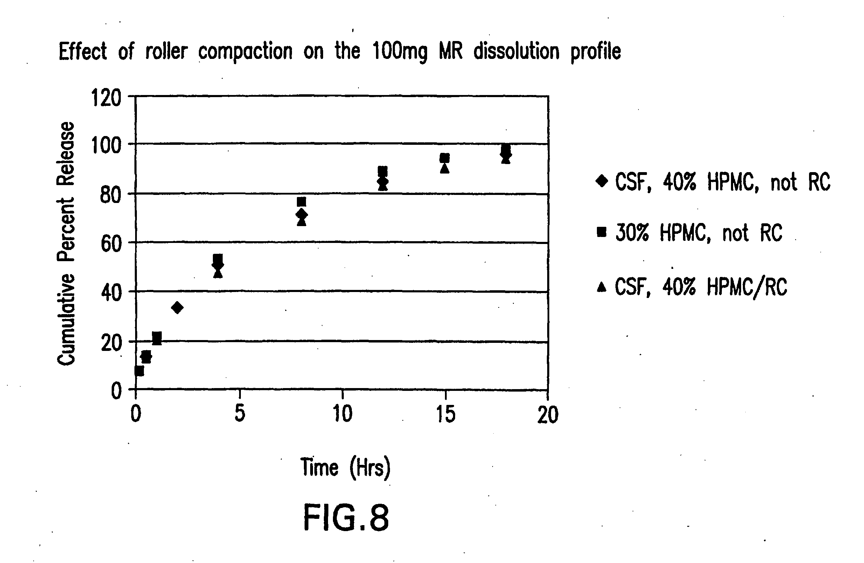

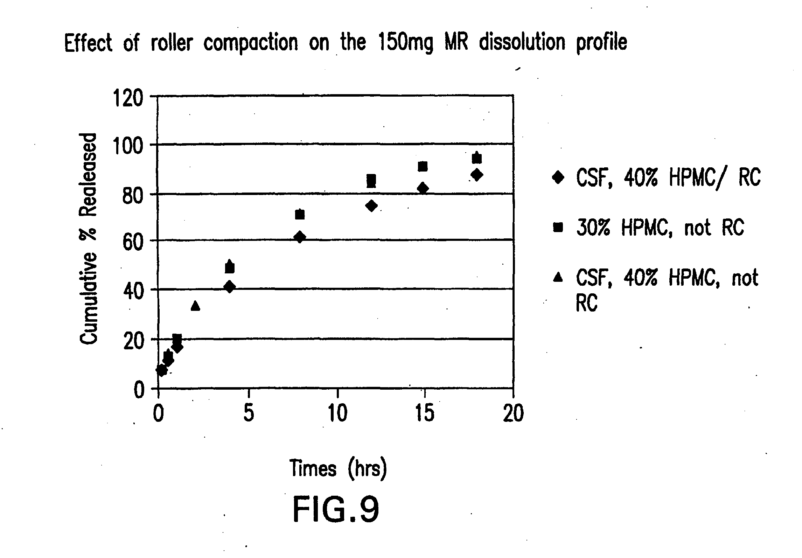

As shown in Figures 8 and 9, the effect of roller compaction on the dissolution profile was studied with 40% HPMC K100M and compared to the dissolution profile of the clinical batch. It was concluded that roller compaction did not impact the dissolution profile.

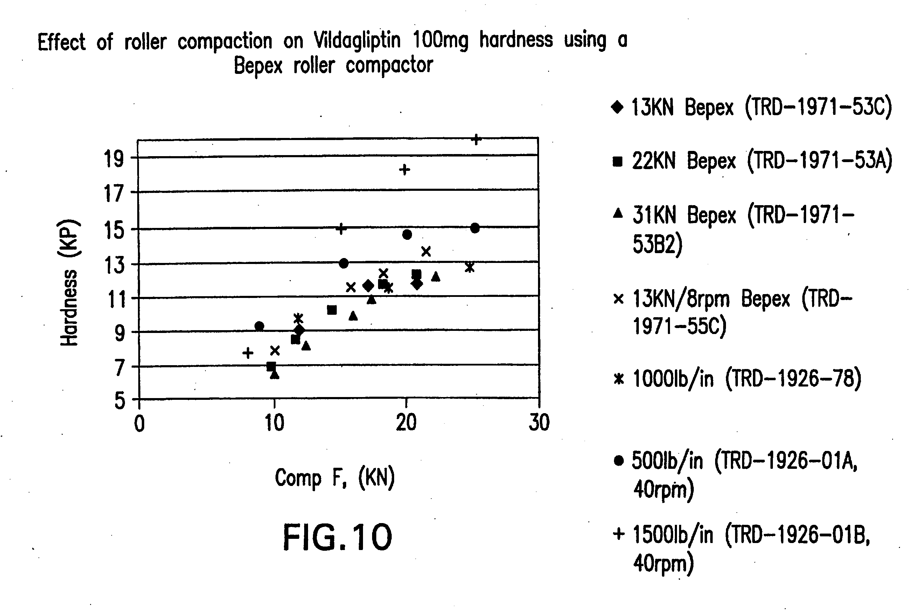

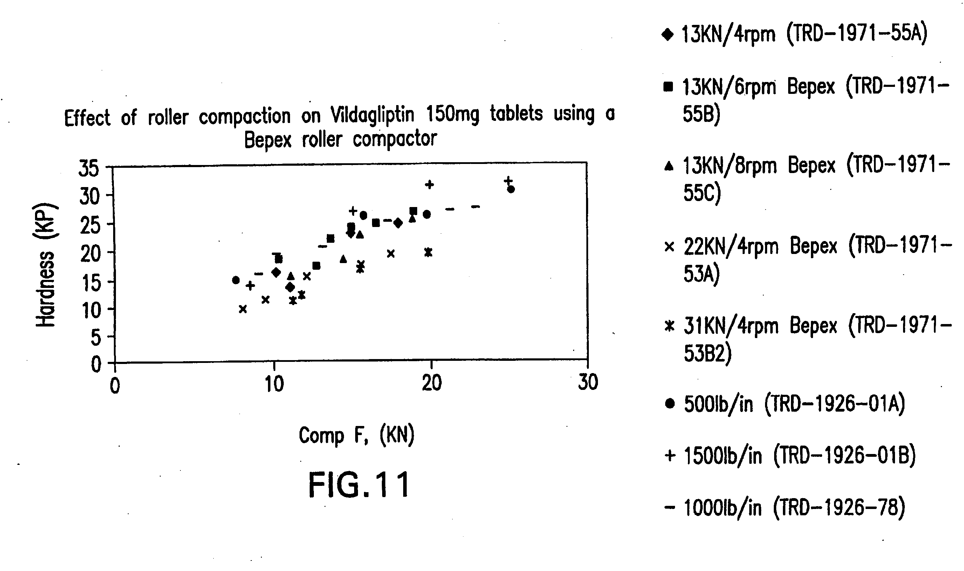

The roller compaction process was scaled to a 50mm Bepex roller compactor using a linear relationship developed for a 50mm Fitzpatrick roller compactor (in Ib/in units, model IR520) and a 50mm Bepex ( in KN units) roller compactor. As shown in Figures 10 and 11, the effect of increasing compaction force was studied. It was concluded that the process was scaleable to a Bepex roller compactor, and the tablet hardness was not significantly affected with increasing roller compaction force (13-31KN), and roller compactor roll speed (4-8rpm).

### Overview of Compositions Including CSF in Clinical Studies

See section "*B. Formulation and Alternative Variants.*"

### C. Manufacturing Process

All variants were manufactured as detailed in the process flow chart shown in Figure 1, except variants without roller compaction. A batch size of up to 12 Kg was used. Drug substance from lots; 0223007, 0223008, 0223009 were used for all batches manufactured

### Description and Assessment of the Processes Tested

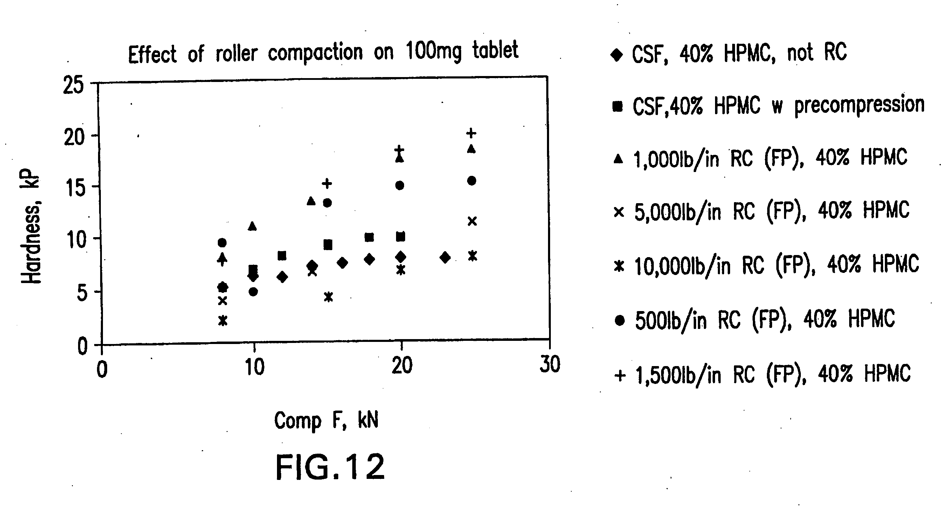

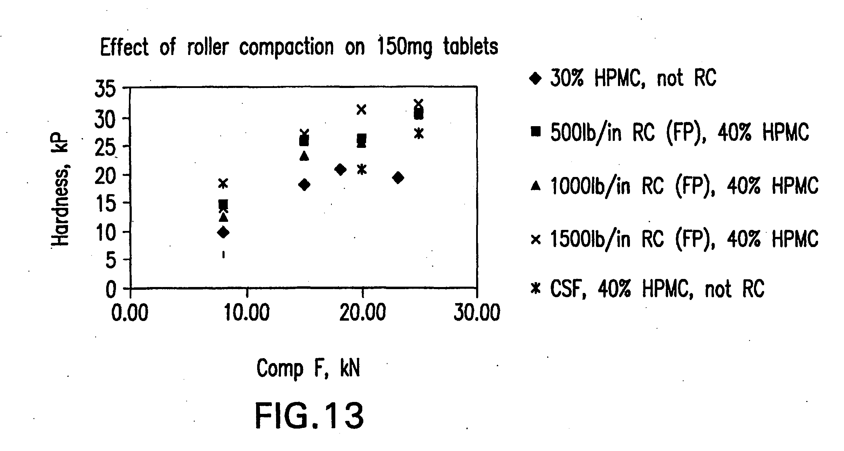

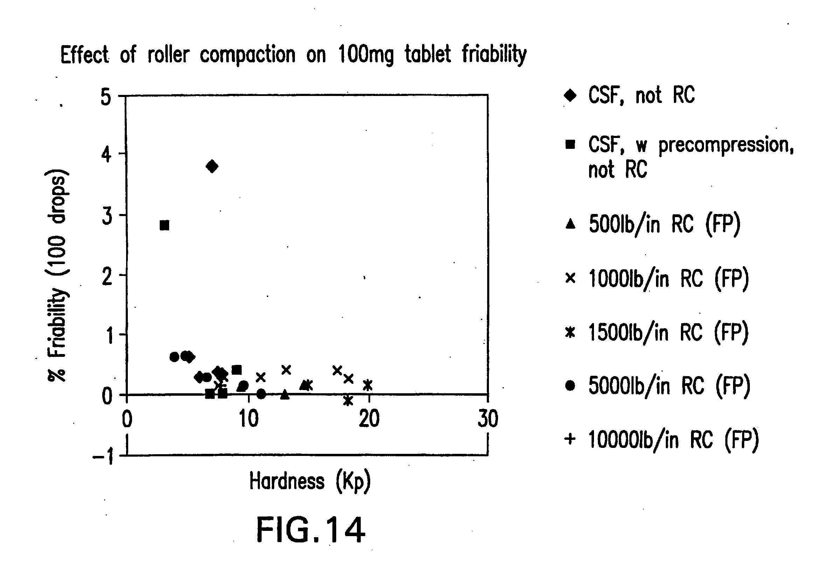

The effect of roller compaction was evaluated with 30% and 40% HPMC. Roller compaction was utilized to densify the powder blend prior to compression. The effect of Fitzpatrick (Chilsonator®, model IR220) roller compactor at compaction forces from 500 to 10,000 lb/in on a powder blend containing 40% HPMC, are shown in Figures 12 and 13. The data indicated that with increasing roller compaction force, led to a decrease in tablet hardness. As shown in Figure 14, roller compaction forces greater than 5,000 Ib/in (or 43.75 KN) produced tablets with a compression profile worst than the CSF without roller compaction or pre-compression.

The effect of roller compaction on tablet friability is shown in Figure 14.

### Key Section Criteria and Rationale for Final Process

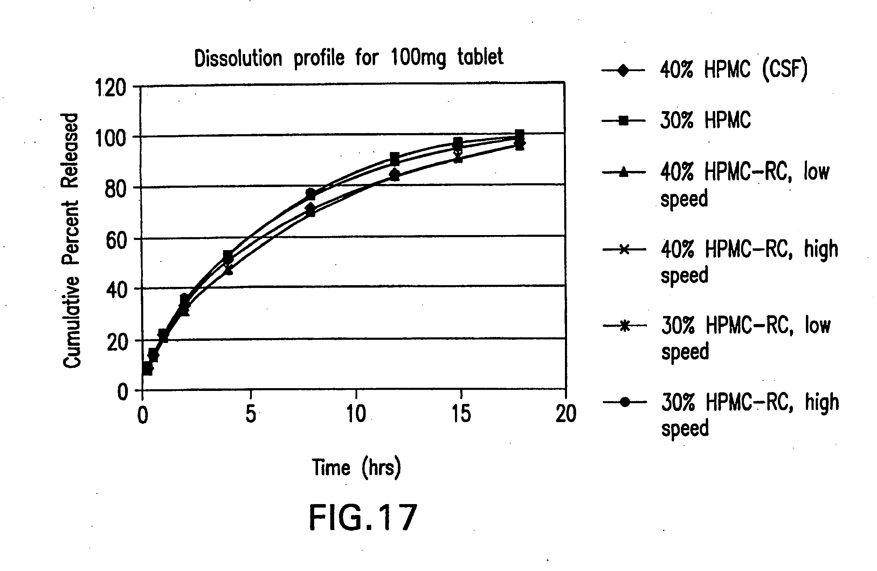

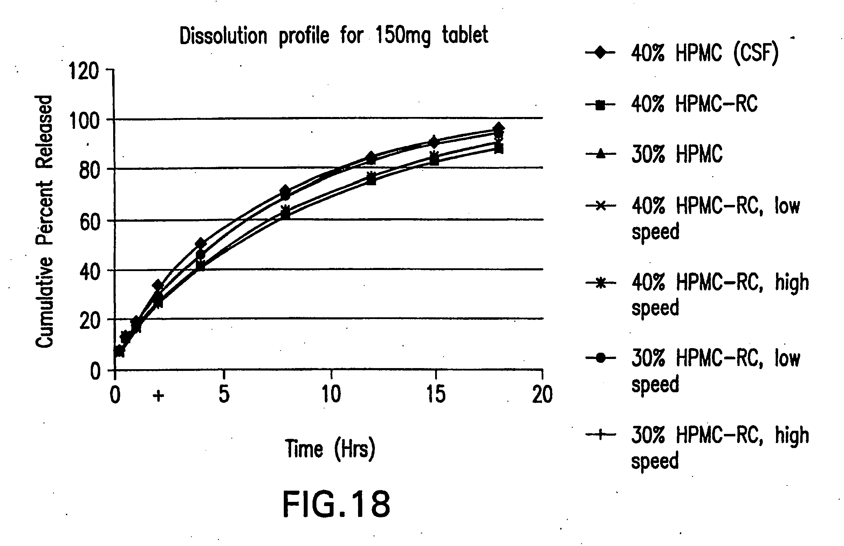

The key criteria for the final process was based on the dissolution profile. Since a PK/safety study was conducted in *vivo* using the 100mg, 150mg and 200mg tablets, the formulation containing 40% HPMC K100M was desired. Roller compaction was added to the process to improve the compression profile. As shown in Figures 17 and 18, the dissolution profile for roller compacted material did not differ from the CSF dissolution profile. The Japanese Ministry of Health has imposed 40mg/kg as a daily intake limit for HPMC K100M. It is not anticipated that the daily intake limits will become a regulatory hurdle for the 1-[(3-Hydroxy-adamant-1-ylamino)-acetyl]-pyrrolidine-2(S)-carbonitrile MF.

The physical properties of the various powder blends are shown in Tables 2-12 and 2-13.

**Table 2-12 Physical properties of roller compacted powder blend (Fitzpatrick)**

| Sample | Roller Compaction Force (lb/in) [calculated KN] | Powder Flow Quality (Sotax) | Bulk Density/Tap Density (g/mL) |
|---|---|---|---|
| TRD-1926-78 40% HPMC | NA | 0.38 | 0.38/0.576 |
| TRD-1971-53 40% HPMC | NA | 0.33 | 0.38/0.66 |
| TRD-1971-55 40% HPMC | NA | 0.36 | 0.36/0.59 |
| TRD-1926-79 30% HPMC | NA | 0.21 | 0.385/0.60 |
| TRD-1926-79 30% HPMC | 1,000 [8.75] | 0.37 | 0.432/ 0.645 |
| TRD-1926-01 40% HPMC | 500 [4.37] | 0.25 | 0.398/ 0.588 |
| TRD-1926-78 40% HPMC | 1,000 [8.75] | 0.49 | 0.448/0.652 |
| TRD-1926-01 40% HPMC | 1,500 [13.1] | 0.53 | 0.448/ 0.666 |
| TRD-1771-123B 40% HPMC | 5,000 [43.75] | 0.76 | 0.526/ 0.714 |
| TRD-1771-123C 40% HPMC | 10,000 [87.5] | ND (not tabletable) | 0.566/ 0.732 |
| Avicel PH102 | NA | 0.64 | 0.29/0.35 |

**Table 2-13 Physical properties of roller compacted powder blend (Bepex)**

| Sample | Roller Compaction Force (kn) [calculated lb/in] | Roll Speed (rpm) | Powder Flow Quality (Sotax) | Bulk Density/Tap Density (g/mL) |
|---|---|---|---|---|
| TRD-1926-7840% HPMC | NA | NA | 0.38 | 0.38/0.576 |
| TRD-1971-53 40% HPMC | NA | NA | 0.33 | 0.38/0.66 |
| TRD-1971-55 40% HPMC | NA | NA | 0.36 | 0.36/ 0.59 |
| TRD-1971-53C 40% HPMC | 13 [1500 Ib/in] | 4 | 0.25 | 0.398/ 0.588 |
| TRD-1971-53A 40% HPMC | 22 [2500 Ib/in] | 4 | 0.49 | 0.448/ 0.652 |
| TRD-1971-53B2 40% HPMC | 31 [3500 Ib/in] | 4 | 0.53 | 0.448/ 0.666 |
| TRD-1971-55A 40% HPMC | 13 | 4 | 0.76 | 0.526/ 0.714 |
| TRD-1971-55B 40% HPMC | 13 | 6 | ND (not tabletable) | 0.566/ 0.732 |
| TRD-1971-55C 40% HPMC | 13 | 8 | | |
| Avicel PH102 | NA | NA | 0.64 | 0.29/ 0.35 |

The use of roller compaction indicated an increase in the powder flow quality and an increase in bulk density. This was limited by the roller compaction force, where tablets from 5,000 lb/in and 10,000 lb/in force exhibited a poor friability of 0.6% and > 10%, respectively (100mg tablets).

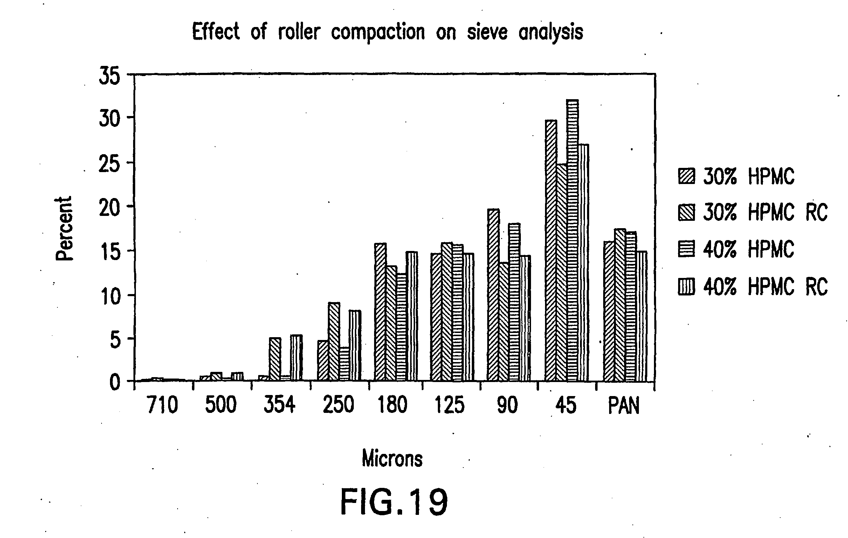

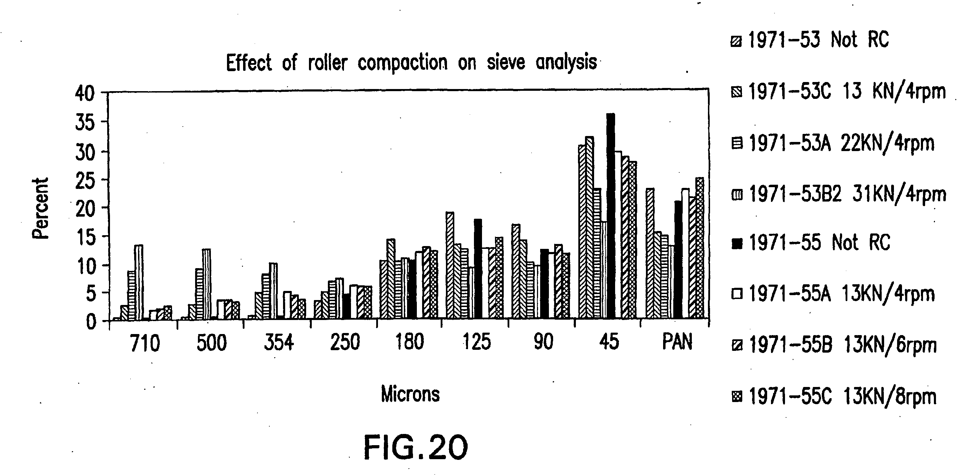

The effect of roller compaction on the sieve analysis is shown in Figures 19 and 20. All roller compacted material was screened using an 18 mesh screen. Selection of screen size as a means to particle size control, and will be further evaluated during the Lab Phase FMI.

### Equipment Used

**Table 2-14 Process equipment**

| Description | Model |
|---|---|
| Bin Blender | LB Bohle, 10L, 25L, 40L |
| Oscillating mill | Ferwitt |
| Roller compactor | Fitzpatrick, Chilsonator, IR220 |
| Roller Compactor | Bepex, 50mm |
| Tablet Press | Manesty Beta Press, 16 sta. |

### Statement on the Up-Scaling Potential and Robustness of the final process

**Table 2-15 IPC summary for vildagliptin 100mg MR containing 40% HPMC with roller compaction (400mg tablet weight, 11mm FFBE)**

| Sample | Thickness (mm) IPC: 2.8-4.2mm | Hardness (Kp) | %Friability (100 drops) |
|---|---|---|---|
| TRD-1926-78 | 3.95 - 3.72 | 40 rpm: 11.5 - 13.2 | 40 rpm: < 0.5 |
| 1,000 lb/in RC (FP) | | 60 rpm: 9.76 - 12.69 | 60 rpm: < 0.5 |
| | | 80 rpm: 9.76 - 12.84 | 80 rpm: < 0.5 |
| TRD-1771-123A 1,000 Ib/in RC (FP) | 4.07 - 3.68 | 40 rpm: 8-18.3 | 40 rpm: < 0.5 |
| TRD-1926-01A 500 lb/in RC (FP) | 4.0 - 3.81 | 40 rpm: 9.3 -14.8 | 40 rpm: 0.1 |
| TRD-1 926-01 B 1,500 Ib/in RC (FP) | 4.02 - 3.66 | 40 rpm: 7.6 - 19.9 | 40 rpm: 0.1 |
| TRD-1771-31 Not RC | 4.13 - 3.94 | 40 rpm: 5.1 - 7.75 | 40 rpm: 6.3, capping |

**Table 2-16 IPC summary for vildagliptin 150mg MR containing 40% HPMC with roller compaction (600mg tablet weight, 17 x 6.7mm ovaloid)**

| Sample | Thickness (mm) IPC: 2.8-4.2mm | Hardness (Kp) | %Friability (100 drops) |
|---|---|---|---|
| TRD-1926-78 | 40 rpm: 6.5 - 6.08 | 40 rpm: 17.5 - 25.9 | 40 rpm: < 0.2 |
| 1,000 lb/in RC (FP) | 60 rpm: 6.4 - 6.03 | 60 rpm: 15.5 - 24.8 | 60 rpm: < 0.2 |
| | 80 rpm: 6.6 - 6.07 | 80 rpm: 11.2 - 24.4 | 80 rpm: < 0.2 |
| TRD-1771-123A 1,000 lb/in RC (FP) | 6.4 - 5.84 | 40 rpm: 12.3 - 30.4 | 40 rpm: < 0.25 |
| TRD-1926-01A 500 lb/in RC (FP) | 6.56 - 5.99 | 40 rpm: 14.6 - 30.3 | 40 rpm: 0.5, 1 tablet capped at 20Kn |
| TRD-1926-01 B 1,500 lb/in RC (FP) | 6.5 - 5.84 | 40 rpm: 13.9 - 32 | 40 rpm: 0.25 |
| TRD-1771-33 Not RC | 6.59 - 5.97 | 40 rpm: 18.3 - 27.2 | 40 rpm: 0.6, lamination at 20Kn |

Roller compaction improved bulk powder flow and compression profile without the use of pre-compression. The acceptable hardness range was 10 to 13Kp and 11 to 25Kp for the 100mg and 150mg tablets, respectively. Lamination was observed at compression forces greater than 18 KN for the 150mg tablet. The formulation does not appear to be dwell time sensitive. The lab phase FMI activities will start pending the confirmation of the FMI tablet shape and size for the 100mg strength.

### II. Analytical Investigations for Stress Tests of Variants

### A. Elaboration of Analytical Methods

### Assay and Related Substances

The assay of vildagliptin and the quantitation of degradation products of vildagliptin were performed by a gradient HPLC method [AM54001B(AS6105)]. The sample was extracted with methanol and diluted to target concentration using 10:90 methanol/acetonitrile and then chromatographed using a reverse phase HPLC method with UV detection at 210 nm.

### Drug Release

Dissolution of vildagliptin from six 100mg vildagliptin tablets was determined under the test conditions elaborated in Table 3-1

Samples obtained were analyzed by using an isocratic reverse phase HPLC method [AM50161A(AS6105)] with UV detection at 2010 nm. Same experimental have been done for the 150mg and 200mg tablets.

### B. Analytical Results

### Stability Protocol

The technical and clinical batches slow and fast formulations of CSF were put on stability and the stability was monitored up to 12 months. The stability of technical batches of MF variants, 100mg and 150mg is currently ongoing as shown in the protocol, Table 3-2.

**Table 3-2 Drug product stability - storage conditions and testing intervals batch TRD-1926-078A(100mg), and TRD-1926-078B (150mg); [packaging: HDPE, CR cap, 1g desiccant].**

| **Storage condition** | **Time (months)** |
|---|---|
| 5°C | [24] |
| 25°C/60%RH | 3, 6, 9, 12, 24 |
| 30°C/65%RH | [3], [6], [9], [12] |
| 40°C/75%RH | 1.5, 3, 6 |

| | |
|---|---|
| [ ] Optional testing | |

### Stability Results

**Table 3-3 Summary of stability for vildagliptin 100 mg tablets, TRD-0739-0113**

| | | | | | **Degradation products** | | | | | | |
|---|---|---|---|---|---|---|---|---|---|---|---|
| **Conditions** | **Time** | **Appearance** | **%Assay** | **% Amide** | **%Cycli c** | **%Diket.** | **%Unk. (RRT = 0.26)** | **%Unk (RRT = 0.77)** | **%Unk (RRT = 1.10)** | **%Total % deg.** | **Moisture** |
| | Initial | Complies | 100.8 | <LOQ | <LOD | ND | ND | <LOQ | <LOQ | - | 3.52 |
| 25°C/60%RH | 3M | Complies | 102.6 | <LOQ | <LOQ | <LOQ | ND | <LOQ | <LOQ | - | 2.40 |
| | 6M | Complies | 101.0 | 0.06 | 0.05 | <LOQ | ND | <LOQ | <LOQ | 0.11 | 2.66 |
| | 9M | Complies | 100.1 | 0.08 | 0.05 | <LOQ | ND | <LOQ | <LOQ | 0.13 | 2.74 |
| | 12M | Complies | 102.5 | 0.11 | 0.05 | <LOQ | ND | <LOQ | <LOQ | 0.16 | 3.16 |
| 30°C/60%RH | 3M | Complies | 102.3 | 0.06 | <LOQ | <LOQ | ND | <LOQ | <LOQ | 0.06 | 2.51 |
| | 6M | Complies | 104.3 | 0.12 | 0.05 | <LOQ | ND | <LOQ | <LOQ | 0.17 | 2.79 |
| | 9M | Complies | 100.3 | 0.17 | 0.05 | <LOQ | ND | <LOQ | <LOQ | 0.22 | 3.07 |
| | 12M | Complies | 100.7 | 0.21 | 0.05 | 0.07 | ND | <LOQ | <LOQ | 0.33 | 3.59 |
| 40°C/75%RH | 6W | Complies | 101.4 | 0.10 | 0.05 | <LOQ | ND | <LOQ | <LOQ | 0.15 | 2.73 |
| | 3M | Complies | 103.3 | 0.20 | 0.05 | 0.07 | ND | <LOQ | <LOQ | 0.32 | 2.90 |
| | 6M | Complies | 100.7 | 0.39 | 0.05 | 0.12 | <LOQ | <LOQ | <LOQ | 0.56 | 3.35 |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not Detected | | | | | | | | | | | |

**Table 3-4 Summary of dissolution data for vildagliptin 100mg tablets, TRD-0739-0113**

| | | | | | **Percent released** | | | | | | |
|---|---|---|---|---|---|---|---|---|---|---|---|
| **Conditions** | **Time** | **0.5 hr** | **1hr** | **2 hr** | **4 hr** | **6 hr** | **8 hr** | **10 hr** | **12 hr** | **18 hr** | **24 hr** |
| | Initial | 23 | 36 | 56 | 82 | 95 | 102 | 104 | 104 | 104 | 102 |
| 25°C/60%RH | 3M | NT | | | | | | | | | |
| | 6M | NT | | | | | | | | | |
| | 9M | NT | | | | | | | | | |
| | 12M | NT | | | | | | | | | |
| 30°C/60%RH | 3M | NT | | | | | | | | | |
| | 6M | NT | | | | | | | | | |
| | 9M | NT | | | | | | | | | |
| | 12M | 24 | | 57 | | 94 | | | | 100 | |
| 40°C/75%RH | 6W | NT | | | | | | | | | |
| | 3M | 23 | 38 | 57 | 84 | 98 | 103 | 103 | 104 | 102 | 101 |
| | 6M | 24 | - | 57 | - | 95 | - | - | - | 100 | - |

| | | | | | | | | | | | |
|---|---|---|---|---|---|---|---|---|---|---|---|
| NT = Not tested Note: Dissolution was tested only at accelerated conditions as these represent worst case situation. | | | | | | | | | | | |

**Table 3-5 Summary of stability for vildagliptin 100mg tablets, TRD-0739-0121**

| | | | | **Degradation products** | | | | | | | |
|---|---|---|---|---|---|---|---|---|---|---|---|
| **Conditions** | **Time** | **Appearance** | **%Assay** | **% Amide** | **%Cyclic** | **%Diket.** | **%Unk. (RRT= 0.26)** | **%Unk. (RRT= 0.77)** | **%Unk. (RRT= 1.10)** | **%Total** | **% Moisture** |
| | Initial | complies | 103.1 | <LOQ | <LOD | ND | ND | <LOQ | 0.07 | 0.07 | 3.19 |
| 25°C/60%RH | 3M | Complies | 103.3 | <LOQ | <LOQ | <LOQ | ND | <LOQ | <LOQ | 0.00 | 2.53 |
| | 6M | Complies | 104.3 | 0.05 | <LOQ | <LOD | ND | <LOQ | <LOQ | 0.05 | 2.70 |
| | 9M | Complies | 102.2 | 0.07 | <LOQ | <LOQ | ND | <LOQ | <LOQ | 0.07 | 3.03 |
| | 12M | Complies | 101.4 | 0.08 | <LOQ | <LOQ | ND | <LOQ | <LOQ | 0.08 | 3.31 |
| 30°C/60%RH | 3M | Complies | 104.7 | 0.05 | <LOQ | <LOQ | ND | <LOQ | <LOQ | 0.05 | 2.61 |
| | 6M | Complies | 103.9 | 0.09 | <LOQ | <LOQ | ND | <LOQ | <LOQ | 0.09 | 2.88 |
| | 9M | Complies | 100.8 | 0.11 | <LOQ | <LOQ | ND | <LOQ | <LOQ | 0.11 | 3.33 |
| | 12M | Complies | 102.4 | 0.14 | <LOQ | 0.05 | ND | <LOQ | <LOQ | 0.19 | 3.65 |
| 40°C/75%RH | 6W | Complies | 102.1 | 0.08 | <LOQ | <LOQ | ND | <LOQ | <LOQ | 0.08 | 2.81 |
| | 3M | Complies | 104.8 | 0.13 | <LOQ | 0.05 | ND | <LOQ | <LOQ | 0.18 | 2.97 |
| | 6M | Complies | 102.9 | 0.25 | <LOQ | 0.08 | <LOQ | <LOQ | <LOQ | 0.33 | 3.52 |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not Detected | | | | | | | | | | | |

**Table 3-6 Summary of dissolution data for vildagliptin 100mg tablets, TRD-0739-0121**

| | | | | | **Percent** | **released** | | | | | |
|---|---|---|---|---|---|---|---|---|---|---|---|
| **Conditions** | **Time** | **0.5 hr** | **1hr** | **2 hr** | **4 hr** | **6 hr** | **8 hr** | **10 hr** | **12 hr** | **18 hr** | **24 hr** |
| | Initial | 12 | 20 | 30 | 45 | 56 | 65 | 72 | 78 | 89 | 95 |
| 25°C/60%RH | 3M | NT | | | | | | | | | |
| | 6M | NT | | | | | | | | | |
| | 9M | NT | | | | | | | | | |
| | 12M | NT | | | | | | | | | |
| 30°C/60%RH | 3M | NT | | | | | | | | | |
| | 6M | NT | | | | | | | | | |
| | 9M | NT | | | | | | | | | |
| | 12M | 13 | - | 32 | - | 60 | - | - | - | 94 | |
| 40°C/75%RH | 6W | NT | | | | | | | | | |
| | 3M | 13 | 21 | 32 | 49 | 59 | 70 | 77 | 83 | 93 | 98 |
| | 6M | 13 | - | 33 | - | 61 | - | - | - | 95 | - |

| | | | | | | | | | | | |
|---|---|---|---|---|---|---|---|---|---|---|---|
| NT = Not tested Note: Dissolution was tested only at accelerated conditions as these represent worst case situation. | | | | | | | | | | | |

**Table 3-7 References for the stability data in Table 3-3 through Table 3-6**

| | Assay & Purity | % Moisture Content | Dissolution |
|---|---|---|---|
| Initial | TRD0958/057 (XG) | TRD0958/109 (XG) | WS18431 |
| 6W | TRD0958/057 (XG) | TRD0958/109 (XG) | NA |
| 3M | TRD0983/082 (XG) | TRD0978I110 (MZ) | WS20247 |
| 6M | TRD1514/110 (XG) | TRD1514/110 (XG) | WS21349 |
| 9M | TRD16451129 (MZ) | TRD1645/101 (MZ) | NA |
| 12M | TRD1642/069 (JT) | TRD1642/145 (JT) | WS22591 |

**Table 3-8 Summary of stability for vildagliptin 100mg tablets, AEUS/2002-0080**

| | | | | | | **Degradation products** | | | | | |
|---|---|---|---|---|---|---|---|---|---|---|---|
| **Conditions** | **Time** | **Appearance** | **%Assay** | **% Amide** | **%Cyclic** | **%Diket.** | **%Unk (RRT = 0.26)** | **%Unk (RRT = 0.77)** | **%Unk (RRT = 1.10)** | **% Total Deg.** | **% Moisture** |
| Initial | 0 | complies | 102.0 | ND | ND | ND | - | | | - | 2.62 |
| 5°C | 3M | complies | 104.3 | 0.02 | 0.02 | ND | ND | 0.04 | 0.03 | 0.00 | 2.17 |
| | 6M | complies | 101.8 | 0.02 | 0.02 | ND | ND | 0.05 | 0.03 | 0.00 | 2.32 |
| | 9M | complies | 102.3 | 0.02 | 0.03 | ND | ND | 0.04 | ND | 0.00 | 2.39 |
| | 12M | NT | | | | | | | | | |
| 25°C/60%RH | 3M | complies | 101.1 | 0.04 | 0.05 | ND | ND | 0.04 | 0.03 | 0.05 | 2.06 |
| | 6M | complies | 103.0 | 0.07 | 0.06 | 0.02 | ND | 0.04 | 0.04 | 0.13 | 2.34 |
| | 9M | complies | 100.6 | 0.08 | 0.06 | 0.02 | ND | 0.04 | ND | 0.14 | 2.43 |
| | 12M | complies | 101.9 | 0.09 | 0.05 | 0.02 | ND | 0.03 | 0.02 | 0.14. | 2.72 |
| 25°C/60%RH | 3M | complies | 101.4 | 0.04 | 0.05 | ND | ND | 0.04 | 0.03 | 0.05 | 2.22 |
| (With Cotton) | 6M | complies | 102.0 | 0.06 | 0.06 | ND | ND | 0.04 | 0.05 | 0.12 | 2.67 |
| | 9M | complies | 102.2 | 0.09 | 0.07 | ND | ND | 0.04 | ND | 0.16 | 2.69 |
| | 12M | complies | 101.7 | 0.11 | 0.07 | 0.03 | 0.02 | 0.04 | 0.02 | 0.18 | 2.85 |
| °C/60%RH | 3M | complies | 102.6 | 0.06 | 0.06 | ND | ND | 0.04 | 0.04 | 0.12 | 2.12 |
| | 6M | complies | 102.7 | 0.11 | 0.06 | 0.03 | ND | 0.04 | 0.05 | 0.17 | 2.60 |
| | 9M | complies | 101.7 | 0.15 | 0.07 | 0.03 | ND | 0.03 | ND | 0.22 | 2.78 |
| | 12M | complies | 101.3 | 0.19 | 0.06 | 0.05 | ND | 0.03 | 0.02 | 0.30 | 2.80 |
| 40°C/75%RH | 6W | complies | 101.7 | 0.10 | 0.06 | 0.03 | ND | 0.04 | 0.06 | 0.22 | 2.28 |
| | 3M | complies | 101.9 | 0.20 | 0.07 | 0.05 | ND | 0.03 | 0.05 | 0.32 | 2.59 |
| | 6M | complies | 102.5 | 0.40 | 0.07 | 0.11 | 0.03 | 0.04 | 0.06 | 0.64 | 3.41 |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not detected | | | | | | | | | | | |

**Table 3-9 Summary of dissolution data for vildagliptin 100mg tablets, AEUS/2002-0080**

| | **Percent released** | | | | | | | | | | |
|---|---|---|---|---|---|---|---|---|---|---|---|
| **Conditions** | **Tim e** | **0.5 hr** | **1hr** | **2 hr** | **4 hr** | **6 hr** | **8 hr** | **10 hr** | **12 hr** | **18 hr** | **24 hr** |
| | Initial | 24 | 37 | 55 | 79 | 92 | 99 | 101 | 101 | 100 | 99 |
| 25°C/60%RH | 3M | NT | | | | | | | | | |
| | 6M | NT | | | | | | | | | |
| | 9M | NT | | | | | | | | | |
| | 12M | NT | | | | | | | | | |
| 25°C/60%RH | 3M | NT | | | | | | | | | |
| (With cotton) | 6M | NT | | | | | | | | | |
| | 9M | NT | | | | | | | | | |
| 30°C/60%RH | 3M | NT | | | | | | | | | |
| | 6M | NT | | | | | | | | | |
| | 9M | 22 | | 53 | | 90 | | | | 102 | |
| | 12M | NT | | | | | | | | | |
| 40°C/75%RH | 6W | NT | | | | | | | | | |
| | 3M | 23 | | 54 | | 92 | | | | 101 | |
| | 6M | 24 | | 56 | | 93 | | | | 104 | |

| | | | | | | | | | | | |
|---|---|---|---|---|---|---|---|---|---|---|---|
| NT = Not tested Note: Dissolution was tested only at accelerated conditions as these represent worst case situation. | | | | | | | | | | | |

**Table 3-10 Summary of stability data for vildagliptin 100mg tablets, AEUS/2002-0081**

| | | | | | | **Degradation products** | | | | | |
|---|---|---|---|---|---|---|---|---|---|---|---|
| **Conditions** | **Time** | **Appearance** | **%Assay** | **% Amide** | **%cyclic** | **%Diket** | **%Unk. (RRT = 0.26)** | **%Unk. (RRT = 0.77) =1.10)** | **%Unk. (RRT** | **% Total deg.** | **% Moisture** |
| Initial | 0 | complies | 102.5 | ND | ND | ND | - | - | - | - | 2.45 |
| 5°C | 3M | complies | 103.4 | 0.02 | 0.01 | ND | ND | 0.04 | 0.04 | 0.00 | 2.35 |
| | 6M | complies | 102.7 | 0.02 | 0.02 | ND | ND | 0.04 | 0.05 | 0.00 | 2.19 |
| | 9M | complies | 101.4 | 0.02 | ND | ND | ND | 0.04 | 0.02 | 0.00 | 2.24 |
| | 12M | NT | | | | | | | | | |
| 25°C/60%RH | 3M | complies | 103.1 | 0.03 | 0.03 | ND | ND | 0.04 | 0.05 | 0.00 | 2.01 |
| | 6M | complies | 102.9 | 0.05 | 0.03 | ND | ND | 0.05 | 0.07 | 0.12 | 2.33 |
| | 9M | complies | 100.8 | 0.06 | 0.03 | ND | ND | 0.04 | ND | 0.06 | 2.44 |
| | 12M | complies | 101.7 | 0.07 | 0.03 | 0.02 | 0.02 | 0.04 | 0.04 | 0.07 | 2.57 |
| 25°C/60%RH | 3M | complies | 101.8 | 0.03 | 0.03 | ND | ND | 0.04 | 0.04 | 0.00 | 2.14 |
| (With Cotton) | 6M | complies | 104.1 | 0.05 | 0.04 | ND | ND | 0.04 | 0.06 | 0.11 | 2.61 |
| | 9M | complies | 102.9 | 0.06 | 0.04 | ND | ND | 0.04 | ND | 0.06 | 2.58 |
| 30°C/60%RH | 12M | complies | 104.9 | 0.08 | 0.03 | 0.02 | ND | 0.03 | 0.03 | 0.08 | 2.57 |
| | 3M | complies | 101.0 | 0.05 | 0.03 | 0.01 | ND | 0.04 | 0.05 | 0.05 | 2.06 |
| | 6M | complies | 103.4 | 0.08 | 0.04 | 0.03 | ND | 0.04 | 0.07 | 0.15 | 2.64 |
| | 9M | complies | 102.3 | 0.10 | 0.04 | 0.03 | ND | 0.03 | ND | 0.10 | 2.75 |
| | 12M | complies | 101.0 | 0.13 | 0.04 | 0.04 | 0.02 | 0.04 | 0.03 | 0.13 | 2.62 |
| 40°C/75%RH | 6W | complies | 102.8 | 0.07 | 0.03 | 0.03 | ND | 0.04 | 0.07 | 0.14 | 2.24 |
| | 3M | complies | 102.6 | 0.13 | 0.04 | 0.03 | ND | 0.04 | 0.05 | 0.13 | 2.63 |
| | 6M | complies | 103.1 | 0.25 | 0.05 | 0.08 | 0.02 | 0.04 | 0.07 | 0.45 | 3.58 |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not detected | | | | | | | | | | | |

**Table 3-11 Summary of dissolution data for vildagliptin 100mg tablets, AEUS/2002-0081, 30HDPE90**

| | **Percent released** | | | | | | | | | | |
|---|---|---|---|---|---|---|---|---|---|---|---|
| **Conditions** | **Tim e** | **0.5 hr** | **1hr** | **2 hr** | **4 hr** | **6 hr** | **8 hr** | **10 hr** | **12 hr** | **18 hr** | **24 hr** |
| | Initial | 13 | 21 | 33 | 49 | 61 | 70 | 77 | 83 | 94 | 98 |
| | | | | | | | | | | | |
| 25°C/60%RH | 3M | NT | | | | | | | | | |
| | 6M | NT | | | | | | | | | |
| | 9M | NT | | | | | | | | | |
| | 12M | NT | | | | | | | | | |
| 25°C/60%RH | 3M | NT | | | | | | | | | |
| (With cotton) | 6M | NT | | | | | | | | | |
| | 9M | NT | | | | | | | | | |
| 30°C/60%RH | 3M | NT | | | | | | | | | |
| | 6M | NT | | | | | | | | | |
| | 9M | 13 | | 33 | | 62 | | | | 95 | |
| | 12M | NT | | | | | | | | | |
| 40°C/75%RH | 6W | NT | | | | | | | | | |
| | 3M | 12 | | 31 | | 57 | | | | 89 | |
| | 6M | 12 | | 30 | | 56 | | | | 89 | |

| | | | | | | | | | | | |
|---|---|---|---|---|---|---|---|---|---|---|---|
| NT = Not tested Note: Dissolution was tested only at accelerated conditions as these represent worst case situation. | | | | | | | | | | | |

**Table 3-12 Summary of stability data for vildagliptin 200mg tablets, AEUS/2002-0082**

| | | | | | | **Degradation products** | | | | | |
|---|---|---|---|---|---|---|---|---|---|---|---|
| **Conditions** | **Time** | **Appearance** | **%Assay** | **% Amide** | **%Cyclic** | **%Diket.** | **%Unkn (RRT = 0.26)** | **%Unk (RRT 0.77)** | **%Unk (RRT = 1.10)** | **%Total** | **% Moisture** |
| Initial | 0 | complies | 100.6 | ND | ND | ND | - | | | - | 2.61 |
| 5°C | 3M | complies | 100.7 | 0.02 | 0.02 | ND | ND | 0.04 | 0.02 | 0.00 | 2.23 |
| | 6M | complies | 100.3 | 0.02 | 0.02 | ND | ND | 0.04 | 0.03 | 0.00 | 2.54 |
| | 9M | complies | 99.9 | 0.02 | 0.02 | ND | ND | 0.04 | ND | 0.00 | 2.46 |
| | 12M | NT | | | | | | | | | |
| 25°C/60%RH | 3M | complies | 100.6 | 0.04 | 0.04 | ND | ND | 0.04 | 0.02 | 0.00 | 2.16 |
| | 6M | complies | 100.1 | 0.06 | 0.05 | ND | ND | 0.04 | 0.04 | 0.11 | 2.50 |
| | 9M | complies | 99.9 | 0.08 | 0.06 | 0.02 | ND | 0.03 | ND | 0.14 | 2.56 |
| | 12M | complies | 99.9 | 0.09 | 0.05 | 0.03 | 0.02 | 0.03 | 0.02 | 0.14 | 2.53 |
| 25°C/60%RH | 3M | complies | 100.8 | 0.04 | 0.05 | ND | ND | 0.04 | 0.02 | 0.05 | 2.22 |
| (With Cotton) | 6M | complies | 99.3 | 0.06 | 0.05 | ND | ND | 0.04 | 0.04 | 0.11 | 2.59 |
| | 9M | complies | 99.1 | 0.08 | 0.05 | 0.02 | ND | 0.03 | ND | 0.14 | 2.64 |
| | 12M | complies | 100.3 | 0.10 | 0.06 | 0.03 | 0.02 | 0.03 | 0.02 | 0.16 | 2.63 |
| 30°C/60%RH | 3M | complies | 99.5 | 0.06 | 0.06 | ND | ND | 0.04 | 0.03 | 0.12 | 2.18 |
| | 6M | complies | 100.8 | 0.11 | 0.05 | 0.03 | ND | 0.04 | 0.04 | 0.16 | 2.59 |
| | 9M | complies | 100.4 | 0.16 | 0.06 | 0.03 | ND | 0.03 | ND | 0.22 | 2.75 |
| | 12M | complies | 99.2 | 0.19 | 0.05 | 0.05 | 0.02 | 0.03 | 0.02 | 0.29 | 2.77 |
| 40°C/75%RH | 6W | complies | 101.2 | 0.11 | 0.05 | 0.03 | ND | 0.03 | 0.06 | 0.22 | 2.31 |
| | 3M | complies | 99.3 | 0.20 | 0.05 | 0.05 | ND | 0.03 | 0.03 | 0.30 | 2.51 |
| | 6M | complies | 99.5 | 0.36 | 0.05 | 0.10 | 0.02 | 0.04 | 0.05 | 0.51 | 3.16 |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not detected | | | | | | | | | | | |

**Table 3-13 Summary of dissolution data for 200mg tablets, AEUS/2002-0082**

| | **Percent released** | | | | | | | | | | |
|---|---|---|---|---|---|---|---|---|---|---|---|
| **Conditions** | **Tim e** | **0.5 hr** | **1 hrs** | **2 hr** | **4 hr** | **6 hr** | **8 hr** | **10 hr** | **12 hr** | **18 hr** | **24 hr** |
| | Initial | 24 | 36 | 52 | 73 | 86 | 93 | 98 | 99 | 99 | 97 |
| 5°C | 3M | NT | | | | | | | | | |
| | 6M | NT | | | | | | | | | |
| | 9M | NT | | | | | | | | | |
| | 12M | NT | | | | | | | | | |
| 25-C/60%RH | 3M | NT | | | | | | | | | |
| | 6M | NT | | | | | | | | | |
| | 9M | NT | | | | | | | | | |
| | 12M | NT | | | | | | | | | |
| 25°C/60%RH | 3M | NT | | | | | | | | | |
| (With cotton) | 6M | NT | | | | | | | | | |
| | 9M | NT | | | | | | | | | |
| 30°C/60%RH | 3M | NT | | | | | | | | | |
| | 6M | NT | | | | | | | | | |
| | 9M | 23 | | 51 | | 84 | | | | 99 | |
| | 12M | NT | | | | | | | | | |
| 40-C/75%RH | 6W | NT | | | | | | | | | |
| | 3M | 24 | | 52 | | 87 | | | | 99 | |
| | 6M | 25 | | 54 | | 87 | | | | 99 | |

| | | | | | | | | | | | |
|---|---|---|---|---|---|---|---|---|---|---|---|
| NT = Not tested Note: Dissolution was tested only at accelerated conditions as these represent worst case situation. | | | | | | | | | | | |

**Table 3-14 Summary of dissolution data for 200mg tablets, AEUS/2002-0083**

| | | | | | | **Degradation products** | | | | | |
|---|---|---|---|---|---|---|---|---|---|---|---|
| **Conditions** | **Time** | **Appearance** | **%Assay** | **% Amide** | **%Cyclic** | **%Diket** | **%Unkn (RRT = 0.26)** | **%Unkn (RRT = 0.77)** | **%Unkn (RRT = 1.10)** | **%Total** | **% Moisture** |
| Initial | 0 | complies | 100.8 | ND | ND | ND | - | | | - | 2.36 |
| 5°C | 3M | complies | 100.8 | 0.02 | ND | ND | ND | 0.04 | 0.03 | 0.00 | 2.06 |
| | 6M | complies | 100.5 | ND | ND | ND | ND | 0.04 | 0.04 | 0.00 | 2.41 |
| | 9M | complies | 101.1 | ND | ND | ND | ND | 0.04 | 0.03 | 0.00 | 2.36 |
| | 12M | NT | | | | | | | | | |
| 25°C/60%RH | 3M | complies | 99.9 | 0.03 | 0.02 | ND | ND | 0.04 | 0.03 | 0.00 | 2.04 |
| | 6M | complies | 100.6 | 0.05 | 0.03 | ND | ND | 0.04 | 0.05 | 0.05 | 2.32 |
| | 9M | complies | 99.6 | 0.06 | 0.03 | ND | ND | 0.03 | 0.02 | 0.06 | 2.49 |
| | 12M | complies | 100.2 | 0.08 | 0.02 | 0.02 | 0.02 | 0.04 | 0.04 | 0.08 | 2.50 |
| 25°C/60%RH | 3M | complies | 100.5 | 0.03 | 0.03 | ND | ND | 0.04 | 0.04 | 0.05 | 2.09 |
| (With Cotton) | 6M | complies | 100.2 | 0.05 | 0.03 | ND | ND | 0.04 | 0.05 | 0.05 | 2.50 |
| | 9M | complies | 100.1 | 0.07 | 0.03 | ND | ND | 0.03 | ND | 0.07 | 2.66 |
| | 12M | complies | 100.0 | 0.08 | 0.03 | 0.02 | ND | 0.04 | 0.03 | 0.08 | 2.54 |
| 30°C/60%RH | 3M | complies | 100.4 | 0.05 | 0.03 | ND | ND | 0.04 | 0.04 | 0.05 | 2.07 |
| | 6M | complies | 100.3 | 0.08 | 0.03 | 0.02 | ND | 0.04 | 0.08 | 0.16 | 2.52 |
| | 9M | complies | 100.2 | 0.11 | 0.03 | 0.03 | ND | 0.04 | ND | 0.11 | 2.67 |
| | 12M | compiles | 100.3 | 0.13 | 0.02 | 0.05 | 0.02 | 0.04 | 0.03 | 0.18 | 2.71 |
| 40°C/75%RH | 6W | complies | 101.2 | 0.08 | 0.03 | 0.03 | ND | 0.03 | 0.08 | 0.16 | 2.24 |
| | 3M | complies | 100.7 | 0.14 | 0.03 | 0.03 | ND | 0.03 | 0.04 | 0.14 | 2.47 |
| | 6M | complies | 99.8 | 0.23 | 0.03 | 0.07 | 0.02 | 0.04 | 0.06 | 0.36 | 3.14 |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not detected | | | | | | | | | | | |

**Table 3-15 Summary of dissolution data for 200mg tablets, AEUS/2002-0083**

| | | | | | **Percent released** | | | | | | |
|---|---|---|---|---|---|---|---|---|---|---|---|
| **Conditions** | **Time** | **0.5 hr** | **1 hr** | **2 hr** | **4 hr** | **6 hr** | **8 hr** | **10 hr** | **12 hr** | **18 hr** | **24 hr** |
| | Initial | 11 | 18 | 27 | 40 | 50 | 59 | 66 | 71 | 84 | 91 |
| 5°C | 3M | NT | | | | | | | | | |
| | 6M | NT | | | | | | | | | |
| | 9M | NT | | | | | | | | | |
| | 12M | NT | | | | | | | | | |
| 25°C/60%RH | 3M | NT | | | | | | | | | |
| | 6M | NT | | | | | | | | | |
| | 9M | NT | | | | | | | | | |
| | 12M | NT | | | | | | | | | |
| 25°C/60%RH | 3M | NT | | | | | | | | | |
| (With cotton) | 6M | NT | | | | | | | | | |
| | 9M | NT | | | | | | | | | |
| 30°C/60%RH | 3M | NT | | | | | | | | | |
| | 6M | NT | | | | | | | | | |
| | 9M | 11 | | 27 | | 51 | | | | 84 | |
| | 12M | 10 | | 25 | | 48 | | | | 81 | |
| 40°C/75%RH | 6W | NT | | | | | | | | | |
| | 3M | 10 | | 25 | | 49 | | | | 81 | |
| | 6M | 10 | | 25 | | 48 | | | | 81 | |

| | | | | | | | | | | | |
|---|---|---|---|---|---|---|---|---|---|---|---|
| NT = Not tested Note: Dissolution was tested only at accelerated conditions as these represent worst case situation. | | | | | | | | | | | |

**Table 3-16 References for the stability data in Table 3-8 through Table 3-15**

| Reference | Assay & Purity | Moisture Content | Dissolution |
|---|---|---|---|
| Initial | WS17548 | TRD0958/109XG | WS19437,19427,19449,19455 |
| 6W | WS20721 | TRD0983/99XG | NA |
| 3M | WS20768 | WS20768 | WS21307, 20772, 21321 |
| 6M | WS21855 | WS21855 | WS22761, 22771, 22775 |
| 9M | WS23831 | WS23831 | WS22591, 22599 |
| 12M | WS25593 | WS25593 | WS25471, 25507 |

**Table 3-17 Summary of stability data for 100mg tablets, TRD-1926-078A (packaging: HDPE, 1g desiccant and CR cap)**

| | | | | | **Degradation products** | | | | | | |
|---|---|---|---|---|---|---|---|---|---|---|---|
| **Conditions** | **Time** | **Appearance** | **%Assay** | **% Amide** | **%Cyclic** | **%Diket.** | **%Unk. (RRT = 0.26)** | **%Unk (RRT = 0.77)** | **%Unk (RRT = 1.10)** | **%Total deg.** | **% Moisture** |
| | Initial | Complies | 102.0 | <LOQ | <LOQ | ND | ND | <LOQ | <LOQ | <LOQ | 2.39 |
| 40°C/75%RH | 6W | Complies | 98.3 | 0.10 | 0.06 | <LOQ | <LOQ | <LOQ | 0.06 | 0.22 | |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not Detected. Ref: Initial - TRD 1928/127 JT: 6W - TRD 1980/ | | | | | | | | | | | |

**Table 3-18 Summary of dissolution data for 100mg tablets, TRD-1926-078A (packaging: HDPE, 1g desiccant and CR cap)**

| | **Percent released** | | | | | | | | | | |
|---|---|---|---|---|---|---|---|---|---|---|---|
| **Conditions** | **Time** | **0.25 hr** | **0.5hr** | **1 hr** | **2 hr** | **4 hr** | **6 hr** | **8 hr** | **10 hr** | **12 hr** | **18 hr** |
| | Initial | 7.6 | 12.3 | 19.5 | 30.5 | 46.5 | - | 68.4 | 82.3 | 89.1 | 93.8 |
| 40°C/75%RH | 6W Not tested | | | | | | | | | | |

| | | | | | | | | | | | |
|---|---|---|---|---|---|---|---|---|---|---|---|
| Ref: WS35331 KE | | | | | | | | | | | |

**Table 3-19 Summary of stability data for 150mg tablets, TRD-1926-078B (packaging: HDPE, 1g desiccant and CR cap)**

| | | | | | **Degradation products** | | | | | | |
|---|---|---|---|---|---|---|---|---|---|---|---|
| **Conditions** | **Tim e** | **Appearance** | **%Assay** | **% Amide** | **%Cyclic** | **%Diket.** | **%Unk. (RRT = 0.26)** | **%Unk (RRT = 0.77)** | **%Unk (RRT = 1.10)** | **%Total deg.** | **% Moisture** |
| | Initial | Complies | 101.7 | <LOQ | <LOQ | ND | ND | <LOQ | <LOQ | <LOQ | 2.31 |
| 40°C/75%RH | 6W | Complies | 99.1 | 0.10 | 0.07 | <LOQ | ND | <LOQ | 0.06 | 0.23 | |

| | | | | | | | | | | | |
|---|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not Detected Ref: Initial - TRD1928/127 JT: 6W - TRD | | | | | | | | | | | |

**Table 3-20 Summary of dissolution data for 150mg tablets, TRD-1926-078B(packaging: HDPE, 1g desiccant and CR cap)**

| | **Percent released** | | | | | | | | | | |
|---|---|---|---|---|---|---|---|---|---|---|---|
| **Conditions** | **Time** | **0.25hr** | **0.5 hr** | **1 hr** | **2 hr** | **4 hr** | **6 hr** | **8 hr** | **10 hr** | **12 hr** | **18 hr** |
| | Initial | 6.8 | 11.1 | 17.7 | 28.3 | | 43.9 | 65.1 | 79.5 | 86.5 | 91.5 |
| 40°C/75%RH | 6W Not tested | | | | | | | | | | |

| | | | | | | | | | | | |
|---|---|---|---|---|---|---|---|---|---|---|---|
| Ref: WS35331 KE | | | | | | | | | | | |

**Table 3-21 Summary of assay and related substances data for the initial analysis of 1-[(3-Hydroxy-adamant-1-ylamino)-acetyl]-pyrrolidine-2(S)-carbonitrile MR tablets (technical batches)**

| | | | | **Degradation products** | | | | | | |
|---|---|---|---|---|---|---|---|---|---|---|
| **Batch no.** | **Appearance** | **%Assay** | **% Amide** | **%Cyclic** | **%Diket.** | **%Unk. (RRT= 0.26)** | **%Unk (RRT = 0.77)** | **%Unk (RRT = 1.10)** | **%Total deg.** | **% Moisture** |
| TRD-1971- 053C 100mg | Complies | 100.9 | ND | ND | ND | ND | <LOQ | ND | <LOQ | 2.44 |
| TRD-1971- 055A 150mg | Complies | 120.4 | ND | ND | ND | ND | <LOQ | ND | <LOQ | 2.31 |

| | | | | | | | | | | |
|---|---|---|---|---|---|---|---|---|---|---|
| NT = not tested, Cyclic = cyclic amidine, Diketo = diketopiperazine, LOQ = 0.05% for cyclic, diketo and amide, 0.06% for vildagliptin; LOD = 0.02%; ND = Not Detected Ref: TRD 1980/096 JT | | | | | | | | | | |

**Table 3-22 Summary of dissolution data for the initial analysis of vildagliptin MR tablets (technical batches)**

| | **Percent released** | | | | | | | | | |
|---|---|---|---|---|---|---|---|---|---|---|
| Batch no. | **0.25hr** | **0.5 hr** | **1 hr** | **2 hr** | **4 hr** | **6 hr** | **8 hr** | **10 hr** | **12 hr** | **18 hr** |
| TRD-1971-053C100mg TRD-1971-055A 150mg | | | | | | | | | | |
| Ref: | | | | | | | | | | |

### Specifications

Tentative analytical specifications were laid down in specifications during early development of vildagliptin 25mg, 50mg and 100mg tablets [AS6105B].

### III. Conclusions

A Clinical Service Form (CSF) was developed to provide a "slow" release profile containing 40% HPMC K100M as the controlled release polymer. A direct compression powder blend was developed for the 100mg and 150mg strengths. It was determined that the use of roller compaction improved the compression profile and bulk powder flow of the CSF. Roller compaction exhibits a dissolution profile similar to the CSF (which was tested in vivo in humans). The formulation does not appear to be effected by roller compaction roll speed, or dwell time on compression.

For the CSF 100mg, 150mg and 200mg-strengths, the stability data are available up to 12 months at 25°C/60%RH and 30°60%RH. The appearance, assay, purity and dissolution data were found to within specification limits after 12 months at 30°C/60%RH. These data support a 24 month re-test period with specification "do not store above 25°C" for 100mg, 150mg and 200mg modified release tablets. The composition of vildagliptin 150mg modified release tablets is bracketed by 100mg and 200mg strengths. Therefore, the stability of 150mg tablets is expected to be same as 100mg and 200mg strengths. Thus a re-test period similar to that of 100 and 200mg strengths is applied for 150mg tablets. The stability profile of 100mg and 150mg tablets manufactured by roller compaction after 6W, 40°C/75%RH was found to be comparable to that of CSF formulations. Thus, the stability does not appear to be affected by the inclusion of roller compaction step in the manufacturing process.

The roller compacted formulation containing 40% or 30% HPMC K100M has been selected.

Preferred formulations are described in the below table:

| | | | | |
|---|---|---|---|---|
| | **100** | | **150** | |
| Component | mg/ unit | % | mg/ unit | % |
| Vildagliptin | 100 | 25.0 | 150 | 25.0 |
| Microcrystalline cellulose, PH102 | 120 | 30.0 | 180 | 30.0 |
| Lactose, anhydrous DT | 16 | 4.0 | 24 | 4.0 |
| Hydroxypropylmethylcelluose K100M | 160 | 40.0 | 240 | 40.0 |
| Magnesium stearate | 4 | 1.0 | 6 | 1.0 |
| Total tablet weight (mg) | 400 | | 600 | |
| KN number | 3768652 | | 6001732 | |
| Tablet diameter, shape | 11 mm, FFBE | | 17x6.7mm, ovaloid | |

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the appended claims, the invention may be practiced otherwise than as specifically described.

### Example A : Mechanical stress (particle size distribution)

The material in the desired particle size range can be produced from any form of LAF237 e.g. amorphous form or crystal form, by mechanical stress. This stress can be mediated by impact, shear or compression. In most commercially available grinding equipment a combination of these principles occurs. For LAF237 preferably a mechanical impact or jet mill is used. The most preferable mechanical impact mill can be equipped with different kind of beaters, screens, liners or with pin plates. For our process preferably an impact mill with plate beater and a slit screen 5 * 2.5 cm is used. The impact speed should be variable between 20 and 100 m/s (as peripheral speed) to adapt to any batch to batch variation. In our case a peripheral speed of the beater of about 40 - 50 m/s is used.

### Pharmacokinetic and Pharmacodynamic study:

- AUC: area under the concentration time curve
- AUC₀₋ₜ: The area under the plasma concentration-time curve from time zero to the last quantifiable data point t [ng*hr/mL]
- AUC_{0-inf} or: The area under the plasma concentration-time curve from time zero to infinity
- AUC _{(0-∞)}: [ng*hr/mL]
- BAPK: Bioanalytics and Pharmacokinetics section
- Cₘₐₓ: maximum plasma concentration
- CRF: case report / record form
- CRO: Clinical Research Organization
- CV: Coefficient of variation
- ECG: Electrocardiogram
- DPP-4: dipeptidyl peptidase 4; dipeptidyl peptidase IV
- FMI: Final Market Image
- GLP-1: glucagon-like peptide 1
- ICH: International Council on Harmonization
- IRB: Institutional Review Board
- Lay237: Vildagliptin
- LC-MS/MS: Liquid chromatography-mass spectrometry/mass spectrometry
- LOQ: limit of quantitation
- o.d.: once a day
- PD: Pharmacodynamics
- PK: Pharmacokinetics
- p.o: per os / by mouth / orally
- QC: Quality Control
- SOP: Standard Operating Procedures
- SD: standard deviation
- tₘₐₓ: time to reach Cₘₐₓ
- t_{½}: elimination half-life
- Vd/f: volume of distribution, corrected for the absolute bioavailability

### Study synopsis and assessment schedule

Title of study: A randomized, open-label, three period, cross-over study to evaluate the pharmacokinetics, pharmacodynamics, safety, and tolerability, of two new LAF237 100mg and 150mg modified release formulations in patients with type 2 diabetes.

### Objectives:

### Primary objective

- To evaluate the pharmacokinetic profiles (single and multiple dose) after oral administration of two dosage strengths (100mg and 150mg) of a modified release formulation of LAF237 in patients with type 2 diabetes.
- To evaluate the effect of a modified release formulation of LAF237 at two dosage strengths (100mg and 150mg) (single and multiple dose) on DPP-IV activity.
- To evaluate the safety and tolerability of the modified release formulations after oral administration for 7 days to patients with type 2 diabetes.

### Secondary objective(s)

- To evaluate the effect of a modified release formulation of LAF237 at two dosage strengths (100mg and 150mg) on the glucose profile after 7 days of treatment.
- To compare the pharmacokinetic profiles of the modified release formulations of LAF237 at two dosage strengths when given with and without an ADA breakfast.

**Design:** This study is a single center, randomized, open-label, three-period, crossover design study. A total of 27 patients with type 2 diabetes (both male and female) are enrolled. Each subject participate in a 28 day screening period, three base-line periods, three 11-day treatment periods separated by two 7-day washout periods and a study completion evaluation.

Subjects are randomized to the following 3 treatment sequence groups under William's design.

| **Sequence** | **Period 1** | **Period 2** | **Period 3** |
|---|---|---|---|
| I | A | B | C |
| II | B | C | A |
| III | C | A | B |

### • Treatment A:

- Day 1:100mg LAF237 MR OD, single dose
- Days 3-10: 100mg LAF237 MR OD, for 8 days
- Day 11-1,7: Wash out

### • Treatment B:

- Day 1: 150mg LAF237 MR OD, single dose
- Days 3-10: 150mg LAF237 MR OD, for 8 days
- Day 11-17: Wash out

### • Treatment C:

- Day 1: 50mg LAF237 BID, two doses
- Days 3-10: 50mg LAF237 BID, for 8 days
- Day 11-17: Wash out

Patients receiving metformin therapy are required to stop taking metformin for at least 21 days prior to dosing on Day 1. On the evening of Day -1, patients who satisfy all inclusion/exclusion criteria at screening (Days -28 to -2) enter the study center for verification of inclusion/exclusion criteria, baseline evaluations and subsequent randomization to one of three sequences. Patients remain domiciled until the morning of Day 3 (48 h after the first dose of LAF237 is given).

On Day 1 of each treatment period, patients receive the first dose of LAF237 following an over night fast of 10-12 hours. They continue to fast for 5 hours post-dose. Patients are discharged from the site after the Day 3 morning dose of LAF237 with study drug medication, instructions on how to administer drug at home during the out-patient treatment days (Days 3 to 7) and a dosing diary.

The modified release formulation of LAF237 (100mg and 150mg) are administered once daily before breakfast and the 50mg LAF237 is administered twice daily, once before breakfast and once before dinner. A dosing diary is kept by the patients for all non-domiciled doses.

Patients return to the site on the morning of Day 5 for pre-dose pharmacokinetic sample collection. They are admitted to the site at least 10 h prior to the Day 8 morning dose of LAF237 and remain domiciled until the morning of Day 12 (48 h after the Day 10 dose is given). On Day 9, patients are served an ADA breakfast within 30 minutes of the morning dose of LAF237.

Meal times on all domiciled days are strictly enforced. Breakfast are served within 30 minutes of the morning dose, except on days 1 and 10 when patients fast for 5 hours after dosing. Lunch, dinner and a snack are served 5, 10 and 12 hours post-dose, respectively.

| Screening | Periods One, Two, and Three | | | | | |
|---|---|---|---|---|---|---|
| Days -28 to -2 | Day -1 | Days 1-2 | Days 3-8 | Day 9 | Day 10-11 | Days 12-17 |
| Patients receiving metformin required to stop therapy on Day -21 | B/L | 48 hr Sampling under fasted conditions | Day 3: Start of multiple dosing | 24 hr Sampling, ADA Breakfast served | 48 hr Sampling under fasted conditions | w/o |
| | | PK profile | Days 5 and 8: Pre-dose PK sampling | PK profiling | PK profiling | (Not required after Period 3) |
| | | DPP-IV profile | Days 3-7: Out patient Day 8: Domiciled | DPP-IV profiling | DPP-IV profiling | |
| | | | | Glucose profiling | | |

**Number of subjects:** 27 patients with type 2 diabetes (male and female)

### Criteria for inclusion:

- Male and female (postmenopausal, surgically sterile or using double-barrier method of contraception) type two diabetic patients between 20 and 65 years of age.
- Hb1Ac range between 6.5 and 9%.
- Body mass index between 22 - 40 kg/m2.
- Patients must be drug-naive to diabetic medications or on metformin treatment.
- Patients must be able to complete a 3 week wash-out of current metformin therapy. Patients will monitor FPG at home and call PI if values exceed 250mg/dL.
- Patients must not be taking prescription or non-prescription medications.
- Patients must have a fasting blood glucose of 7.0 -12.2 mmol/dL (100-240 mg/dL) at screening and at predose on Day 1.
- Patients on diuretics or cyclic hormone therapy must be on stable dose (at least 3 months prior to screening) and the maintenance dose may not be adjusted during the study.
- All subjects must be able to provide written informed consent prior to participation the study.

### Criteria for exclusion:

- History of type 1 diabetes mellitus, diabetes that is a result of pancreatic injury, or secondary forms of diabetes e.g. Cushing's syndrome and acromegaly.
- Use of thiazolindiones in the previous 3 months.
- History of ketoacidosis.
- Need for insulin within the previous 3 months.
- Significant concomitant diseases or complications of diabetes.
- Fasting triglycerides >5.1 mmol/L (>450 mg/dL) within the past 4 weeks.
- Treatment with systemic steroids.
- Patients with any history of gastrointestinal surgery e.g. partial bowel resections, partial gastric resections, etc.

### Investigational drug:

LAF237 100mg MR (herein described preferred modified release MR formulation)
LAF237 150mg MR (herein described preferred modified release MR formulation)
LAF237 50mg (as described in the US provisional patent application No. 60/604274)

**Duration of treatment:** 46 days total study length, including washout periods.

**Treatment A (LAF237 100mg MR OD):** Single dose treatment on Day 1and 8 days of multiple dose treatment starting on Day 3.

**Treatment B (LAF237 150mg MR OD):** Single dose treatment on Day 1 and 8 days of multiple dose treatment starting on Day 3.

**Treatment C (50mg BID):** BID dose treatment on Day 1 and 8 days of multiple dose treatment starting on Day 3.

Two 7 day wash out periods between above treatments.

### Assessments and evaluations:

### Background, demographic and administrative assessments

- Inclusion/exclusion criteria; Relevant medical history/Current medical conditions: screening, review at baselines
- Demography: screening
- Physical examination: screening, baselines, end of study
- Hepatitis screen, HIV screen: screening
- Alcohol test, Drug screen, Urine cotinine: screening, baselines
- Pregnancy test: screening, baselines, end of study
- Drug administration record: each time study drug is administered
- Meal record: days 1, 2, 8, 9, 10 and 11.
- Study completion information: end of study
- Comments: as required

### Safety and tolerability assessments

- Vital signs and body measurements
- Body height: screening
- Body weight: screening, baselines, end of study
- Body temperature: screening, baselines, end of study
- Blood pressure, pulse rate: screening*, baseline*, predose and 1, 6, 12, and 24 h postdose on Days 1, 2, 8, 9, and 10; end of study (Note: measurements are supine, or supine and after 3 min standing if indicated by an *asterisk)
- ECG evaluation: screening, baselines, end of study
- Hematology; Blood chemistry; Urinalysis: screening, baselines, end of study
- Adverse events; Concomitant medications/Significant non-drug therapies: from time of first administration of study drug until end of study

### Pharmacokinetic assessments

- Blood collection (2 mL blood per sample, heparin tubes (plasma)):
   On Days 1, 9*,10: pre-dose, 0.5, 1, 1.5, 2, 2.5, 3, 4, 6, 8,10,12,14,16,24,36, and 48 hr post-dose
   On Days 5 and 8: predose.
      *Samples collected to 24 hr postdose on Day 9. The Day 10 predose sample will also serve as the Day 9 24 hr sample
- Analytes, media and methods: LAF237 in plasma will be determined by LC-MS-MS with LOQ at 2ng/mL.
- PK parameters: AUC₀₋ₜ, AUC (0-∞), Cₘₐₓ, tₘₐₓ, t_{½}, and Cₘₐₓ/AUC₍₀₋ₜ₎ will be evaluated for each treatment period.

### Pharmacodynamic assessments

DPP-IV activity will be measured on Days 1, 9 and 10.
- Blood collection (1 mL blood per sample, EDTA tubes (plasma)):
   On Days 1, 9* and 10: pre-dose, 0.25, 0.5, 0.75,1,2,4,6,8,10, 12,14,16,24,36 and 48 hr post-dose
      *Samples collected to 24 hr post-dose on Day 9. The Day 10 predose sample will also serve as the Day 9 24 hr sample.

### Glucose samples on Day 9.

- Blood Collection (2 mL blood per sample, sodium fluoride tubes (plasma)) Following morning dose- 0.75, 1, 1.25, 1.5, 2, 2.5, 3, 4, 5 (pre-lunch meal), 5.5, 5.75, 6, 6.5, 7, 8, 9.75 (prior to evening dose and pre-dinner meal), 10.25, 10.75, 11, 11.25, 11.5, 12, 12.51 13, 14, 16 and 24 hr post-dose.

### Statistical methods

**Sample Size:** The sample size is determined based on a two one-sided t-test at 5% significance level (Chen et. al. 1997) for PK parameters. When test-reference ratio is equal to 0.95, a sample size of 24 subjects allow at least 88% power to meet the bioequivalence criterion, i.e., to have the 90% Cl for test-reference ratio of a bioavailability measure contained in the bioequivalence range of (0.8, 1.25) if intrasubject CV is not greater than 0.20, or 72% power if the intra-subject CV is equal to 0.25. The intrasubject CV values used in the sample size determination are derived from a previous study of similar formulation (LAF237A2214) in healthy subjects in which the largest observed intrasubject CV is around 0.20 for Cmax.

**Statistical analysis:** An analysis of variance (ANOVA) are performed on log-transformed AUC and Cmax data using the PROC MIXED SAS procedure. The sources of variation included in the ANOVA model are sequence, subject (sequence), period, and treatment, with subject (sequence) as random effect. Using the ESTIMATE statement of the PROC MIXED SAS procedure, the contrast are constructed between the test and the reference treatments to obtain the p-value, the estimated mean difference, an the 90% confidence interval (CI) for the log-scale test-reference difference. The anti-logs of the estimated mean difference and the 90% Cl constitute the ratio of geometric means and the 90% Cl for the true test-reference ratio. The outputs from the comparison are tabulated.

For comparison of bioavailability between MR OD and IR BID, the test will be the MR OD and the reference are IR BID. The LAF237 AUC and Cmax are compared between the two treatments within the ANOVA.

The PD endpoints are analyzed using the same ANOVA model as described above.

### Drug administration

On Days 1, 3, 5, 8, 9 and 10 study medication arre administered by the study center personnel with 240 mL of water between 0700 and 0800, after at least a 10-hour fast. All subjects are dosed within a maximum of a 1-hour interval. Subjects must be instructed not to chew the medication, but to swallow it whole. The investigator has to check each subject's mouth to ensure that the medication was swallowed. Unless performing a study assessment, subjects have to rest quietly in the upright position for the next 4 hours. With the exception of Days 1 and 10 when the first meal is served 5 hours post-dose, an ADA breakfast is served within 30 minutes of dosing.

During Treatment C, patients receive the evening dose of 50mg LAF237 30 minutes prior to dinner.

### General study conditions and restrictions

### Definition of study phases

### Screening

"Screening" is defined as assessments made for 2 to 28 days prior to initiation of the study (i.e. prior to the day of first dosing, Day 1) to determine eligibility for entry into this study. Potentially eligible subjects whose screening examinations show any disqualifying abnormality may have the examination repeated ONCE if the investigator wishes to enroll the subject.

### Baseline

If not yet domiciled, subjects should be admitted to the study site at least 10-hours prior to dosing in each treatment period. "Baseline" is defined as the period of a subject's continuous presence at the study facility to 30 minutes prior to the administration of the study drug (i.e.

Day -1). Baseline activities include safety assessments, as outlined in the Study synopsis and on the Assessment schedule.

### Treatment period(s)

Each treatment period includes the predose activities (within 30 minutes prior to dosing), drug administration, the postdose evaluations until 24-hours after dosing, and - if applicable - the washout period until predose of the following treatment period.

### End of study

End of study evaluations will be performed on the last day of the last treatment period, prior to discharge from the study site.

### Domiciling and physician coverage

Patients are confined to the study center for at least 10 hours before administration of study drug on Day 1 until one hour after study drug is administered on Day 3. Patients return to the study center on the morning of day 5 for pre-dose blood collection. Patients are domiciled at least 10 hours before study drug is administered on Day 8 until 48 hours after the Day 10 morning dose of LAF237 is received.

Presence of a physician on site is expected at a minimum for the first 2 hours after dosing during each period. A physician shall be available by pager at all other times throughout the study.

### Dietary, fluid and other restrictions

During recruitment, informed consent review, and baseline .period, the subjects is informed and reminded of the following restrictions:
- No strenuous physical exercise (e.g., weight training, aerobics, football) for 7 days before dosing until after the end of study evaluation.
- Diet should be free of read meat for at least 72 hours prior to the study entry until the end of study.
- No alcohol for 72 hours before dosing until after the end of study evaluation.
- Intake of xanthine (e.g. caffeine) containing food or beverage must be discontinued 49 hours prior to dosing. Consumption of such food and beverage (i.e. coffee, tea, soda, chocolate) is not permitted at any time while the subjects are domiciled. If a deviation occurs during the domiciled period, it must be noted on the Comments CRF page.

On all domiciled days, except Days 1 and 10, patients receive the study drug 30 minutes prior to an ADA breakfast and an overnight fast of 10 hours. On Day 1 and 10, no breakfast is served.

During Treatment C, patients also receive the evening dose of study drug 30 minutes prior to dinner. The tested drug should be administered with 240 mL (8 fl oz) of water. No fluid intake apart from the fluid given at the time of drug intake is allowed from 2 h before until 2 h after dosing. Otherwise, subjects should have a fluid intake of at least 200 mL every 4 hours during waking hours in addition to fluid taken with meals and medication.

During domiciled days, lunch and dinner are served at 5 hours and 10 hours post dose, respectively, and a large snack is served at 12 hours post dose.

Subjects follow a standard weight maintaining diet while domiciled on non-dosing days. No other food is consumed at any time during confinement. Subjects should consume the entire contents of the meal. Meals should be similar in caloric content and distribution for all subjects on the day of dosing.

When meal and blood draw times coincide, blood is drawn BEFORE the meal is provided.

Intake of xanthine (e.g., caffeine) containing food or beverages must be discontinued 48 hours before dosing. Consumption of such foods and beverages (i.e., coffee, tea, and soda, chocolate) is not permitted at any time while the subjects are domiciled. If a deviation occurs during the domicile period, it must be noted on the Comments CRF page.

### Background, demographic and administrative assessments

### Inclusion/exclusion criteria

Subject selection is to be established by checking through all standard inclusion/exclusion criteria. A relevant record (e.g. checklist) must be stored with the source documentation at the study site. Violation of any entry criterion excludes a subject from enrollment into the study unless granted specific clearance by the sponsor.

### Drug administration record

Date and time of dose administration is recorded in the Dosage administration record section of the CRF and - if applicable on the Blood collection CRF page for pharmacokinetics and pharmacodynamic evaluations.

### Meal record

The date and start time of meal consumption is recorded in the appropriate section of the CRF for all domiciled days.

### Study completion information

Information on the date the subject last took drug, the subject's completion or discontinuation of the study and the reason for discontinuation of the study is recorded on the Study completion CRF page.

### Pharmacokinetic assessments

Blood collection : All blood samples is taken by either direct venipuncture or an indwelling cannula inserted in a forearm vein.

**LAF237**: For each scheduled LAF237 sample, collect a 2 mL blood sample into a sodium or lithium heparin tube at the times specified in the assessment schedule.

Handling of blood samples

Immediately after each tube of blood is drawn, it should be inverted gently several times to insure the mixing of tube contents (e.g., anticoagulant). Avoid prolonged sample contact with the rubber stopper. Place the tube upright in a test tube rack (e.g. surrounded by ice or at room temperature) until centrifugation. Within 15 minutes, centrifuge the sample between 3 and 5°C for 15 minutes at approximately 2500 rpm. Transfer all available plasma to a polypropylene screw-cap tube and freeze at -70°C or below within 60 minutes of venipuncture.

Analytical method(s)

Analytes, media and methods: LAF237 in plasma by LC-MS-MS; LLOQ at 2 ng/mL.

**Pharmacodynamic assessments:** All blood samples are taken either by direct venipuncture or an indwelling cannula inserted in a forearm vein from each patient.

Glucose: For each scheduled glucose sample, collect a 2 mL blood sample into a sodium flouride (grey top) tube at the times specified in the assessment schedule. The central laboratory to be used for analysis of plasma glucose samples has not been determined at this point. Once determined, a protocol supplement do outline the sample handling and data transfer procedures.

**DPP-IV:** DPP-IV measurements is conducted by Novartis Laboratory. For each scheduled sample collection for DPP-4 enzymatic analysis, collect a 1 ml blood sample into a tube containing potassium EDTA. Invert gently several times to mix the contents of the tube. Avoid prolonged sample contact with the rubber stopper. Place the tube upright in rack surrounded by ice until centrifugation. Within 15 minutes after collection, centrifuge the sample between 3 and 5°C for 15 mins at approximately 2500 rpm. Transfer all available plasma to a polypropylene screw-cap micro tube and freeze at -70°C or below within 60 minutes of venipuncture

For labeling and shipment instructions, see Part B, Section 9.

### Data analysis

Analysis of the data will be under the direction of Novartis personnel.

### General statistical considerations

### Sample size determination

The sample size is determined based on a two one-sided t-test at 5% significance level (Chen et. al. 1997 - Chen KW, Chow SC, Li G (1997). A note on sample size determination for bioequivalence studies with higher-order crossover designs. J Parmarcokin, Biopharm. 25: 753-765.) for PK parameters. When test-reference ratio is equal to 0.95, a sample size of 24 subjects allows at least 88% power to meet the bioequivalence criterion, i.e., to have the 90% CI for test-reference ratio of a bioavailability measure contained in the bioequivalence range of (0.8, 1.25) if intrasubject CV is not greater than 0.20, or 72% power if the intra-subject CV is equal to 0.25. The intrasubject CV values used in the sample size determination are derived from a previous study of similar formulation [LAF237A2214] in healthy subjects in which the largest observed intrasubject CV is around 0.17 for Cₘₐₓ.

### Background, demographic and administrative data analysis

Descriptive statistics are provided for background and demographic variables such as age, weight; height, gender and race.

Relevant medical history, current medical conditions, results of laboratory screens, drug tests and any other relevant information are listed.

### Safety and tolerability data analysis

All subjects who received at least one treatment are included in the safety and tolerability evaluation.

### Pharmacokinetic data analysis

All completed subjects are included in the pharmacokinetic data analysis.

### Pharmacokinetic variables

The following pharmacokinetic parameters are evaluated for each treatment period: AUC₀₋ₜ, AUC_{(0-∞)}, Cₘₐₓ, tₘₐₓ, t_{½}, and Cₘₐₓ/AUC₍₀₋ₜ₎.

Biofluid concentrations are expressed in mass per volume units. All concentrations below the limit of quantitation or missing data are labeled as such in the concentration data listings. Concentrations below the limit of quantitation are treated as zero in summary statistics and for the calculation of pharmacokinetic parameters.

Descriptive statistics of pharmacokinetic parameters include mean, SD, and CV, min and max. When a geometric mean is presented it is stated as such. A range of values is presented for selected variables. Since tₘₐₓ is generally evaluated by a nonparametric method, median values and ranges are given for this parameter.

Pharmacokinetic parameters are determined based on non-compartmental method(s) using WinNonlin Pro.

### Statistical methods for pharmacokinetic analyses

An analysis of variance (ANOVA) are performed on log-transformed AUC and Cmax data using the PROC MIXED SAS procedure. The sources of variation included in the ANOVA model are sequence, subject (sequence), period, and treatment, with subject (sequence) as random effect. Using the ESTIMATE statement of the PROC MIXED SAS procedure, the contrast are constructed between the test and the reference treatments to obtain the p-value, the estimated mean difference, an the 90% confidence interval (CI) for the log-scale test-reference difference. The anti-logs of the estimated mean difference and the 90% CI constitute the ratio of geometric means and the 90% CI for the true test-reference ratio. The outputs from the comparison are tabulated.

For comparison of bioavailability between MR OD and IR BID, the test treatment is the MR OD and the reference is IR BID. The LAF237 AUC and Cmax are compared between the two treatments within the ANOVA.

### Pharmacodynamic data analysis

All subjects who complete the trial with evaluable pharmacodynamic (PD) measurements are included in the data analysis.

### Pharmacodynamic variables

Following glucose parameters may be derived for treatment comparison:
- AUE: area under the effect-time curve from time 0 to last available observation by the linear-trapezoidal rule
- Eₘₐₓ: maximal effect (minimum value)
- tₘₐₓ: time to the first occurrence of the maximal effect.

In order not to lose data from subjects due to missing values, the algorithm below are employed to replace missing values:
- If the first or the last observation of a subject's record is missing then they are replaced by the second or the previous observation.
- If an observation is missing within a series of non-missing observations, then the missing values are substituted by the result of a linear regression based on its neighbors.
- If two consecutive observations are missing, then the record is completely ignored.

### Statistical methods for pharmacodynamic analyses

The glucose parameters are analyzed using the same ANOVA model as described above for statistical analysis of PK parameters.

In addition, timepoint-wise comparison of glucose concentration are performed using the same ANOVA model as described above.

### Results:

### Pharmacokinetic results (described on figures 24, 25, 26)

| **Treatment** | **Tmax (h) median (min, max)** | **Cmax (ng/mL) mean ± SD (CV%)** | **AUC a (h·ng/mL) mean ± SD (CV%)** | **CL/F (L/h) mean ± SD (CV%)** | **t½ (h) mean ± SD (CV%)** |
|---|---|---|---|---|---|
| **Day 1 (Single Dose Fasted) (N=27)** | | | | | |
| LAF237 100 mg MR OD | 4.00 (1.50, 6.00) | 205 ± 47 (23) | 1449 ± 376 (26) | 73.60 ± 19.60 (27) | 11.69 ± 5.68 (49) |
| LAF237 150 mg MR OD | 4.00 (1.50, 6.00) | 257 ± 59 (23) | 2271 ± 925 (41) | 75.63 ± 28.54 (38) | 10.75 ± 5.01 (47) |
| LAF237 50 mg MR BID | 1.00 (0.50, 4.00) | 278 ± 75 (27) | 2159 ± 425 (20) | 45.14 ± 7.91 (18) | 2.43 ± 1.12 (46) |

| **Day 9 (Multiple Dose Fed) (N=27)** | | | | | |
|---|---|---|---|---|---|
| LAF237 100 mg MR OD | 2.00 (1.00, 6.00) | 200 ± 64 (32) 64 | 1489 ± 488 (33) | 75.45 ± 29.07 (39) | 8.70 ± 3.75 (43) |
| LAF237 150 mg MR OD | 2.50 (1.00, 6.00) | 272 ± 111 (41) | 2242 ± 965 (43) | 78.84 ± 31.30 (40) | 10.34 ± 5.06 (49) |
| LAF237 50 mg BID | 1.00 (1.00, 4.00) | 285 ± 91 (32) | 1992 ± 461 (23) | 50.92 ± 16.51 (32) | 2.83 ± 0.62 (22) |

| **Day 10 (Multiple Dose Fasted) (N=27)** | | | | | |
|---|---|---|---|---|---|
| LAF237 100 mg MR OD | 3.00 (1.00, 6.00) | 215 ± 68 (31) | 1638 ± 535 (33) | 66.77 ± 20.87 (31) | 11.80 ± 5.69 (48) |
| LAF237 150 mg MR OD | 4.00 (2.50, 6.00) | 309 ± 91 (29) | 2458 ± 815 (33) | 67.19 ± 21.27 (32) | 10.54 ± 5.39 (51) |
| LAF237 50 mg MR BID | 2.00 (0.50, 9.97) | 257 ± 60 (23) | 2198 ± 492 (22) | 44.92 ± 10.97 (24) | 3.38 ± 2.62 (77) |
| a: AUCO-inf on Day 1, AUCO-24 on Days 9 and 10, AUCO-24 on Day 1 for LAF237 50 mg BID | | | | | |

### Results:

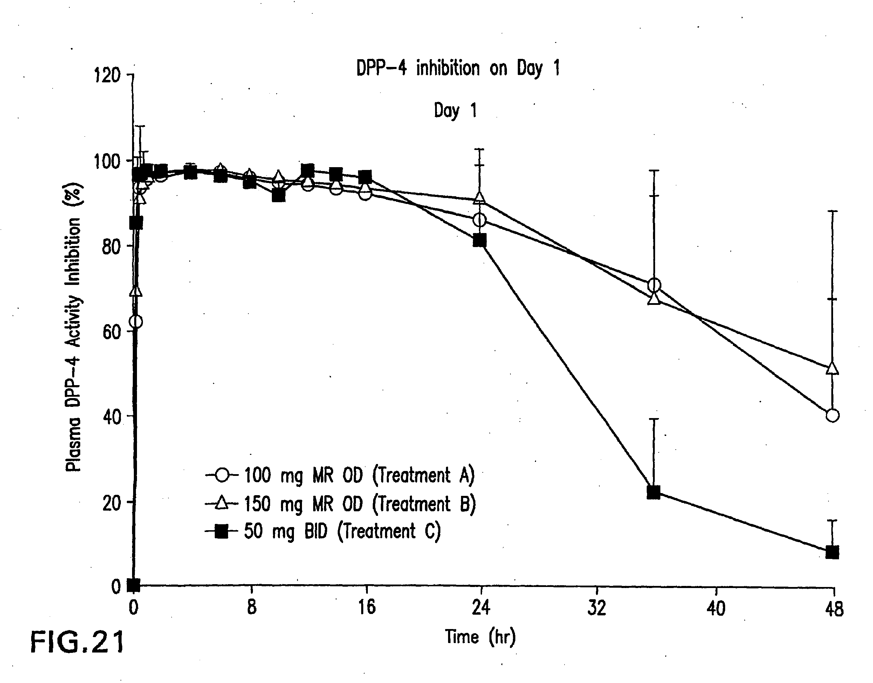

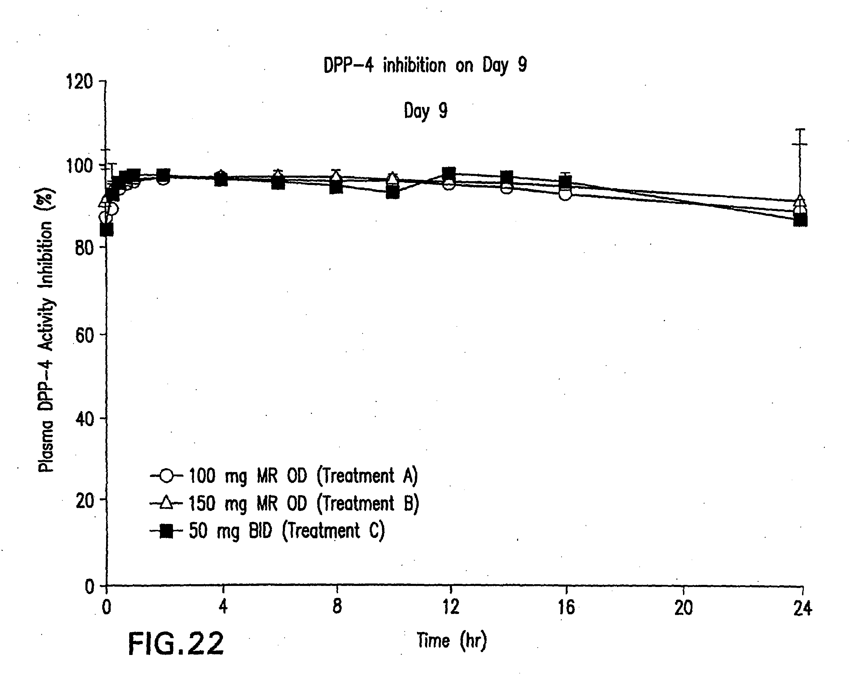

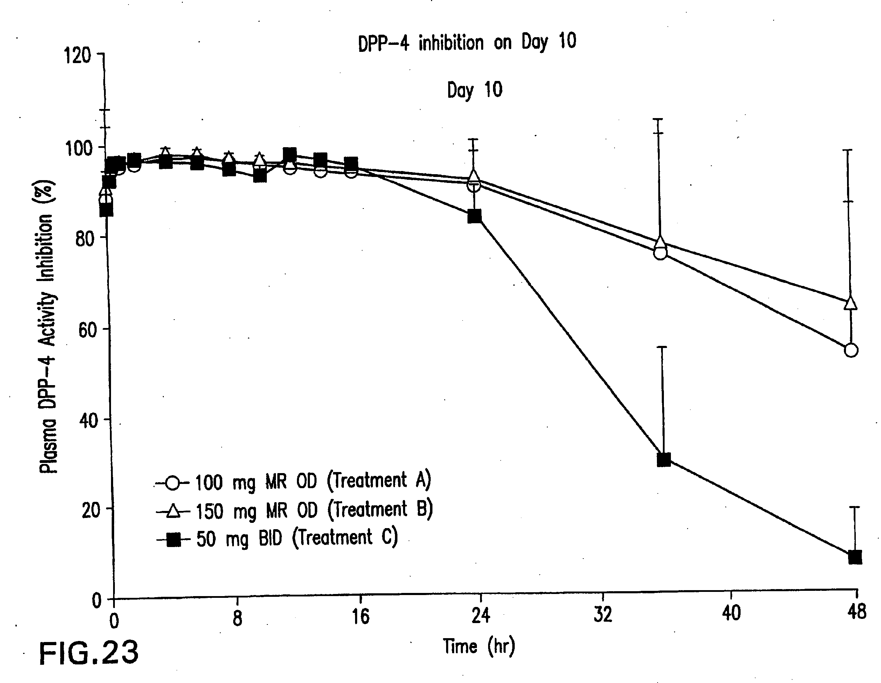

### Pharmacodynamic results (described on figures 21, 22, 23)

| **Treatment** | **DPP-4-AUC24 (%.h) mean ± SD (CV%** | **DPP-4-MRT (h) mean ± SD (CV%)** | **DPP-4-Avg (%) mean ± SD (CV%)** | **DPP-4-24h (%) mean ± SD (CV%)** |
|---|---|---|---|---|
| **Day 1 (Single Dose Fasted) (N=27)** | | | | |
| LAF237 100 mg MR OD | 2208 ± 73 (3) | 11.8 ± 0.3 (3) | 92.01 ± 3.03 | 85.64 ± 12.76 |
| LAF237 150 mg MR OD | 2242 ± 70 (3) | 11.9 ± 0.3 (3) | 93.43 ± 2.93 (3) | 90.04 ± 11.91 (13) |
| LAF237 50 mg MR BID mg MR | 2225 ± 48 (2) | 11.7 ± 0.2 (2) | 92.69 ± 2.01 (2) | 80.92 ± 9.03 (11) |
| **Day 9 (Multiple Dose Fed) (N=27)** | | | | |
| LAF237 100 mg MR OD | 2240 ± 98 (4) | 11.8 ± 0.5 (4) | 93.35 ± 4.09 | 87.78 ± 16.37 (19) |
| LAF237 150 mg MR OD | 2270 ± 86 (4) | 11.8 ± 0.5 (4) | 94.59 ± 3.57 (4) | 90.40 ± 17.50 (19) |
| LAF237 50 mg BID | 2252 ± 33 (1) | 11.8 ± 0.1 (1) | 93.85 ± 1.36 (1) | 86.15 ± 4.78 (6) |
| **Day 10 (Multiple Dose Fasted) (N=27)** | | | | |
| LAF237 100 mg MR OD | 2261 ± 40 (2) | 11.9 ± 0.2 (1) | 94.21 ± 1.66 (2) | 90.20 ± 7.35 (8) |
| LAF237 150 mg MR OD | 2281 ± 49 (2) | 11.9 ± 0.2 (2) | 95.06 ± 2.03 | 91.64 ± 8.47 (9) |
| LAF237 50 mg MR BID | 2243 ± 46 (2) | 11.7 ± 0.2 (1) | 93.44 ± 1.94 (2) | 82.80 ± 7.39 (9) |

## Claims

1. A pharmaceutical tablet formulation comprising per unit dosage form e.g. per tablet the following ingredients:
(a) a compound as an active ingredient, wherein the compound is (S)-1-[(3-hydroxy-1-adamantyl)amino]acetyl-2-cyano-pyrrolidine (vildagliptin) or the corresponding amount of any of its salts; and
(b) a hydroxypropyl methylcellulose with an apparent viscosity of 80,000 cP to 120,000 cP (nominal value 100,000 cP) when present in a 1% solution,
and optionally a filler and/or a lubricant.

2. A pharmaceutical tablet formulation according to claim 1, wherein in the unit dosage form, the ratio of the weight of vildagliptin and any of its corresponding salts to the weight of hydroxypropyl methylcellulose is of 0.16 to 2.5, preferably 0.3 to 1.16 or 0.4 to

3. A pharmaceutical tablet formulation according to any one of claims 1 or 2 comprising;
(a) 15-55% preferably 25-45% by weight on a dry weight basis of a pharmaceutically acceptable filler; and optionally
(b) 0.1-10% preferably 0.1-3% by weight on a dry weight basis of a pharmaceutically acceptable lubricant.

4. A pharmaceutical tablet formulation according to any one of claims 1 to 3, wherein the filler is lactose and/or microcrystalline cellulose and/or the lubricant is magnesium stearate.

5. A pharmaceutical tablet formulation according to claim 4, wherein the lactose is present in an amount from 1% to 8% by weight.

6. A pharmaceutical tablet formulation according to any one of the preceding claims,
wherein the compound is a crystal form of vildagliptin preferably the crystal form "A" or any of its salts.

7. A pharmaceutical tablet formulation according to any one of the preceding claims,
wherein relative to the weight of the formulation:
(a) vildagliptin, or the corresponding amount of any of its salts is present in an amount from 15% to 35% by weight;
(b) the hydroxypropyl methylcellulose is present in an amount from 30% to 50% by weight;
(c) the microcrystalline cellulose is present in an amount from 25% to 35% by weight; and
(d) the magnesium stearate is present in an amount from 0.1% to 3% by weight.

8. A pharmaceutical tablet formulation, comprising per 600 mg tablet the following ingredients:
(a) vildagliptin in an amount of 150 mg, or the corresponding amount of any of its salt;
(b) a hydroxypropyl methylcellulose in an amount of about 240 mg, wherein the hydroxypropyl methylcellulose has an apparent viscosity of 80,000 cP to 120,000 cP (nominal value 100,000 cP) when present in a 1% solution;
(c) a microcrystalline cellulose in an amount of 180 mg;
(d) a lactose in an amount of about 24 mg; and
(e) a magnesium stearate in an amount of 6 mg.

9. A pharmaceutical tablet formulation, comprising per 400 mg tablet the following ingredients:
(a) vildagliptin in an amount of 100 mg, or the corresponding amount of any of its salts;
(b) a hydroxypropyl methylcellulose in an amount of about 160 mg, wherein the hydroxypropyl methylcellulose has an apparent viscosity of 80,000 cP to 120,000 cP (nominal value 100,000 cP) when present in a 1% solution;
(c) a microcrystalline cellulose in an amount of 120 mg;
(d) a lactose in an amount of about 16 mg; and
(e) a magnesium stearate in an amount of 4 mg.

10. A method of producing a pharmaceutical tablet formulation according to any one of claims 1 to 9, where the method comprises combining the ingredients in the amounts recited in any one of these claims.

11. A pharmaceutical multilayer tablet wherein the pharmaceutical tablet formulation according to any of claims 1 to 9, represents one of the layers of the tablet.

12. A pharmaceutical multilayer tablet according to claim 11, wherein the further layer contains a glitazone (e.g. pioglitazone or rosiglitazon) or metformin.

13. A pharmaceutical multilayer tablet according to claim 12 wherein the further layer is an immediate release formulation.

14. A pharmaceutical tablet obtained by compression of a pharmaceutical tablet formulation according to any one of claims 1 to 13.

15. A pharmaceutical tablet obtained by compression of a pharmaceutical tablet formulation according to any of the claims 1 to 13, wherein the pharmaceutical tablet formulation is subject to roller compaction before compression into tablet.

16. A pharmaceutical tablet according to claim 15 comprising 100 mg of vildagliptin or a pharmaceutically acceptable salt thereof wherein the tablet hardness range is of between 10 to 13Kp.

17. A pharmaceutical tablet according to claim 15 comprising 150 mg of vildagliptin or a pharmaceutically acceptable salt thereof wherein the tablet hardness range is of between 11 to 25Kp.

18. A pharmaceutical tablet formulation according to any one of claims 1 to 9 or a pharmaceutical tablet according to any one of claims 11 to 17, comprising at least one further therapeutic agent.

19. A pharmaceutical tablet formulation or a pharmaceutical tablet according to claim 18, wherein the at least one further therapeutic agent is an antidiabetic.

20. A pharmaceutical tablet formulation or a pharmaceutical tablet according to claim 19, wherein the antidiabetic agent is selected from pioglitazone, rosiglitazone or metformin.

21. A pharmaceutical tablet formulation or a pharmaceutical tablet according to claim 18, wherein the at least one further therapeutic agent is an angiotensin II antagonist or a statin.

22. A process for preparing a tablet, in unit dosage form, the process comprising the steps of:
a) blending a pharmaceutical tablet formulation according to any one of the preceding claims;
(b) compressing the formulation prepared during step (a) to form the compressed tablet in unit dose form.

23. A process for preparing a tablet, in unit dosage form, the process comprising the steps of:
a) blending a pharmaceutical tablet formulation according to any one of claims 1 to 21;
(b) roller compacting the formulation prepared during step (a); and
(c) compressing the formulation prepared during step (b) to form the compressed tablet in unit dosage form.

24. A process for preparing a tablet, in unit dosage form, the process comprising the steps of:
(a) blending a pharmaceutical tablet formulation according to any one of claims 1 to 21;
(b) roller compacting the formulation prepared during step (a) with a compaction force comprised between 10 and 16 KN and;
(c) compressing the formulation prepared during step (b) to form the compressed tablet in unit dosage form.

25. A sustained release solid oral pharmaceutical dosage form according to any of the preceding claims which is:
i-1) a solid oral pharmaceutical dosage form comprising about 100 mg of vildagliptin free base, or a respective amount of a pharmaceutically acceptable salt thereof, and a carrier medium, said dosage form providing an arithmetic mean maximum plasma concentration of vildagliptin ranging from about 15.8 ng/mL +/- 6.85 ng/mL to about 173 ng/mL +/- 52 ng/mL between about 0.5 and about 16 hours following oral administration of said dosage form in a patient not treated with vildagliptin before said administration and wherein patient is under fasted conditions, and/or
i-2) a solid oral pharmaceutical dosage form comprising about 100 mg of vildagliptin free base, or a respective amount of a pharmaceutically acceptable salt thereof, and a carrier medium, said dosage form providing an arithmetic mean maximum plasma concentration of vildagliptin ranging from about 26.3 ng/mL +/- 13.1 ng/mL to about 175 ng/mL +/- 62.5 ng/mL between about 0.5 and about 16 hours following administration of said dosage form on day 9 in a patient treated with said dosage form once a day since day 1 and wherein said patient has been served an ADA breakfast within 30 minutes of the morning administration of said dosage form, and/or
i-3) a solid oral pharmaceutical dosage form comprising about 100 mg of vildagliptin free base, or a respective amount of a pharmaceutically acceptable salt thereof, and a carrier medium, said dosage form providing an arithmetic mean maximum plasma concentration of vildagliptin ranging from about 26.9 ng/mL +/- 14.1 ng/mL to about 186 ng/mL +/- 80.6 ng/mL between about 0.5 and about 16 hours following administration of said dosage form on day 10 in a patient treated with said dosage form once a day since day 1 and wherein said patient is under fasted conditions, and/or
ii-1) a solid oral dosage form comprising about 100 mg vildagliptin free base, or a respective amount of a pharmaceutically acceptable salt thereof, and a carrier medium, said dosage form providing an arithmetic mean AUC_{(0-inf)} of vildagliptin ranging from about 1073 to about 1825 ng·h/mL i.e. 1449 ng·h/mL +/- 376 ng·h/mL following oral administration of said dosage form , in a patient not treated with vildagliptin before said administration and wherein said patient is under fasted conditions, and/or
ii-2) a solid oral dosage form comprising about 100 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1001 to about 1977 ng·h/mL i.e. 1489 ng·h/mL +/- 488 ng·h/mL following oral administration of said dosage form , on day 9 in a patient treated with said dosage form once a day since day 1 and wherein said patient has been served an ADA breakfast within 30 minutes of the morning administration of said dosage form, and/or
ii-3) a solid oral dosage form comprising about 100 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1103 to about 2173 ng·h/mL i.e. 1638 ng·h/mL +/- 535 ng·h/mL following oral administration of said dosage form, on day 10 in a patient treated with said dosage form once a day since day 1 and wherein said patient is under fasted conditions, and/or
iii-1) a solid oral dosage form comprising about 100 mg vildagliptin free base, or a respective amount of a pharmaceutically acceptable salt thereof, and a carrier medium, said dosage form providing an arithmetic mean tₘₐₓ of vildagliptin of 3.61 hr +/- 1.44 hr following oral administration of said dosage form , in a patient not treated with vildagliptin before said administration and wherein said patient is under fasted conditions, and/or
iii-2) a solid oral dosage form comprising about 100 mg vildagliptin free base, or a respective amount of a pharmaceutically acceptable salt thereof, and a carrier medium, said dosage form providing an arithmetic mean tₘₐₓ of vildagliptin of 2.59 hr +/- 1.4 hr following oral administration of said dosage form , on day 9 in a patient treated with said dosage form once a day since day 1 and wherein said patient has been served an ADA breakfast within 30 minutes of the morning administration of said dosage form, and/or
iii-3) a solid oral dosage form comprising about 100 mg vildagliptin free base, or a respective amount of a pharmaceutically acceptable salt thereof, and a carrier medium, said dosage form providing an arithmetic mean tₘₐₓ of vildagliptin of 3.74 hr +/- 1.44 hr following oral administration of said dosage form , on day 10 in a patient treated with said dosage form once a day since day 1 and wherein said patient is under fasted conditions, and/or
iv-1) a solid oral dosage form comprising about 100 mg vildagliptin free base, or a respective amount of a pharmaceutically acceptable salt thereof, and a carrier medium, said dosage form providing an arithmetic mean Cₘₐₓ of vildagliptin of 205 ng/ml +/- 47 ng/ml following oral administration of said dosage form , in a patient not treated with vildagliptin before said administration and wherein said patient is under fasted conditions, and/or
iv-2) a solid oral dosage form comprising about 100 mg vildagliptin free base, or a respective amount of a pharmaceutically acceptable salt thereof, and a carrier medium, said dosage form providing an arithmetic mean Cₘₐₓ of vildagliptin of 200 ng/ml +/- 64 ng/ral following oral administration of said dosage form , on day 9 in a patient treated with said dosage form once a day since day 1 and wherein said patient has been served an ADA breakfast within 30 minutes of the morning administration of said dosage form, and/or
iv-3) a solid oral dosage form comprising about 100 mg vildagliptin free base, or a respective amount of a pharmaceutically acceptable salt thereof, and a carrier medium, said dosage form providing an arithmetic mean Cₘₐₓ of vildagliptin of 245 ng/ml +/- 68 ng/ml following oral administration of said dosage form , on day 10 in a patient treated with said dosage form once a day since day 1 and wherein said patient is under fasted conditions, and/or
v-1) a solid oral dosage form comprising about 100 mg vildagliptin free base, or a respective amount of a pharmaceutically acceptable salt thereof, and a carrier medium, said dosage form providing an arithmetic mean % inhibition of DPP-IV activity over 24 hours of 85.64% +/- 12.76% following oral administration of said dosage form , in a patient not treated with vildagliptin before said administration and wherein said patient is under fasted conditions, and/or
v-2) a solid oral dosage form comprising about 100 mg vildagliptin free base, or a respective amount of a pharmaceutically acceptable salt thereof, and a carrier medium, said dosage form providing an arithmetic mean % inhibition of DPP-IV activity over 24 hours of 87.78% +/- 16.37% following oral administration of said dosage form , on day 9 in a patient treated with said dosage form once a day since day 1 and wherein said patient has been served an ADA breakfast within 30 minutes of the morning administration of said dosage form, and/or
v-3) a solid oral dosage form comprising about 100 mg vildagliptin free base, or a respective amount of a pharmaceutically acceptable salt thereof, and a carrier medium, said dosage form providing an arithmetic mean % inhibition of DPP-IV activity over 24 hours of 90.20% +/- 7.35% following oral administration of said dosage form , on day 10 in a patient treated with said dosage form once a day since day 1 and wherein said patient is under fasted conditions, and/or
vi-1) a solid oral dosage form comprising about 100 mg vildagliptin free base, or a respective amount of a pharmaceutically acceptable salt thereof, and a carrier medium, said dosage form providing a pharmacokinetic profile as substantially depicted in figure 24, following oral administration of said dosage form, in a patient not treated with vildagliptin before said administration and wherein said patient is under fasted conditions, and/or
vi-2) a solid oral dosage form comprising about 100 mg vildagliptin free base, or a respective amount of a pharmaceutically acceptable salt thereof, and a carrier medium, said dosage form providing a pharmacokinetic profile as substantially depicted in figure 25, following oral administration of said dosage form, on day 9 in a patient treated with said dosage form once a day since day 1 and wherein said patient has been served an ADA breakfast within 30 minutes of the morning administration of said dosage form, and/or
vi-3) a solid oral dosage form comprising about 100 mg vildagliptin free base, or a respective amount of a pharmaceutically acceptable salt thereof, and a carrier medium, said dosage form providing a pharmacokinetic profile as substantially depicted in figure 26, following oral administration of said dosage form, on day 10 in a patient treated with said dosage form once a day since day 1 and wherein said patient is under fasted conditions.

26. A sustained release solid oral pharmaceutical dosage form according to any of the preceding claims which is:
i-a) a solid oral pharmaceutical dosage form comprising about 150 mg of vildagliptin free base, or a respective amount of a pharmaceutically acceptable salt thereof, and a carrier medium, said dosage form providing an arithmetic mean maximum plasma concentration of vildagliptin ranging from about 30.7 ng/mL +/- 21.9 ng/mL to about 223 ng/mL +/- 77.3 ng/mL between about 0.5 and about 16 hours following oral administration of said dosage form in a patient not treated with vildagliptin before said administration and wherein patient is under fasted conditions, and/or
i-b) a solid oral pharmaceutical dosage form comprising about 150 mg of vildagliptin free base, or a respective amount of a pharmaceutically acceptable salt thereof, and a carrier medium, said dosage form providing an arithmetic mean maximum plasma concentration of vildagliptin ranging from about 48.7 ng/mL +/- 25.8 ng/mL to about 223 ng/mL +/- 99.7 ng/mL between about 0.5 and about 16 hours following administration of said dosage form on day 9 in a patient treated with said dosage form once a day since day 1 and wherein said patient has been served an ADA breakfast within 30 minutes of the morning administration of said dosage form, and/or
i-c) a solid oral pharmaceutical dosage form comprising about 150 mg of vildagliptin free base, or a respective amount of a pharmaceutically acceptable salt thereof, and a carrier medium, said dosage form providing an arithmetic mean maximum plasma concentration of vildagliptin ranging from about 44.6 ng/mL +/- 28.5 ng/mL to about 263 ng/mL +/- 84.4 ng/mL between about 0.5 and about 16 hours following administration of said dosage form on day 10 in a patient treated with said dosage form once a day since day 1 and wherein said patient is under fasted conditions, and/or
ii-a) a solid oral dosage form comprising about 150 mg vildagliptin free base, or a respective amount of a pharmaceutically acceptable salt thereof, and a carrier medium, said dosage form providing an arithmetic mean AUC_{(0-inf)} of vildagliptin ranging from about 1346 to about 3196 ng·h/mL i.e. 2271 ng·h/mL +/- 925 ng·h/mL following oral administration of said dosage form , in a patient not treated with vildagliptin before said administration and wherein said patient is under fasted conditions, and/or
ii-b) a solid oral dosage form comprising about 150 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1277 to about 3207 ng·h/mL i.e. 2242 ng·h/mL +/- 965 ng·h/mL following oral administration of said dosage form , on day 9 in a patient treated with said dosage form once a day since day 1 and wherein said patient has been served an ADA breakfast within 30 minutes of the morning administration of said dosage form, and/or
ii-c) a solid oral dosage form comprising about 150 mg vildagliptin free base, or a respective amount of a pharmaceutically acceptable salt thereof, and a carrier medium, said dosage form providing an arithmetic mean AUC₍₀₋₂₄₎ of vildagliptin ranging from about 1643 to about 3273 ng·h/mL i.e. 2458 ng·h/mL +/- 815 ng·h/mL following oral administration of said dosage form, on day 10 in a patient treated with said dosage form once a day since day 1 and wherein said patient is under fasted conditions, and/or
iii-a) a solid oral dosage form comprising about 150 mg vildagliptin free base, or a respective amount of a pharmaceutically acceptable salt thereof, and a carrier medium, said dosage form providing an arithmetic mean tₘₐₓ of vildagliptin of 3.57 hr +/- 1.17 hr following oral administration of said dosage form , in a patient not treated with vildagliptin before said administration and wherein said patient is under fasted conditions, and/or
iii-b) a solid oral dosage form comprising about 150 mg vildagliptin free base, or a respective amount of a pharmaceutically acceptable salt thereof, and a carrier medium, said dosage form providing an arithmetic mean tₘₐₓ of vildagliptin of 2.87 hr +/- 1.59 hr following oral administration of said dosage form , on day 9 in a patient treated with said dosage form once a day since day 1 and wherein said patient has been served an ADA breakfast within 30 minutes of the morning administration of said dosage form, and/or
iii-c) a solid oral dosage form comprising about 150- mg vildagliptin free base, or a respective amount of a pharmaceutically acceptable salt thereof, and a carrier medium, said dosage form providing an arithmetic mean tₘₐₓ of vildagliptin of 4.13 hr +/- 1.24 hr following oral administration of said dosage form , on day 10 in a patient treated with said dosage form once a day since day 1 and wherein said patient is under fasted conditions, and/or
iv-a) a solid oral dosage form comprising about 150 mg vildagliptin free base, or a respective amount of a pharmaceutically acceptable salt thereof, and a carrier medium, said dosage form providing an arithmetic mean Cₘₐₓ of vildagliptin of 257 ng/ml +/- 59 ng/ml following oral administration of said dosage form , in a patient not treated with vildagliptin before said administration and wherein said patient is under fasted conditions, and/or
iv-b) a solid oral dosage form comprising about 150 mg vildagliptin free base, or a respective amount of a pharmaceutically acceptable salt thereof, and a carrier medium, said dosage form providing an arithmetic mean Cₘₐₓ of vildagliptin of 272 ng/ml +/- 111 ng/ml following oral administration of said dosage form , on day 9 in a patient treated with said dosage form once a day since day 1 and wherein said patient has been served an ADA breakfast within 30 minutes of the morning administration of said dosage form, and/or
iv-c) a solid oral dosage form comprising about 150 mg vildagliptin free base, or a respective amount of a pharmaceutically acceptable salt thereof, and a carrier medium, said dosage form providing an arithmetic mean Cₘₐₓ of vildagliptin of 308 ng/ml +/- 91 ng/ml following oral administration of said dosage form , on day 10 in a patient treated with said dosage form once a day since day 1 and wherein said patient is under fasted conditions, and/or
v-a) a solid oral dosage form comprising about 150 mg vildagliptin free base, or a respective amount of a pharmaceutically acceptable salt thereof, and a carrier medium, said dosage form providing an arithmetic mean % inhibition of DPP-IV activity over 24 hours of 90.04% +/- 11.91 % following oral administration of said dosage form , in a patient not treated with vildagliptin before said administration and wherein said patient is under fasted conditions, and/or
v-b) a solid oral dosage form comprising about 150 mg vildagliptin free base, or a respective amount of a pharmaceutically acceptable salt thereof, and a carrier medium, said dosage form providing an arithmetic mean % inhibition of DPP-IV activity over 24 hours of 90.4% +/- 17.50% following oral administration of said dosage form , on day 9 in a patient treated with said dosage form once a day since day 1 and wherein said patient has been served an ADA breakfast within 30 minutes of the morning administration of said dosage form, and/or
v-c) a solid oral dosage form comprising about 150 mg vildagliptin free base, or a respective amount of a pharmaceutically acceptable salt thereof, and a carrier medium, said dosage form providing an arithmetic mean % inhibition of DPP-IV activity over 24 hours of 91.64% +/- 8.47% following oral administration of said dosage form, on day 10 in a patient treated with said dosage form once a day since day 1 and wherein said patient is under fasted conditions, and/or
vi-a) a solid oral dosage form comprising about 150 mg vildagliptin free base, or a respective amount of a pharmaceutically acceptable salt thereof, and a carrier medium, said dosage form providing a pharmacokinetic profile as substantially depicted in figure 24, following oral administration of said dosage form, in a patient not treated with vildagliptin before said administration and wherein said patient is under fasted conditions, and/or
vi-b) a solid oral dosage form comprising about 150 mg vildagliptin free base, or a respective amount of a pharmaceutically acceptable salt thereof, and a carrier medium, said dosage form providing a pharmacokinetic profile as substantially depicted in figure 25, following oral administration of said dosage forum, on day 9 in a patient treated with said dosage form once a day since day 1 and wherein said patient has been served an ADA breakfast within 30 minutes of the morning administration of said dosage form, and/or
vi-c) a solid oral dosage form comprising about 150 mg vildagliptin free base, or a respective amount of a pharmaceutically acceptable salt thereof, and a carrier medium, said dosage form providing a pharmacokinetic profile as substantially depicted in figure 26, following oral administration of said dosage form, on day 10 in a patient treated with said dosage form once a day since day 1 and wherein said patient is under fasted conditions.

27. A sustained release solid oral pharmaceutical dosage form according to any one of claims 25 or 26, which comprises a pharmaceutical tablet formulation according to any one of claims 1 to 25.

28. A sustained release solid oral pharmaceutical dosage form according to any one of claims 25 or 26, which comprises a hydroxypropyl methylcellulose, preferably between 20% and 60%, between 30% and 50% by weight on a dry weight basis of a hydroxypropyl methylcellulose.

29. A sustained release solid oral pharmaceutical dosage form according to any one of claims 25 or 26, which comprises a hydroxypropyl methylcellulose with an apparent viscosity of 80,000 cP to 120,000 cP (nominal value 100,000 cP) when present in a 1% solution, preferably between 20% and 60%, or between 30% and 50% by weight on a dry weight basis of a hydroxypropyl methylcellulose with an apparent viscosity of 80,000 cP to 120,000 cP (nominal value 100,000 cP) when present in a 1% solution.

30. A sustained release solid oral pharmaceutical dosage form according to any one of claims 25 or 26, wherein:
i) the formulation is a matrix formulation containing a pharmaceutically acceptable hydrophilic polymer which can retard diffusion of vildagliptin,
ii) the solid oral pharmaceutical dosage form is a compressed tablet, and optionally
iii) the elution rate of vildagliptin at 30 minutes after starting the test is less than 30% when conducting the Paddle method.

31. Use of the ingredients in the amounts recited in any one of the claims 1 to 30, in the manufacture of a pharmaceutical table formulation, for inhibiting dipeptidyl peptidase IV activity in a subject.

32. Use of the ingredients in the amounts recited in any one of the claims 1 to 30, in the manufacture of a pharmaceutical table formulation, for treating a condition alleviated by dipeptidyl peptidase IV inhibition in a subject suffering from the condition.

33. Use according to claim 32, wherein the condition is non-insulin-dependent diabetes mellitus.

34. Use according to claim 32, wherein the condition is obesity, arthritis or osteoporosis.

35. Use according to any one of claims 31 to 34, wherein the pharmaceutical tablet formulation is used in combination with a therapeutically effective dose of an anti-diabetic, arthritis drug or anti-obesity drug.

## Patentansprüche

1. Pharmazeutische Tablettenformulierung, umfassend pro Einheitsdosierungsform beispielsweise pro Tablette die folgenden Bestandteile:
(a) eine Verbindung als wirksamen Bestandteil, wobei die Verbindung (S)-1-[(3-Hydroxy-1-adamantyl)amino]acetyl-2-cyano-pyrrolidin (Vildagliptin) oder die entsprechende Menge eines beliebigen Salzes hiervon ist, und
(b) eine Hydroxypropylmethylcellulose mit einer Scheinviskosität von 80 000 cP bis 120 000 cP (Nominalwert 100 000 cP) bei Gegenwart in einer 1%-igen Lösung,
und optional einen Füllstoff und/oder ein Schmiermittel.

2. Pharmazeutische Tablettenformulierung nach Anspruch 1, wobei in der Einheitsdosierungsform das Verhältnis des Gewichts von Vildagliptin und eines beliebigen entsprechenden Salzes hiervon zu dem Gewicht der Hydroxypropylmethylcellulose 0,76 bis 2,5, vorzugsweise 0,3 bis 1,16 oder 0,4 bis 1 beträgt.

3. Pharmazeutische Tablettenformulierung nach einem der Ansprüche 1 oder 2, umfassend:
(a) 15-55 Gew.-%, vorzugsweise 25-45 Gew.-% auf Trockengewichtsbasis eines pharmazeutisch akzeptablen Füllstoffs und optional
(b) 0,1-10 Gew.-%, vorzugsweise 0,1-3 Gew.-% auf Trockengewichtsbasis eines pharmazeutisch akzeptablen Schmiermittels.

4. Pharmazeutische Tablettenformulierung nach einem der Ansprüche 1 bis 3, wobei der Füllstoff Lactose und/oder mikrokristalline Cellulose ist und/oder das Schmiermittel Magnesiumstearat ist.

5. Pharmazeutische Tablettenformulierung nach Anspruch 4, wobei die Lactose in einer Menge von 1-8 Gew.-% vorhanden ist.

6. Pharmazeutische Tablettenformulierung nach einem der vorhergehenden Ansprüche, wobei die Verbindung in kristalliner Form von Vildagliptin, vorzugsweise in der Kristallform "A", oder von einem beliebigen der Salze hiervon vorliegt.

7. Pharmazeutische Tablettenformulierung nach einem der vorhergehenden Ansprüche, wobei bezogen auf das Gewicht der Formulierung:
(a) Vildagliptin oder die entsprechende Menge eines beliebigen Salzes hiervon in einer Menge von 15-35 Gew.-% vorhanden ist,
(b) die Hydroxypropylmethylcellulose in einer Menge von 30 bis 50 Gew.-% vorhanden ist,
(c) die mikrokristalline Cellulose in einer Menge von 25 bis 35 Gew.-% vorhanden ist und
(d) das Magnesiumstearat in einer Menge von 0,1 bis 3 Gew.-% vorhanden ist.

8. Pharmazeutische Tablettenformulierung, umfassend pro 600 mg Tablette die folgenden Bestandteile:
(a) Vildagliptin in einer Menge von 150 mg oder die entsprechende Menge eines beliebigen Salzes hiervon;
(b) eine Hydroxypropylmethylcellulose in einer Menge von etwa 240 mg, wobei die Hydroxypropylmethylcellulose eine Scheinviskosität von 80 000 cP bis 120 000 cP (Nominalwert 100 000 cP) bei Anwesenheit in einer 1%-igen Lösung aufweist;
(c) eine mikrokristalline Cellulose in einer Menge von 180 mg;
(d) eine Lactose in einer Menge von etwa 24 mg und
(e) ein Magnesiumstearat in einer Menge von 6 mg.

9. Pharmazeutische Tablettenformulierung, umfassend pro 400 mg Tablette die folgenden Bestandteile:
(a) Vildagliptin in einer Menge von 100 mg oder die entsprechende Menge eines beliebigen Salzes hiervon;
(b) eine Hydroxypropylmethylcellulose in einer Menge von etwa 160 mg, wobei die Hydroxypropylmethylcellulose eine Scheinviskosität von 80 000 cP bis 120 000 cP (Nominalwert 100 000 cP) bei Anwesenheit in einer 1%-igen Lösung aufweist;
(c) eine mikrokristalline Cellulose in einer Menge von 120 mg;
(d) eine Lactose in einer Menge von etwa 16 mg und
(e) ein Magnesiumstearat in einer Menge von 4 mg.

10. Verfahren zur Herstellung einer pharmazeutischen Tablettenformulierung nach einem der Ansprüche 1 bis 9, wobei das Verfahren ein Kombinieren der Bestandteile in den in einem beliebigen dieser Ansprüche angegebenen Mengen umfasst.

11. Pharmazeutische Mehrschichtentablette, wobei die pharmazeutische Tablettenformulierung gemäß einem der Ansprüche 1 bis 9 eine der Schichten der Tablette darstellt.

12. Pharmazeutische Mehrschichtentablette nach Anspruch 11, wobei die weitere Schicht ein Glitazon (beispielsweise Pioglitazon oder Rosiglitazon) oder Metformin enthält.

13. Pharmazeutische Mehrschichtentablette nach Anspruch 12, wobei die weitere Schicht eine für eine sofortige Freisetzung sorgende Formulierung ist.

14. Pharmazeutische Tablette, erhalten durch Verpressen einer pharmazeutischen Tablettenformulierung nach einem der Ansprüche 1 bis 13.

15. Pharmazeutische Tablette, erhalten durch Verpressen einer pharmazeutischen Tablettenformulierung nach einem der Ansprüche 1 bis 13, wobei die pharmazeutische Tablettenformulierung vor Verpressen zu einer Tablette einem Walzenverdichten unterzogen worden ist.

16. Pharmazeutische Tablette nach Anspruch 15, umfassend 100 mg Vildagliptin oder ein pharmazeutisch akzeptables Salz hiervon, wobei die Tablettenhärte in einem Bereich von zwischen 10 und 13 kp liegt.

17. Pharmazeutische Tablette nach Anspruch 15, umfassend 150 mg Vildagliptin oder ein pharmazeutisch akzeptables Salz hiervon, wobei die Tablettenhärte in einem Bereich von zwischen 11 und 25 kp liegt.

18. Pharmazeutische Tablettenformulierung nach einem der Ansprüche 1 bis 9 oder pharmazeutische Tablette nach einem der Ansprüche 11 bis 17, umfassend mindestens ein weiteres therapeutisches Mittel.

19. Pharmazeutische Tablettenformulierung oder pharmazeutische Tablette nach Anspruch 18, wobei das mindestens eine weitere therapeutische Mittel ein Antidiabetikum ist.

20. Pharmazeutische Tablettenformulierung oder pharmazeutische Tablette nach Anspruch 19, wobei das antidiabetische Mittel aus Pioglitazon, Rosiglitazon oder Metformin ausgewählt ist.

21. Pharmazeutische Tablettenformulierung oder pharmazeutische Tablette nach Anspruch 18, wobei das mindestens eine weitere therapeutische Mittel ein Angiotensin-II-Antagonist oder ein Statin ist.

22. Verfahren zur Herstellung einer Tablette in Einheitsdosierungsform, wobei das Verfahren die folgenden Stufen umfasst:
(a) Vermischen einer pharmazeutischen Tablettenformulierung nach einem der vorhergehenden Ansprüche;
(b) Verpressen der in Stufe (a) hergestellten Formulierung unter Bildung der verpressten Tablette in Einheitsdosierungsform.

23. Verfahren zur Herstellung einer Tablette in Einheitsdosierungsform, wobei das Verfahren die folgenden Stufen umfasst:
(a) Vermischen einer pharmazeutischen Tablettenformulierung nach einem der Ansprüche 1 bis 21;
(b) Walzenverdichten der in Stufe (a) hergestellten Formulierung und
(c) Verpressen der in Stufe (b) hergestellen Formulierung unter Bildung der verpressten Tablette in Einheitsdosierungsform.

24. Verfahren zur Herstellung einer Tablette in Einheitsdosierungsform, wobei das Verfahren die folgendes Stufen umfasst:
(a) Vermischen einer pharmazeutischen Tablettenformulierung nach einem der Ansprüche 1 bis 21;
(b) Walzenverdichten der in Stufe (a) hergestellten Formulierung mit einem Verdichtungsdruck von zwischen 10 und 16 kN und
(c) Verpressen der in Stufe (b) hergestellten Formulierung unter Bildung der verpressten Tablette in Einheitsdosierungsform.

25. Für eine verzögerte Freisetzung sorgende feste orale pharmazeutische Dosierungsform nach einem der vorhergehenden Ansprüche, nämlich:
i-1) eine feste orale pharmazeutische Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere maximale Plasmakonzentration von Vildagliptin im Bereich von etwa 15,8 ng/ml ± 6,75 ng/ml bis etwa 173 mg/ml ± 52 ng/ml zwischen etwa 0,5 und etwa 16 h nach einer oralen Verabreichung der Dosierungsform bei einem mit Vildagliptin vor der Verabreichung nicht behandelten Patienten, wobei sich der Patient unter Fastenbedingungen befindet, liefert und/oder
i-2) eine feste orale pharmazeutische Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere maximale Plasmakonzentration von Vildagliptin im Bereich von etwa 26,3 ng/ml ± 13,1 ng/ml bis etwa 175 mg/ml ± 62,5 ng/ml zwischen etwa 0,5 und etwa 16 h nach Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
i-3) eine feste orale pharmazeutische Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere maximale Plasmakonzentration von Vildagliptin im Bereich von etwa 26,9 ng/ml ± 14,1 ng/ml bis etwa 186 mg/ml ± 80,6 ng/ml zwischen etwa 0,5 und etwa 16 h nach einer Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
ii-1) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere AUC_{(0-inf)} an Vildagliptin im Bereich von etwa 1073 bis etwa 1825 ng · h/ml, d.h. 1449 ng· h/ml ± 376 ng · h/ml nach einer oralen Verabreichung der Dosierungsform bei einem mit Vlidagliptin vor der Verabreichung nicht behandelten Patienten, wobei sich der Patient unter Fastentredingungen befindet, liefert und/oder
ii-2) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere AUC₍₀₋₂₄₎ an Vildagliptin im Bereich von etwa 1001 bis etwa 1977 ng · h/ml, d.h. 1489 ng · h/ml ± 488 ng · h/ml nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
ii-3) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere AUC₍₀₋₂₄₎ an Vildagliptin im Bereich von etwa 1103 bis etwa 2173 ng · h/ml, d.h. 1638 ng · h/ml ± 535 ng · h/ml nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
iii-1) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform einen arithmetischen mittleren tₘₐₓ-Wert an Vildagliptin von 3,61 h ± 1,44 h nach oraler Verabreichung der Dosierungsform bei einem mit Vildagliptin vor der Verabreichung nicht behandelten Patienten, wobei sich der Patient unter Fastenbedingungen befindet, liefert und/oder
iii-2) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform einen arithmetischen mittleren tₘₐₓ-Wert an Vildagliptin von 2,59 h ± 1,4 h nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
iii-3) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform einen arithmetischen mittleren tₘₐₓ-Wert an Vildagliptin von 3,74 h ± 1,44 h nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
iv-1) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform einen arithmetischen mittleren Cₘₐₓ-Wert an Vildagliptin von 205 ng/ml ± 47 ng/ml nach oraler Verabreichung der Dosierungsform, bei einem mit Vildagliptin vor der Verabreichung nicht behandelten Patienten, wobei sich der Patient unter Fastenbedingungen befindet, liefert und/oder
iv-2) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform einen arithmetischen mittleren Cₘₐₓ-Wert an Vildagliptin von 200 ng/ml ± 64 ng/ml nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
iv-3) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform einen arithmetischen mittleren Cₘₐₓ-Wert an Vildagliptin von 245 ng/ml ± 68 ng/ml nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
v-1) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere prozentuale Hemmung der DPP-IV-Aktivität über 24 h von 85,64 % ± 12,76 % nach oraler Verabreichung der Dosierungsform bei einem mit Vildagliptin vor der Verabreichung nicht behandelten Patienten, wobei sich der Patient unter Fastenbedingungen befindet, liefert und/oder
v-2) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere prozentuale Hemmung der DPP-IV-Aktivität über 24 h von 87,78 % ± 16,37 % nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
v-3) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform eine arithmetische mittlere prozentuale Hemmung der DPP-IV-Aktivität über 24 h von 90,20 % ± 7,35 % nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
vi-1) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform ein im wesentlichen in Figur 24 dargestelltes pharmakokinetisches Profil nach einer oralen Verabreichung der Dosierungsform bei einem mit Vildagliptin vor der Verabreichung nicht behandelten Patienten, wobei sich der Patient unter Fastenbedingungen befindet, liefert und/oder
vi-2) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform ein im wesentlichen in Figur 25 dargestelltes pharmakokinetisches Profil nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
vi-3) eine feste orale Dosierungsform, umfassend etwa 100 mg Vildagliptin in Form der freien Base oder eine respektive Menge eines pharmazeutisch akzeptablen Salzes hiervon und ein Trägermedium, wobei die Dosierungsform ein im wesentlichen in Figur 26 dargestelltes pharmakokinetisches Profil nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert.

26. Für eine verzögerte Freisetzung sorgende feste orale pharmazeutische Dosierungsform nach einem der vorhergehenden Ansprüche, nämlich:
i-a) eine feste orale pharmazeutische Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere maximale Plasmakonzentration von Vildagliptin im Bereich von etwa 30,7 ng/ml ± 21,9 ng/ml bis etwa 223 mg/ml ± 77,3 ng/ml zwischen etwa 0,5 und etwa 16 h nach einer oralen Verabreichung der Dosierungsform bei einem mit Vildagliptin vor der Verabreichung nicht behandelten Patienten, wobei sich der Patient unter Fastenbedingungen befindet, liefert und/oder
i-b) eine feste orale pharmazeutische Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere maximale Plasmakonzentration von Vildagliptin im Bereich von etwa 48,7 ng/ml ± 25,8 ng/ml bis etwa 223 mg/ml ± 99,7 ng/ml zwischen etwa 0,5 und etwa 16 h nach Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
i-c) eine feste orale pharmazeutische Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere maximale Plasmakonzentration von Vildagliptin im Bereich von etwa 44,6 ng/ml ± 28,5 ng/ml bis etwa 263 mg/ml ± 84,4 ng/ml zwischen etwa 0,5 und etwa 16 h nach einer Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
ii-a) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere AUC_{(0-inf)} an Vildagliptin im Bereich von etwa 1346 bis etwa 3196 ng · h/ml, d.h. 2271 ng · h/ml ± 925 ng · h/ml nach einer oralen Verabreichung der Dosierungsform bei einem mit Vildagliptin vor der Verabreichung nicht behandelten Patienten, wobei sich der Patient unter Fastenbedingungen befindet, liefert und/oder
ii-b) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere AUC₍₀₋₂₄ an Vildagliptin im Bereich von etwa 1277 bis etwa 3207 ng · h/ml, d.h. 2242 ng · h/ml ± 965 ng · h/ml nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
ii-c) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere AUC₍₀₋₂₄₎ an Vildagliptin im Bereich von etwa 1643 bis etwa 3273 ng · h/ml, d.h. 2458 ng · h/ml ± 815 ng · h/ml nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
iii-a) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform einen arithmetischen mittleren tₘₐₓ-Wert an Vildagliptin von 3,57 h ± 1,17 h nach oraler Verabreichung der Dosierungsform bei einem mit Vildagliptin vor der Verabreichung nicht behandelten Patienten, wobei sich der Patient unter Fastenbedingungen befindet, liefert und/oder
iii-b) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform einen arithmetischen mittleren tₘₐₓ-Wert an Vildagliptin von 2,87 h ± 1,59 h nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
iii-c) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform einen arithmetischen mittleren tₘₐₓ-Wert an Vildagliptin von 4,13 h ± 1,24 h nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
iv-a) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform einen arithmetischen mittleren Cₘₐₓ-Wert an Vildagliptin von 257 ng/ml ± 59 ng/ml nach oraler Verabreichung der Dosierungsform bei einem mit Vildagliptin vor der Verabreichung nicht behandelten Patienten, wobei sich der Patient unter Fastenbedingungen befindet, liefert und/oder
iv-b) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform einen arithmetischen mittleren Cₘₐₓ-Wert an Vildagliptin von 272 ng/ml ± 111 ng/ml nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
iv-c) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform einen arithmetischen mittleren Cₘₐₓ-Wert an Vildagliptin von 308 ng/ml ± 91 ng/ml nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
v-a) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere prozentuale Hemmung der DPP-IV-Aktivität über 24 h von 90,04 % ± 11,91 % nach oraler Verabreichung der Dosierungsform bei einem mit Vildagliptin vor der Verabreichung nicht behandelten Patienten, wobei sich der Patient unter Fastenbedingungen befindet, liefert und/oder
v-b) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere prozentuale Hemmung der DPP-IV-Aktivität über 24 in von 90,4 % ± 17,50 % nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
v-c) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform eine arithmetische mittlere prozentuale Hemmung der DPP-IV-Aktivität über 24 h von 91,64 % ± 8,47 % nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert und/oder
vi-a) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform ein im wesentlichen in Figur 24 dargestelltes pharmakokinetisches Profil nach einer oralen Verabreichung der Dosierungsform bei einem mit Vildagliptin vor der Verabreichung nicht behandelten Patienten, wobei sich der Patient unter Fastenbedingungen befindet, liefert und/oder
vi-b) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform ein im wesentlichen in Figur 25 dargestelltes pharmakokinetisches Profil nach einer oralen Verabreichung der Dosierungsform am 9. Tag bei einem mit der Dosierungsform einmal täglich seit dem 1. Tag behandelten Patienten, wobei dem Patienten ein ADA-Frühstück innerhalb von 30 min nach der morgendlichen Verabreichung der Dosierungsform serviert wurde, liefert und/oder
vi-c) eine feste orale Dosierungsform, umfassend etwa 150 mg Vildagliptin in Form der freien Base oder eine respektive Menge eines pharmazeutisch akzeptablen Salzes hiervon und ein Trägermedium, wobei die Dosierungsform ein im wesentlichen in Figur 26 dargestelltes pharmakokinetisches Profil nach einer oralen Verabreichung der Dosierungsform am 10. Tag bei einem mit der Dosierungsform einmal täglich seit dem 1. Tag behandelten Patienten, wobei sich der Patient unter Fastenbedingungen befindet, liefert.

27. Für eine verzögerte Freisetzung sorgende feste orale pharmazeutische Dosierungsform nach einem der Ansprüche 25 oder 26, die eine pharmazeutische Tablettenformulierung nach einem der Ansprüche 1 bis 25 umfasst.

28. Für eine verzögerte Freisetzung sorgende feste orale pharmazeutische Dosierungsform nach einem der Ansprüche 25 oder 26, die eine Hydroxypropylmethylcellulose, vorzugsweise eine Menge zwischen 20 und 60 Gew.-%, zwischen 30 und 50 Gew.-% auf Trockengewichtsbasis einer Hydroxypropylmethylcellulose umfasst.

29. Für eine verzögerte Freisetzung sorgende feste orale pharmazeutische Dosierungsform nach einem der Ansprüche 25 oder 26, die eine Hydroxypropylmethylcellulose mit einer Scheinviskosität zwischen 80 000 cP und 120 000 cP (Nominalwert 100 000 cP) bei Gegenwart in einer 1%-igen Lösung, vorzugsweise eine Menge zwischen 20 und 60 Gew.-% oder zwischen 30 und 50 Gew.-% auf Trockengewichtsbasis einer Hydroxypropylmethylcellulose mit einer Scheinviskosität zwischen 80 000 cP und 120 000 cP (Nominalwert 100 000 cP) bei Gegenwart in einer 1%-igen Lösung umfasst.

30. Für eine verzögerte Freisetzung sorgende feste orale pharmazeutische Dosierungsform nach einem der Ansprüche 25 oder 26, worin:
i) die Formulierung eine Matrixformulierung ist, die ein pharmazeutisch akzeptables hydrophiles Polymer enthält, das die Diffusion von Vildagliptin verzögern kann,
ii) die feste orale pharmazeutische Dosierungsform eine Presstablette ist und optional
iii) die Eluierrate von Vildagliptin 30 min nach Beginn des Tests weniger als 30 % bei Durchführung des Paddle-Verfahrens beträgt.

31. Verwendung der Bestandteile in den in einem der Ansprüche 1 bis 30 angegebenen Mengen bei der Herstellung einer pharmazeutischen Tablettenformulierung zur Hemmung einer Dipeptidylpeptidase-IV-Aktivität bei einem Patienten.

32. Verwendung der Bestandteile in den in einem der Ansprüche 1 bis 30 angegebenen Mengen bei der Herstellung einer pharmazeutischen Tablettenformulierung zur Behandlung eines durch Dipeptidylpeptidase-IV-Hemmung gelinderten Zustands bei einem an diesem Zustand leidenden Patienten.

33. Verwendung nach Anspruch 32, wobei der Zustand ein nichtinsulinabhängiger Diabetes mellitus ist.

34. Verwendung nach Anspruch 32, wobei der Zustand Fettleibigkeit, Arthritis oder Osteoporose ist.

35. Verwendung nach einem der Ansprüche 31 bis 34, wobei die pharmazeutische Tablettenformulierung in Kombination mit einer therapeutisch wirksamen Menge eines Antidiabetikums, eines Arthritiswirkstoffs oder eines gegen Fettleibigkeit gerichteten Wirkstoffs verwendet wird.

## Revendications

1. Formulation pharmaceutique pour comprimés comprenant, par forme galénique unitaire, par exemple par comprimé, les ingrédients suivants :
(a) un composé en tant qu'ingrédient actif, dans laquelle le composé est la (S)-1-[(3-hydroxy-adamantyl)amino] acétyl-2-cyano-pyrrolidine (vildagliptine) ou la quantité correspondante de l'un quelconque de ses sels ; et
(b) une hydroxypropylméthylcellulose présentant une viscosité apparente de 80 000 cP à 120 000 cP (valeur nominale de 100 000 cP) lorsqu'elle est présente dans une solution à 1 % ;
et en option une charge et/ou un lubrifiant.

2. Formulation pharmaceutique pour comprimés selon la revendication 1, dans laquelle, dans la forme galénique unitaire, le rapport entre poids de vildagliptine et l'un quelconque de ses sels correspondants et poids de l'hydroxypropylméthylcellulose est compris entre 0,16 et 2,5, de préférence entre 0,3 et 1,16 ou 0,4 et 1.

3. Formulation pharmaceutique pour comprimés selon l'une quelconque des revendications 1 et 2 comprenant :
(a) 15 à 55 %, de préférence 25 à 45 % en poids, sur la base du poids sec, d'une charge acceptable sur le plan pharmaceutique, et en option
(b) 0,1 à 10 %, de préférence 0,1 à 3 % en poids, sur la base du poids sec, d'un lubrifiant acceptable sur le plan pharmaceutique.

4. Formulation pharmaceutique pour comprimés selon l'une quelconque des revendications 1 à 3, dans laquelle la charge est le lactose et/ou la cellulose microcristalline et/ou le lubrifiant est le stéarate de magnésium.

5. Formulation pharmaceutique pour comprimés selon la revendication 4, dans laquelle le lactose est présent dans une quantité comprise entre 1 % et 8 % en poids.

6. Formulation pharmaceutique pour comprimés selon l'une quelconque des revendications précédentes, dans laquelle le composé est une forme cristalline de la vildagliptine, de préférence la forme cristalline «A» ou l'un quelconque de ses sels.

7. Formulation pharmaceutique pour comprimés selon l'une quelconque des revendications précédentes, dans laquelle, par rapport au poids de la formulation :
(a) la vildagliptine, ou la quantité correspondante de l'un quelconque de ses sels, est présente dans une quantité comprise entre 15 % et 35 % en poids ;
(b) l'hydroxypropylméthylcellulose est présente dans une quantité comprise entre 30 % et 50 % en poids ;
(c) la cellulose microcristalline est présente dans une quantité comprise entre 25 % et 35 % en poids ; et
(d) le stéarate de magnésium est présent dans une quantité comprise entre 0,1 % et 3 % en poids.

8. Formulation pharmaceutique pour comprimés comprenant, pour 600 mg de comprimé, les ingrédients suivants :
(a) la vildagliptine dans une quantité égale à 150 mg, ou la quantité correspondante de l'un quelconque de sels ;
(b) une hydroxypropylméthylcellulose dans une quantité environ égale à 240 mg, où l'hydroxypropylméthylcellulose présente une viscosité apparente de 80 000 cP à 120 000 cP (valeur nominale de 100 000 cP) lorsqu'elle est présente dans une solution à 1 % ;
(c) une cellulose microcristalline dans une quantité égale à 180 mg ;
(d) un lactose dans une quantité environ égale à 24 mg ; et
(e) un stéarate de magnésium dans une quantité égale à 6 mg.

9. Formulation pharmaceutique pour comprimés comprenant, pour 400 mg de comprimé, les ingrédients suivants :
(a) la vildagliptine dans une quantité égale à 100 mg, ou la quantité correspondante de l'un quelconque de ses sels ;
(b) une hydroxypropylméthylcellulose dans une quantité environ égale à 160 mg, où l'hydroxypropylméthylcellulose présente une viscosité apparente de 80 000 cP à 120 000 cP (valeur nominale de 100 000 cP) lorsqu'elle est présente dans une solution à 1 % ;
(c) une cellulose microcristalline dans une quantité égale à 120 mg ;
(d) un lactose dans une quantité environ égale à 16 mg ; et
(e) un stéarate de magnésium dans une quantité égale à 4 mg.

10. Procédé de production d'une formulation pharmaceutique pour comprimés selon l'une quelconque des revendication 1 à 9, dans lequel le procédé comprend la combinaison des ingrédients dans les quantités énoncées dans l'une quelconque de ces revendications.

11. Comprimé pharmaceutique multicouche, dans lequel la formulation pharmaceutique pour comprimés selon l'une quelconque des revendications 1 à 9 représente une des couches du comprimé.

12. Comprimé pharmaceutique multicouche selon la revendication 11, dans lequel l'autre couche contient une glitazone (par exemple pioglitazone ou rosiglitazone) ou une metformine.

13. Comprimé pharmaceutique multicouche selon la revendication 12, dans lequel l'autre couche est une formulation à libération immédiate.

14. Comprimé pharmaceutique obtenu par compression d'une formulation pharmaceutique pour comprimés selon l'une quelconque des revendications 1 à 13.

15. Comprimé pharmaceutique obtenu par compression d'une formulation pharmaceutique pour comprimés selon l'une quelconque des revendications 1 à 13, dans lequel la formulation pharmaceutique pour comprimés est soumise à un compactage au rouleau avant la compression en comprimé.

16. Comprimé pharmaceutique selon la revendication 15, comprenant 100 mg de vildagliptine ou d'un sel de celle-ci acceptable sur le plan pharmaceutique, dans lequel la plage de dureté du comprimé est comprise entre 10 et 13 kp.

17. Comprimé pharmaceutique selon la revendication 15, comprenant 150 mg de vildagliptine ou d'un sel de celle-ci acceptable sur le plan pharmaceutique, dans lequel la plage de dureté du comprimé est comprise entre 11 et 25 kp.

18. Formulation pharmaceutique pour comprimés selon l'une quelconque des revendications 1 à 9 ou comprimé pharmaceutique selon l'une quelconque des revendications 11 à 17, comprenant au moins un autre agent thérapeutique.

19. Formulation pharmaceutique pour comprimés ou comprimé pharmaceutique selon la revendication 18, dans lequel au moins un agent thérapeutique supplémentaire est un agent antidiabétique.

20. Formulation pharmaceutique pour comprimés ou comprimé pharmaceutique selon la revendication 19, dans lequel l'agent antidiabétique est sélectionné parmi la pioglitazone, la rosiglitazone ou la metformine.

21. Formulation pharmaceutique pour comprimés ou comprimé pharmaceutique selon la revendication 18, dans lequel au moins un agent thérapeutique supplémentaire est un antagoniste de l'angiotensine II ou une statine.

22. Procédé de préparation d'un comprimé, sous une forme galénique unitaire, le procédé comprenant les étapes consistant à :
(a) mélanger une formulation pharmaceutique pour comprimés selon l'une quelconque des revendications précédentes ;
(b) comprimer la formulation préparée au cours de l'étape (a) pour former le comprimé fabriqué par compression sous une forme galénique unitaire.

23. Procédé de préparation d'un comprimé, sous une forme galénique unitaire, le procédé comprenant les étapes consistant à :
(a) mélanger une formulation pharmaceutique pour comprimés selon l'une quelconque des revendications 1 à 21 ;
(b) compacter au rouleau la formulation préparée au cours de l'étape (a) ; et
(c) comprimer la formulation préparée au cours de l'étape (b) pour former par compression le comprimé sous une forme galénique unitaire.

24. Procédé de préparation d'un comprimé, sous une forme galénique unitaire, le procédé comprenant les étapes consistant à :
(a) mélanger une formulation pharmaceutique pour comprimés selon l'une quelconque des revendications 1 à 21 ;
(b) compacter au rouleau la formulation préparée au cours de l'étape (a) avec une force de compactage comprise entre 10 et 16 KN ; et
(c) comprimer la formulation préparée au cours de l'étape (b) pour former par compression le comprimé sous une forme galénique unitaire.

25. Forme galénique pharmaceutique orale solide à libération prolongée selon l'une quelconque des revendications précédentes, qui est :
i-1) une forme galénique pharmaceutique orale solide comprenant environ 100 mg de base libre de vildagliptine, ou une quantité respective d'un de ses sels acceptables sur le plan pharmaceutique, et un milieu excipient, ladite forme galénique fournissant une concentration arithmétique maximum moyenne de vildagliptine dans le plasma comprise entre environ 15,8 ng/ml ± 6,85 ng/ml et environ 173 ng/ml ± 52 ng/ml entre environ 0,5 heure et environ 16 heures suivant l'administration par voie orale de ladite forme galénique à un patient non traité avec de la vildagliptine avant ladite administration, le patient se trouvant dans des conditions à jeun, et/ou
i-2) une forme galénique pharmaceutique orale solide comprenant environ 100 mg de base libre de vildagliptine, ou une quantité respective d'un de ses sels acceptables sur le plan pharmaceutique, et un milieu excipient, ladite forme galénique fournissant une concentration arithmétique maximum moyenne de vildagliptine dans le plasma comprise entre environ 26,3 ng/ml ± 13,1 ng/ml et environ 175 ng/ml ± 62,5 ng/ml entre environ 0,5 heure et environ 16 heures suivant l'administration de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
i-3) une forme galénique pharmaceutique orale solide comprenant environ 100 mg de base libre de vildagliptine, ou une quantité respective d'un de ses sels acceptables sur le plan pharmaceutique, et un milieu excipient, ladite forme galénique fournissant une concentration arithmétique maximum moyenne de vildagliptine dans le plasma comprise entre environ 26,9 ng/ml ± 14,1 ng/ml et environ 186 ng/ml ± 80,6 ng/ml entre environ 0,5 heure et environ 16 heures suivant l'administration de ladite forme galénique le jour 10 à un patient traité avec ladite forme galénique une fois par jour depuis le jour 1, ledit patient se trouvant dans un état à jeun, et/ou
ii-1) une forme galénique orale solide comprenant environ 100 mg de base libre de vildagliptine, ou une quantité respective d'un de ses sels acceptables sur le plan pharmaceutique, et un milieu excipient, ladite forme galénique fournissant une aire sous la courbe arithmétique moyenne AUC_{(0-inf)} de vildagliptine allant d'environ 1073 à environ 1825 ng.h/ml, à savoir 1449 ng.h/ml ± 376 ng.h/ml suivant l'administration par voie orale de ladite forme galénique à un patient non traité avec de la vildagliptine avant ladite administration, ledit patient se trouvant dans un état à jeun, et/ou
ii-2) une forme galénique orale solide comprenant environ 100 mg de base libre de vildagliptin, ou une quantité respective d'un de ses sels acceptables sur le plan pharmaceutique, est un milieu excipients, ladite forme galénique fournissant une aire sous la courbe arithmétique moyenne AUC₍₀₋₂₄₎ de vildagliptine allant d'environ 1001 à environ 1977 ng.h/ml, à savoir 1489 ng.h/ml ± 488 ng.h/ml suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
ii-3) une forme galénique orale solide comprenant environ 100 mg de base libre de vildagliptine, ou une quantité respective d'un de ses sels acceptables sur le plan pharmaceutique, et un milieu excipient, ladite forme galénique fournissant une aire sous la courbe arithmétique moyenne AUC₍₀₋₂₄₎ de vildagliptine allant d'environ 1103 à environ 2173 ng.h/ml, à savoir 1638 ng.h/ml ± 535 ng.h/ml suivant l'administration par voie orale de ladite forme galénique le jour 10 à un patient traité avec ladite forme galénique une fois par jour depuis le jour 1, ledit patient se trouvant dans un état à jeun, et/ou
iii-1) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique de tₘₐₓ de vildagliptine de 3,61 h ± 1,44 h suivant l'administration par voie orale de ladite forme galénique à un patient non traité avec de la vildagliptine avant ladite administration, ledit patient se trouvant dans un état à jeun, et/ou
iii-2) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique de tₘₐₓ de vildagliptine de 2,59 h ± 1,4 h suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
iii-3) une forme galénique orale solide comprenant environ 100 mg de base libre de vildagliptine, ou une quantité respective d'un de ses sels acceptable sur le plan pharmaceutique, et un milieu excipient, ladite forme galénique fournissant une moyenne arithmétique de tₘₐₓ de vildagliptine de 3,74 h ± 1,44 h suivant l'administration par voie orale de ladite forme galénique le jour 10 à un patient traité avec ladite forme galénique une fois par jour depuis le jour 1, ledit patient se trouvant dans un état à jeun, et/ou
iv-1) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique de Cₘₐₓ de vildagliptine de 205 ng/ml ± 47 ng/ml suivant l'administration par voie orale de ladite forme galénique à un patient non traité avec de la vildagliptine avant ladite administration, ledit patient se trouvant dans un état à jeun, et/ou
iv-2) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique de Cₘₐₓ de vildagliptine de 200 ng/ml ± 64 ng/ml suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
iv-3) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique de Cₘₐₓ de vildagliptine de 245 ng/ml ± 68 ng/ml suivant l'administration par voie orale de ladite forme galénique le jour 10 à un patient traité avec ladite forme galénique une fois par jour depuis le jour 1, ledit patient se trouvant dans un état à jeun, et/ou
v-1) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique du % d'inhibition de l'activité de la DPP-IV sur 24 heures de 85,64 % ± 12,76 % suivant l'administration par voie orale de ladite forme galénique à un patient non traité avec de la vildagliptine avant ladite administration, ledit patient se trouvant dans un état à jeun, et/ou
v-2) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique du % d'inhibition de l'activité de la DPP-IV sur 24 heures de 87,78 % ± 16,37 % suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
v-3) une forme galénique orale solide comprenant environ 100 mg de base libre de vildagliptine, ou une quantité respective d'un de ses sels acceptables sur le plan pharmaceutique, et un milieu excipient, ladite forme galénique fournissant une moyenne arithmétique du % d'inhibition de l'activité de la DPP-IV sur 24 heures de 90,20 % ± 7,35 % suivant l'administration par voie orale de ladite forme galénique le jour 10 à un patient traité avec ladite forme galénique une fois par jour depuis le jour 1, ledit patient se trouvant dans un état à jeun, et/ou
vi-1) une forme galénique orale solide comprenant environ 100 mg de base libre de vildagliptine, ou une quantité respective d'un de ses sels acceptables sur le plan pharmaceutique, et un milieu excipient, ladite forme galénique fournissant un profil pharmacocinétique tel qu'essentiellement dépeint à la figure 24 suivant l'administration par voie orale de ladite forme galénique à un patient non traité avec de la vildagliptine avant ladite administration, ledit patient se trouvant dans un état à jeun, et/ou
vi-2) une forme galénique orale solide comprenant environ 100 mg de base libre de vildagliptine, ou une quantité respective d'un de ses sels acceptables sur le plan pharmaceutique, et un milieu excipient, ladite forme galénique fournissant un profil pharmacocinétique tel qu'essentiellement dépeint à la figure 25 suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
vi-3) une forme galénique orale solide comprenant environ 100 mg de base libre de vildagliptine, ou une quantité respective d'un de ses sels acceptables sur le plan pharmaceutique, et un milieu excipient, ladite forme galénique fournissant un profil pharmacocinétique tel qu'essentiellement dépeint à la figure 26 suivant l'administration par voie orale de ladite forme galénique le jour 10 à un patient traité avec ladite forme galénique une fois par jour depuis le jour 1, ledit patient se trouvant dans un état à jeun.

26. Forme galénique pharmaceutique orale solide à libération prolongée selon l'une quelconque des revendications précédentes, qui est :
i-a) une forme galénique pharmaceutique orale solide comprenant environ 150 mg de base libre de vildagliptine, ou une quantité respective d'un de ses sels acceptables sur le plan pharmaceutique, et un milieu excipient, ladite forme galénique fournissant une concentration arithmétique maximum moyenne de vildagliptine dans le plasma comprise entre environ 30,7 ng/ml ± 21,9 ng/ml et environ 223 ng/ml ± 77,3 ng/ml entre environ 0,5 heure et environ 16 heures suivant l'administration par voie orale de ladite forme galénique à un patient non traité avec de la vildagliptine avant ladite administration, le patient se trouvant dans des conditions à jeun, et/ou
i-b) une forme galénique pharmaceutique orale solide comprenant environ 150 mg de base libre de vildagliptine, ou une quantité respective d'un de ses sels acceptables sur le plan pharmaceutique, et un milieu excipient, ladite forme galénique fournissant une concentration arithmétique maximum moyenne de vildagliptine dans le plasma comprise entre environ 48,7 ng/ml ± 25,8 ng/ml et environ 223 ng/ml ± 99,7 ng/ml entre environ 0,5 heure et environ 16 heures suivant l'administration de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
i-c) une forme galénique pharmaceutique orale solide comprenant environ 150 mg de base libre de vildagliptine, ou une quantité respective d'un de ses sels acceptables sur le plan pharmaceutique, et un milieu excipient, ladite forme galénique fournissant une concentration arithmétique maximum moyenne de vildagliptine dans le plasma comprise entre environ 44,6 ng/ml ± 28,5 ng/ml et environ 263 ng/ml ± 84,4 ng/ml entre environ 0,5 heure et environ 16 heures suivant l'administration de ladite forme galénique le jour 10 à un patient traité avec ladite forme galénique une fois par jour depuis le jour 1, ledit, patient se trouvant dans un état à jeun, et/ou
ii-a) une forme galénique orale solide comprenant environ 150 mg de base libre de vildagliptine, ou une quantité respective d'un de ses sels acceptables sur le plan pharmaceutique, et un milieu excipient, ladite forme galénique fournissant une aire sous la courbe arithmétique moyenne AUC_{(0-inf)} de vildagliptine allant d'environ 1346 à environ 3196 ng.h/ml, à savoir 2271 ng.h/ml ± 925 ng.h/ml suivant l'administration par voie orale de ladite forme galénique à un patient non traité avec de la vildagliptine avant ladite administration, ledit patient se trouvant dans un état à jeun, et/ou
ii-b) une forme galénique orale solide comprenant environ 150 mg de base libre de vildagliptine, ou une quantité respective d'un de ses sels acceptables sur le plan pharmaceutique, et un milieu excipient, ladite forme galénique fournissant une aire sous la courbe arithmétique moyenne AUC₍₀₋₂₄₎ de vildagliptine allant d'environ 1277 à environ 3207 ng.h/ml, à savoir 2242 ng.h/ml ± 965 ng.h/ml suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
ii-c) une forme galénique orale solide comprenant environ 150 mg de base libre de vildagliptine, ou une quantité respective d'un de ses sels acceptables sur le plan pharmaceutique, et un milieu excipient, ladite forme galénique fournissant une aire sous la courbe arithmétique moyenne AUC₍₀₋₂₄₎ de vildagliptine allant d'environ 1643 à environ 3273 ng.h/ml, à savoir 2458 ng.h/ml ± 815 ng.h/ml suivant l'administration par voie orale de ladite forme galénique le jour 10 à un patient traité avec ladite forme galénique une fois par jour depuis le jour 1, ledit patient se trouvant dans un état à jeun, et/ou
iii-a) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique de tₘₐₓ de vildagliptine de 3,57 h ± 1,17 h suivant l'administration par voie orale de ladite forme galénique à un patient non traité avec de la vildagliptine avant ladite administration, ledit patient se trouvant dans un état à jeun, et/ou
iii-b) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique de tₘₐₓ de vildagliptine de 2,87 h ± 1,59 h suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
iii-c) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique de tₘₐₓ de vildagliptine de 4,13 h ± 1,24 h suivant l'administration par voie orale de ladite forme galénique le jour 10 à un patient traité avec ladite forme galénique une fois par jour depuis le jour 1, ledit patient se trouvant dans un état à jeun, et/ou
iv-a) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique de Cₘₐₓ de vildagliptine de 257 ng/ml ± 59 ng/ml suivant l'administration par voie orale de ladite forme galénique à un patient non traité avec de la vildagliptine avant ladite administration, ledit patient se trouvant dans un état à jeun, et/ou
iv-b) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique de Cₘₐₓ de vildagliptine de 272 ng/ml ± 111 ng/ml suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
iv-c) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique de Cₘₐₓ de vildagliptine de 308 ng/ml ± 91 ng/ml suivant l'administration par voie orale de ladite forme galénique le jour 10 à un patient traité avec ladite forme galénique une fois par jour depuis le jour 1, ledit patient se trouvant dans un état à jeun, et/ou
v-a) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique du % d'inhibition de l'activité de la DPP-IV sur 24 heures de 90,04 % ± 11,91 % suivant l'administration par voie orale de ladite forme galénique, à un patient non traité avec de la vildagliptine avant ladite administration, ledit patient se trouvant dans un état à jeun, et/ou
v-b) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique du % d'inhibition de l'activité de la DPP-IV sur 24 heures de 90,4 % ± 17,50 % suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
v-c) une forme galénique orale solide comprenant environ 150 mg de base libre de vildagliptine, ou une quantité respective d'un de ses sels acceptables sur le plan pharmaceutique, et un milieu excipient, ladite forme galénique fournissant une moyenne arithmétique du % d'inhibition de l'activité de la DPP-IV sur 24 heures de 91,64 % ± 8,47 % suivant l'administration par voie orale de ladite forme galénique le jour 10 à un patient traité avec ladite forme galénique une fois par jour depuis le jour 1, ledit patient se trouvant dans un état à jeun, et/ou
vi-a) une forme galénique orale solide comprenant environ 150 mg de base libre de vildagliptine, ou une quantité respective d'un de ses sels acceptables sur le plan pharmaceutique, et un milieu excipient, ladite forme galénique fournissant un profil pharmacocinétique tel qu'essentiellement dépeint à la figure 24 suivant l'administration par voie orale de ladite forme galénique à un patient non traité avec de la vildagliptine avant ladite administration, ledit patient se trouvant dans un état à jeun, et/ou
vi-b) une forme galénique orale solide comprenant environ 150 mg de base libre de vildagliptine, ou une quantité respective d'un de ses sels acceptables sur le plan pharmaceutique, et un milieu excipient, ladite forme galénique fournissant un profil pharmacocinétique tel qu'essentiellement dépeint à la figure 25 suivant l'administration par voie orale de ladite forme galénique le jour 9 à un patient traité avec ladite forme galénique une fois par jour depuis le jour 1, un petit-déjeuner ADA étant servi au patient dans les 30 minutes suivant l'administration du matin de ladite forme galénique, et/ou
vi-c) une forme galénique orale solide comprenant environ 150 mg de base libre de vildagliptine, ou une quantité respective d'un de ses sels acceptables sur le plan pharmaceutique, et un milieu excipient, ladite forme galénique fournissant un profil pharmacocinétique tel qu'essentiellement dépeint à la figure 26 suivant l'administration par voie orale de ladite forme galénique le jour 10 à un patient traité avec ladite forme galénique une fois par jour depuis le jour 1, ledit patient se trouvant dans un état à jeun.

27. Forme galénique pharmaceutique orale solide à libération prolongée selon l'une quelconque des revendications 25 et 26, qui comprend une formulation pharmaceutique pour comprimés selon l'une quelconque des revendications 1 à 25.

28. Forme galénique pharmaceutique orale solide à libération prolongée selon l'une quelconque des revendications 25 et 26, qui comprend une hydroxypropylméthylcellulose, de préférence entre 20 % et 60 %, entre 30 % et 50 % en poids, sur la base du poids sec, d'une hydroxypropylméthylcellulose.

29. Forme galénique pharmaceutique orale solide à libération prolongée selon l'une quelconque des revendications 25 et 26, qui comprend une hydroxypropylméthylcellulose présentant une viscosité apparente de 80 000 cP à 120 000 cP (valeur nominale de 100 000 cP) lorsqu'elle est présente dans une solution à 1 %, de préférence comprise entre 20 % et 60 %, ou entre 30 % et 50 % en poids, sur la base du poids sec, d'une hydroxypropylméthylcellulose présentant une viscosité apparente de 80 000 cP à 120 000 cP (valeur nominale de 100 000 cP) lorsqu'elle est présente dans une solution à 1 %.

30. Forme galénique pharmaceutique orale solide à libération prolongée selon l'une quelconque des revendications 25 et 26, dans laquelle :
i) la formulation est une formulation de matrice contenant un polymère hydrophile acceptable sur le plan pharmaceutique qui peut retarder la diffusion de la vildagliptine,
ii) la forme galénique pharmaceutique orale solide est un comprimé fabriqué par compression, et en option
iii) la vitesse d'élution de la vildagliptine à 30 minutes suivant le début du test est inférieure à 30 % lorsqu'elle est conduite en utilisant la méthode à palette tournante.

31. Utilisation des ingrédients, dans les quantités énoncées dans l'une quelconque des revendications 1 à 30, dans la fabrication d'une formulation pharmaceutique pour comprimés destinée à inhiber l'activité de la dipeptidyl-peptidase IV chez un sujet.

32. Utilisation des ingrédients, dans les quantités énoncées dans l'une quelconque des revendications 1 à 30, dans la fabrication d'une formulation pharmaceutique pour comprimés destinée à traiter un état soulagé par l'inhibition de la dipeptidyl-peptidase IV chez un sujet souffrant de cet état.

33. Utilisation selon la revendication 32, dans laquelle l'état est un diabète sucré non insulino-dépendant.

34. Utilisation selon la revendication 32, dans laquelle l'état est l'obésité, l'arthrite ou l'ostéoporose.

35. Utilisation selon l'une quelconque des revendications 31 à 34, dans laquelle la formulation pharmaceutique pour comprimés est utilisée en combinaison avec une dose thérapeutiquement efficace d'un médicament antidiabétique, antiarthritique ou antiobésité.
